# NEUE EUROPÄISCHE PATENTSCHRIFT

(11) **EP 1 838 286 B2**
(45) Veröffentlichungstag und Bekanntmachung des Hinweises auf die Entscheidung über den Einspruch: **01.11.2017**
(45) Hinweis auf die Patenterteilung: 15.02.2012
(21) Anmeldenummer: 05823526.8
(22) Anmeldetag: 23.12.2005
(51) Int. Cl.: A61K 9/16, A61K 9/50, A61K 9/51

(54) **HERSTELLUNG VON LIPIDBASIERTEN NANOPARTIKELN UNTER EINSATZ EINER DUALEN ASYMMETRISCHEN ZENTRIFUGE**
PREPARATION OF LIPID BASED NANO-PARTICLES WITH A DUAL ASYMETRIC CENTRIFUGE
PRÉPARATION DE NANO-PARTICULES À BASES LIPIDES EN UTILISANT UNE CENTRIFUGE DUALE ET ASYMÉTRIQUE

(30) Priorität: 23.12.2004 EP 04106939
(43) Veröffentlichungstag der Anmeldung: 03.10.2007
(60) Teilanmeldung: 10178676.2
(73) Patentinhaber: Massing, Ulrich, 79249 Merzhausen (DE)
(72) Erfinder: Massing, Ulrich, 79249 Merzhausen (DE)
(74) Vertreter: dompatent von Kreisler Selting Werner - Partnerschaft von Patent- und Rechtsanwälten mbB
(86) Internationale Anmeldenummer: PCT/EP2005/057157
(87) Internationale Veröffentlichungsnummer: WO 2006/069985

(56) Entgegenhaltungen:
- EP-A- 0 587 106
- EP-A- 1 070 509
- DE-A1- 3 825 374
- US-A- 4 861 477
- US-A- 5 151 264
- US-A- 5 352 037
- US-A- 5 620 703
- US-A- 5 705 196
- US-A1- 2002 172 091
- US-A1- 2003 214 878
- US-A1- 2004 082 521
- US-B1- 6 770 299
- Güthlein, Frank, "Darstellung und Prüfung von vesikulären Phospholipidgelen (VPG zur Therapie solider Tumoren" 30.07.2002, ¬gefunden 14.11.2012|
- Fundstelle: ¬http://www.freidok.uni-freiburg.de/volltex te/500/|

## Beschreibung

Die Erfindung betrifft ein Verfahren zur Herstellung von lipidbasierten Nanopartikeln mit Hilfe einer dualen asymmetrischen Zentrifuge, durch dieses Verfahren hergestellte Produkte, Kits zur Herstellung solcher Nanopartikel mittels dualer asymmetrischer Zentrifuge, sowie Zubehör zur Durchführung des Verfahrens.

### Hintergrund der Erfindung

Lipidbasierte Nanopartikel wie z. B. Liposomen, Nano- und Mikroemulsionen, SLN (feste Lipidnanopartikel), Nanokapseln, Nanosphären und Lipoplexe sind wichtige Hilfsmittel für eine Vielzahl von technischen Prozessen und medizinischen Anwendungen.

Liposomen sind sphärische Gebilde, die aus Lipiden aufgebaut sind. In wässerigen Lösungen entstehen Liposomen durch Selbstaggregation dieser Lipide, wobei sich eine Lipiddoppelschicht bildet, die einen wässerigen Innenraum einschließt.

In Abhängigkeit von physikalischen Parametern wie mechanischen Einflüssen, Druck, Temperatur und der Ionenkonzentration sowie der vorhandenen Lipide und Zusatzstoffe, bilden sich unilamellare, oligolamellare oder multilamellare Liposomen. Die Liposomen können abhängig von ihren Bestandteilen eine positive oder negative Überschussladung tragen.

Liposomen können auch mit Substanzen beladen werden, die je nach Lipophilie oder Hydrophilie überwiegend in der Lipidschicht oder überwiegend im wässerigen Innenraum der Liposomen eingeschlossen sind. Derartige Liposomen werden in diagnostischen Nachweisverfahren, als therapeutische Mittel zum Transport von Wirkstoffen im Organismus oder als Wirkstoffdepot verwendet. Darüber hinaus können solche Liposomen auch in der biologischen und biomedizinischen Forschung und im Pflanzenschutz eingesetzt werden (z. B. zum Substanztransport in Zellen). Auch eine Anwendung in der Kosmetik ist möglich.

Die Eigenschaften der Liposomen wie beispielsweise ihre Stabilität oder Lagerfähigkeit werden wesentlich durch die in der Lipidschicht vorhandenen Substanzen bestimmt.

Zur Herstellung von Liposomen werden membranbildende Lipide, wie beispielsweise Phosphatidylcholin, Phosphatidylglycerin, Phosphatidylserin, Phosphatidylinositol und Phosphatidylethanolamin, Sphingomyelin, kationische Lipide wie beispielsweise DOTAP, DC-Chol, DOTMA u.a., membranbildende Amphiphile, wie beispielsweise Blockpolymere, Alkylester, -ether, -amide von Zuckern, Diolen und Polyolen, Aminen, Aminosäuren, Peptiden und Zuckern und Cholesterol sowie weitere Stoffe verwendet.

Zur Herstellung von möglichst einheitlichen leeren oder substanzbeladenen Liposomen sind verschiedene Verfahren bekannt. Diese Verfahren sind von Lasch et al. übersichtlich zusammengefasst worden (Lasch, J. et al., Preparation of Liposomes; in "Liposomes - a practical approach", Torchilin, V.P. und Weissig, V., Ed., 2. Aufl. (2003)).

Viele Herstellungsverfahren gehen von sog. "hand-shaken vesicles" aus, die durch einfache Rehydratisierung eines Lipidfilms und anschließendes Schütteln (meist in einem Kolben) hergestellt werden können. Diese Liposomen sind meist sehr uneinheitlich bezüglich ihrer Größe und ihrer Lamellarität. Mit den Verfahren der Extrusion, des Gefrier-Tau-Verfahrens oder der Ultrabeschallung können diese Liposomen in ihrer Größe vereinheitlicht (meist verkleinert) und ihrer Lamellarität verringert werden:

Extrusionsverfahren bedienen sich eines Extruders und ermöglichen in der Regel nur die Herstellung kleiner Mengen (Handextrusion) (Macdonald, R.E. et al., BBA 1061:297-303 (1991)). Die Herstellung großer Mengen ist mit hohem apparativen Aufwand (Pumpe, Membranen etc.) und einer aufwändigen Vorbereitung verbunden. Überdies können nur niedrigviskose Medien extrudiert werden, was zu einer niedrigen Einschlusseffizienz führt. Durch den offenen Umgang mit den Medien ist die Herstellung steriler Proben aufwändig und bei radioaktiven Materialien besteht die Gefahr der Kontamination der Umgebung. Lipidmischungen müssen außerdem vor der Extrusion und der Hydratation durch gemeinsames Lösen und anschließendes Evaporieren hergestellt werden.

Injektionsverfahren und Detergenzverfahren sind zwar großtechnisch möglich, aber die Lösungsmittelentfernung bzw. die Entfernung des Detergenz ist problematisch. Hohe Lipidkonzentrationen sind nicht einfach herstellbar, und durch den Überschuss an wässerigem Medium werden nur niedrige Einschlusseffizienzen bei hydrophilen Substanzen erreicht.

Auch durch Gefrier-Tau-Schritte können uneinheitliche, oft multilamellare Liposomen in ihrer Größe vereinheitlicht werden und ihre Lamellarität erniedrigt werden. Die Folge ist meist eine gesteigerte Einschlusseffizienz für wasserlösliche Substanzen.

Bei den bisher genannten Verfahren ist insbesondere die niedrige Einschlusseffizienz für hydrophile Substanzen ein systemimmanentes Problem. Dies begründet sich dadurch, dass diese Verfahren nur mit relativ geringen Lipidmengen durchführbar sind und folglich die gebildeten Liposomen nur einen geringen Anteil des wässerigen Gesamtvolumens einschließen können. Hydrophile, in der wässerigen Phase gelöste Substanzen werden folglich auch nur zu einem geringen Anteil eingeschlossen.

Die Einschlusseffizienz lässt sich z. B. durch eine Liposomen-Herstellung durch Hochdruckhomogenisation steigern, da hier erheblich höhere Lipidmengen eingesetzt werden können. Als Ergebnis erhält man bei sehr hohem Lipidanteil ein sogenanntes Liposomen-Gel, bei dem das wässerige Au ßenvolumen größenordnungsmäßig in etwa dem wässerigen Innenvolümen entspricht. Dementsprechend hoch ist dann auch der Anteil des eingeschlossenen hydrophilen Wirkstoffs. Ein weiterer Vorteil ist die Möglichkeit zur Herstellung von großen Mengen einer Formulierung, die mit Hilfe der Hochdruckhomogenisation einfach gelingt. Die Hochdruckhomogenisation hat zudem den Vorteil, insbesondere kleine Vesikel zu erzeugen, die u.a. im medizinischen Bereich von besonderem Interesse sind, z. B. für ein Tumortargeting unter Nutzung des sogenannten EPR-Effekts (enhanced permeability and retention). Dieser Effekt beruht auf der meist stark erhöhten vaskulären Permeabilität von Blutgefäßen in Tumoren. Durch die Undichtigkeit der Gefäße können kleine Partikel wie z. B. Liposomen (insbesondere wenn sie sehr klein sind) das Gefäßbett verlassen und sich im Tumor anreichern (Yuan, F. et al., Cancer Res. 54:3352-3356 (1994)).

Den wirtschaftlich besonders vorteilhaften Eigenschaften der Hochdruckhomogenisation (kleine Vesikel, hohe Einschlusseffizienz, große Probenmengen) steht eine Reihe von Nachteilen gegenüber:

Die Herstellung von sterilen Materialien, wie sie für die Anwendung an Mensch und Tier unerlässlich sind, ist problematisch. Eine sterile Herstellung ist zwar möglich, aber wegen der notwendigen Durchführung der Hochdruckhomogenisation unter Reinraumbedingungen oder der nachträglichen Autoklavierung des Materials aufwändig. Zusätzlich ergeben sich bei der Autoklavierung von wirkstoffhaltigen vesikulären Phospholipid Gelen (VPG) oft Probleme mit der Wirkstoff- und/oder der Lipidstabilität (Moog, R. et al., Int. J. Pharm. 206:43-53 (2000)).

Mit Hilfe der Hochdruckhomogenisation ist es insbesondere schwierig, kleine Probenmengen herzustellen, wie sie z. B. in medizinischen oder molekularbiologischen Laboren (wenn nur wenig Substanz zur Verfügung steht, bei Verwendung von radioaktiven Substanzen etc.) oder bei einem Screening von sehr vielen verschiedenen Lipidformulierungen (z. B. im Bereich der Präformulierung) benötigt werden.

Zudem wird die Probe durch den Homogenisierungsschritt stark belastet, was insbesondere bei teuren und empfindlichen Substanzen wie z. B. biologischen Materialien (DNA, siRNA, Antikörper, Peptide, Proteine) oder bei empfindlichen niedermolekularen Substanzen wie z. B. Antioxidantien, Lipiden mit bestimmten hochungesättigten Fettsäuren, Zytostatika (Alkylantien etc.) kritisch ist.

Die Ausrüstung für die Hochdruckhomogenisation ist teuer, groß und für viele (insbesondere medizinische/molekularbiologische) Labors nicht akzeptabel. Da bei jedem Wechsel der Zusammensetzung die Maschine gereinigt werden muss, ist der Probendurchsatz niedrig, ein Screening von verschiedenen Mischungen praktisch nicht möglich und nur schwer zu automatisieren (z. B. im Bereich Präformulierung).

Zudem ist überdurchschnittliches technologisches Know-how nötig, nicht zuletzt um die Gefahr einzuschränken, die bei Verwendung gefährlicher Substanzen (z. B. radioaktiver Substanzen oder Zytostatika) für die Umgebung besteht.

Die Probleme bei der Herstellung von Liposomen haben bereits dazu geführt, dass für kosmetische Zwecke Liposomen-Gele eingesetzt werden, welche nur durch Rehydratisierung einer Lipidmischung entstanden sind, wobei auf eine Vereinheitlichung der Vesikel völlig verzichtet wird (DE 10255285). Solche Formulierungen sind für pharmazeutische, biomedizinische und medizinische Zwecke nicht brauchbar und aufgrund der geringen Reproduzierbarkeit der Vesikelzusammensetzung für kosmetische Anwendungen zumindest bedenklich.

SLN sind Nanopartikel mit einer Größe von ca. 50 bis 1000 nm. Eine Übersicht über SLN wird in Pharmazeutische Technologie: Moderne Arzneiformen. R.H. Müller und G.E. Hildebrand, Wissenschaftliche Verlagsgesellschaft Stuttgart, 1998, gegeben. Das Matrixmaterial besteht - im Gegensatz zu Emulsionen - aus festen Lipiden. Hier werden überwiegend eingesetzt: physiologische Lipide (z. B. entsprechende Triglyceride) oder Lipide aus physiologischen Komponenten (z. B. Glyceride aus körpereigenen Fettsäuren). Es wird angenommen, das dadurch eine gute *in vivo* Verträglichkeit erreicht wird (Weyhers, H. et al., Proc. 1st world meeting APV/APGI, Budapest, 489-490 (1995)). Das Matrixmaterial ist aber nicht auf physiologische Lipide beschränkt, vorstellbar sind z. B. auch Wachse und nicht physiologische Triglyceride.

SLN werden bisher durch Hochdruckhomogenisation von in Wasser dispergierten Lipiden im geschmolzenen oder im festen Zustand hergestellt (Heiß- bzw. Kalthomogenisation; siehe Müller, R.H., Pharm. Ind. 49:423-427 (1997); EP 0 605 497; Schwarz, C. et al., J. Controlled Rel. 30:83-96 (1994); Müller, R.H. et al., Eur. J. Pharm. Biopharm. 41:62-69 (1995)). Diese Herstellungstechnik durch Hochdruckhomogenisation zeichnet sich dadurch aus, dass die SLN in ihrer Größe sehr homogen sind und zudem der Anteil an Mikropartikeln sehr gering ist. Der Aufwand für solch eine Hochdruckhomogenisation ist jedoch wie bei der Herstellung von Liposomen sehr hoch.

Ein weiterer Typ lipidbasierter Nanopartikel sind die Tröpfchen in Emulsionen, wobei hier Submikron-Emulsionen (SME) gemeint sind, also O/W-Emulsionen mit Tröpfchen/Partikelgrößen von unter 1 µm (Benita, S. et al., J. Pharm. Sci. 82:1069-1079 (1993)). Nicht davon abgegrenzt werden können sog. Nanoemulsionen mit Tröpfchengrößen von 50-1000 nm.

Solche Emulsionen werden seit langem zur parenteralen Ernährung eingesetzt (Lucks, J. S. et al., Krankenhauspharmazie 15:51-57 (1994)), können aber auch als Arzneistoffträger verwendet werden. Eine Übersicht zu SMEs findet sich in Pharmazeutische Technologie: Moderne Arzneiformen. R.H. Müller und G.E. Hildebrand, Wissenschaftliche Verlagsgesellschaft Stuttgart, 1998.

Unter den derzeit eingesetzten SME-Herstellungsverfahren ist die Hochdruckhomogenisation (durch Kolbenspalthomogenisatoren oder durch Microfluidizer-Technik) die führende Technik. Im Labor- und Technikumsmaßstab kommt auch die Ultraschallhomogenisation zum Einsatz, v.a. deswegen, weil die Hochdruckhomogenisation zu aufwendig ist.

WO 02/09677 beschreibt die Herstellung einer Dispersion, die eine O/W- oder W/O-Emulsion sowie einen in Öl und Wasser schwerlöslichen Wirkstoff (Amphotericin B) umfasst. Diese Dispersion kann eine Menge des Wirkstoffs enthalten, die höher ist als die Menge, die sich additiv aus seiner maximalen Löslichkeit in Wasser oder Öl ergibt. Die Homogenisierung erfolgt allerdings hochenergetisch, also durch Hochdruckhomogenisierung mit den damit verbundenen oben aufgeführten Nachteilen. Das Verfahren ist auch als sog. SolEmuls-Technologie beschrieben (Müller, R.H. et al., Int. J. Pharm. 269:293-302 (2004)). Hierbei werden mit Hilfe der hochenergetischen Hochdruckhomogenisation schwer lösliche Wirkstoffe wie Carbamazepin, Itraconazole oder Amphotericin B durch Co-Homogenisation in Emulsionen eingearbeitet, was zwar eine starke Steigerung insbesondere der Lösungsgeschwindigkeit zur Folge hat, aber auch die bereits benannten Nachteile einer Hochdruckhomogenisation besitzt. In dem US Patent 5,151,264 werden Lipidpartikel offenbart, d.h. Partikel mit einem festen hydrophilen Kern aus Polysachariden, Dextrosen, Stärke, Cellulose. Es handelt sich hierbei jedoch nicht um lipidbasierte Nanopartikel. In der US 2004/0082521 ist die Herstellung liposomaler Digitalisglycoside durch Hochdruckhomogenisierung bekannt. Andererseits ist aus US 2003/214878 eine Mischvorrichtung für Farbkanister mit einem Volumen von mindestens einer Gallone bekannt, in der der Mischbehälter ein im Wesentlichen rundes Gefäß ist. Mischbehälter mit anderer Form für Mischvorrichtungen sind aus US 2002/172091 und US Patent 4,861,477 bekannt. Die der Erfindung zugrundeliegende Aufgabe war es somit, ein möglichst einfaches Verfahren zur Herstellung lipidbasierter Nanopartikel bereitzustellen, das schonender und sicherer als die Hochdruckhomogenisation ist, sich zum Screening eignet und die Herstellung von Nanopartikeln auch und insbesondere im Labormaßstab erlaubt.

### Kurzbeschreibung der Erfindung

Es wurde gefunden, dass sich eine duale asymmetrische Zentrifuge sehr gut zur Herstellung von lipidbasierten Nanopartikeln eignet, wobei diese mit einer oder mehreren physiologisch wirksamen und/oder diagnostisch/biologisch/chemisch/biochemisch relevanten Verbindung(en) beladen sein können.

### Die Erfindung umfasst somit

(1) ein Verfahren zur Herstellung von lipidbasierten Nanopartikeln durch Homogenisieren einer Lipidkomponente mit einer wässerigen Komponente in einer dualen asymmetrische Zentrifuge (DAZ); oder von mit Wirkstoff beladenen lipidbasierten Nanopartikeln durch Homogenisieren einer Lipidkomponente mit einer wässrigen Komponente und dem Wirkstoff in einer DAZ oder durch Einarbeiten des Wirkstoffs in vorgefertigte lipidbasierte Nanopartikel in einer DAZ;
(2) eine bevorzugte Ausführungsform des Verfahrens (1), wobei
   (i) die Homogenisierung bzw. das Einarbeiten bei wenigstens 200 rpm, bevorzugt bei wenigstens 1000 rpm und maximal 4000 rpm, besonders bevorzugt bei 2000 bis 3540 rpm, am bevorzugtesten bei 2500 bis 3540 rpm erfolgt; und/oder
   (ii) die g-Zahl wenigstens 1,2 g, bevorzugt wenigstens 80 g, besonders bevorzugt wenigstens 300 g, ganz besonders bevorzugt von 550 bis 1000 g oder von 620 bis 1500 g beträgt; und/oder
   (iii) das Rückdrehverhältnis von 1:6 bis 6:1 beträgt, bevorzugt kleiner als 5:1, besonders bevorzugt kleiner als 3:1 ist; und/oder
   (iv) die Zentrifugationszeit von 30 s bis 1 h, bevorzugt von 1 bis 40 min, besonders bevorzugt von 3 bis 20 min beträgt; und/oder
   (v) eine Mischhilfe, bevorzugt Glasperlen mit einem Durchmesser von 0,5 bis 6 mm, verwendet wird;
(3) eine bevorzugte Ausführungsform des Verfahrens (1) oder (2), wobei die lipidbasierten Nanopartikel Liposomen einschließlich vesikulärer Phospholipidgele (VPG) sind;
(4) eine bevorzugte Ausführungsform des Verfahrens (1) oder (2), wobei die lipidbasierten Nanopartikel feste Lipidnanopartikel (SLN) sind;
(5) eine bevorzugte Ausführungsform des Verfahrens (1) oder (2), wobei die lipidbasierten Nanopartikel Tröpfchen in Emulsionen sind;
(6) eine bevorzugte Ausführungsform des Verfahrens (1) bis (5), die zum Screening lipidbasierter Nanopartikel, die bevorzugt gemäß dem Verfahren nach einem einer der Ausführungsformen (1) bis (5) hergestellt wurden, im Bereich der Präformulierung geeignet ist;
(7) lipidbasierte mit Wirkstoff beladene Nanopartikel, die Liposomen sind und die gemäß dem Verfahren nach einem oder mehreren der Verfahren (1) bis (3) herstellbar oder hergestellt sind, und die empfindliche oder kurzlebige Wirkstoffe oder Diagnostika enthalten, welche ausgewählt sind aus kurzlebig radioaktiv markierten Verbindungen oder Verbindungen für die Positronen-Emissions-Tomographie (PET)-Diagnostik;
(8) die Verwendung der lipidbasierten Nanopartikel nach Ausführungsform (7) zur Herstellung von pharmazeutisch oder diagnostisch einsetzbaren Zubereitungen;
(9) eine Zusammensetzung, insbesondere pharmazeutische oder diagnostische Zusammensetzung, enthaltend die lipidbasierten Nanopartikel nach Ausführungsform (7);
(10) eine Mischvorrichtung für chemisch und/oder biologische Substanzen, insbesondere eine zur Durchführung der Verfahren (1) bis (6) geeignete DAZ, mit einer ersten Antriebseinrichtung (12) zum Drehen eines Auslegers (16) um eine erste Drehachse (18),
   einem in einem Abstand zur ersten Drehachse (18) mit dem Ausleger (16) verbundenen Mischbehälter (40) zur Aufnahme der Substanzen,
   einer zweiten Antriebseinrichtung (30) zum Drehen des Mischbehälters (40) um eine zweite, durch den Mischbehälter (40) verlaufende Drehachse (28), wobei die Innenwände (46) des Mischbehälters (40) unterschiedliche Abstände zur zweiten Drehachse (28) aufweisen, wobei der Mischbehälter (40) im Wesentlichen zylindrisch ausgebildet ist, und eine Längsachse (47) des Mischbehälters (40) in einem Winkel zur zweiten Drehachse (28) angeordnet ist.
(11) einen Mischbehälter für eine DAZ, insbesondere zur Durchführung des Verfahrens (1) bis (6) und/oder für die Mischvorrichtung (10), mit einem ersten Aufnahmeraum (50) zur Aufnahme einer ersten Substanz, und mindestens einem zweiten Aufnahmeraum (52) zur Aufnahme einer zweiten Substanz und einer Trennwand (70) zwischen den Aufnahmeräumen (50, 52) mit einer Öffnung (68), um beim Auftreten von Zentrifugalkräften ein Überführen einer der Substanzen in den anderen Aufnahmeraum (50, 52) zu ermöglichen.
(12) ein Kit zur Durchführung des Verfahrens nach Ausführungsform (1) bis (6), umfassend
   (i) einen Mischbehälter nach Ausführungsform (11); und
   (ii) eine oder mehrere Lipidkomponente(n), sowie optional eine wässerige Komponente oder die nichtwässerigen Bestandteile der wässerigen Komponente und/oder Prämixe dieser Komponenten; und/oder
   (iii) vorgefertigte lipidbasierte Nanopartikel.

### Kurzbeschreibung der Figuren

Die Erfindung wird anhand der nachfolgenden Figuren in der detaillierten Beschreibung der Erfindung näher erläutert.
- Fig. 1:: Schematischer Aufbau einer dualen asymmetrischen Zentrifuge in Aufsicht.
- Fig. 2:: Schematischer Aufbau einer dualen asymmetrischen Zentrifuge in Seitenansicht.
- Fig. 3:: Mischbehälter für Mischvorrichtung der Ausführungsform (10).
- Fig. 4:: Mischbehälter mit Öffnung und zwei Aufnahmeräumen gemäß Ausführungsform (11).
- Fig. 5:: Partikelgrößenverteilung von Liposomen (Bsp. 1, vial 2 und 3), bestimmt als relative Gauß-Verteilung bezogen auf die Teilchenzahl; Diam: Vesikeldurchmesser; Rel: relativer Anteil in %: a) Herstellung mit DAZ (vial 2) und b) Herstellung durch Hochdruckhomogenisation (vial 3).
- Fig. 6:: Einfluss verschiedener Parameter auf die Partikelgröße bei Speedmixen in einer Injektionsflasche (vgl. Bsp. 17): A) Variation der Lipidkonzentration; B) Unterschiedliche Speedmix-Zeiten und C) Glasperlen unterschiedlicher Größe als Dispergierhilfe
- Fig. 7:: Einfluss der Speedmixer-Geschwindigkeit auf die Partikelgröße
- Fig. 8:: Mischvorrichtung der Ausführungsform (13) zum Speedmixen einer Injektionsflasche
- Fig. 9:: Schematische Schnittansicht der in Fig. 8 dargestellten Ausführungsform
- Fig. 10:: Mischbehälter zur Aufnahme von Kryo-Vials oder Eppendorfgefäßen, der bei Verwendung geeigneter Materialien wie Teflon, PE UHMW, Aluminium auch tieftemperaturgeeignet ist
- Fig. 11:: Schnittansicht entlang der Linie XI-XI in Fig. 10
- Fig. 12:: Schematische perspektivische Ansicht des in den Fg. 10 und 11 dargestellten Mischbehälters

### Detaillierte Beschreibung der Erfindung

Das erfindungsgemäße Verfahren hat sich als eine überraschend einfache Technik zur Herstellung von liposomalen Dispersionen, vesikulären Phospholipid-Gelen sowie anderen lipidbasierten Nanopartikeln herausgestellt, welche entweder keinen Wirkstoff enthalten oder welche als Wirkstoff wenigstens eine physiologisch wirksame oder/und diagnostische Verbindung enthalten. Mit der erfindungsgemäßen Technologie ist es insbesondere möglich, die Partikel steril und in einem Schritt herzustellen und wenn gewünscht, auch mit verschiedenen Substanzen zu beladen, wobei mit der Technik sehr kleine aber auch große Mengen herstellbar sind. Die Technik ist im Gegensatz zum Stand der Technik sehr einfach und rasch durchzuführen und erlaubt auch Personen ohne Lipsomen/Nanopartikel-Know-how die Herstellung. Aufgrund der raschen Durchführbarkeit des Verfahrens können auch instabile (z. B. hydrolyseempfindliche) Substanzen in lipidbasierte Nanopartikel eingeschlossen werden. Da die Herstellung steril und sehr rasch verläuft, können die Nanopartikel zur Herstellung von Medikamenten, beispielsweise von Injektionen, genutzt werden, auch und besonders unmittelbar vor Applikation am Patienten ("Bedside-Preparation"). Durch Veränderung der Parameter der dualen asymmetrischen Zentrifuge (z. B. Zentrifugationszeit und - geschwindigkeit) können lipidbasierte Nanopartikel unterschiedlicher Größe hergestellt werden. Es ist möglich, große wie kleine Moleküle einzuschließen und vieles mehr. Es war besonders überraschend, dass bei der erfindungsgemäßen Herstellung von Liposomen und anderen Nanopartikeln - die ja keinen Extrusionsschritt enthält - nur sehr geringe Anteile an Partikeln mit einem Durchmesser > 1 µm entstehen, ähnlich wie bei der Herstellung von Liposomen durch Hochdruckhomogenisation, wodurch die Formulierungen für die i. v. (intravenöse) Applikation geeignet sind. Aufgrund des minimalen Durchmessers einer Blutkapillare (5-6 µm) muss der Durchmesser der größten applizierten Teilchen in solchen Fällen deutlich unter 5 µm betragen, da es sonst zu einer Embolie kommen kann. Je geringer also die Teilchenzahl mit Partikeldurchmessern > 1 µm, desto vorteilhafter ist dies für die klinische Anwendung.

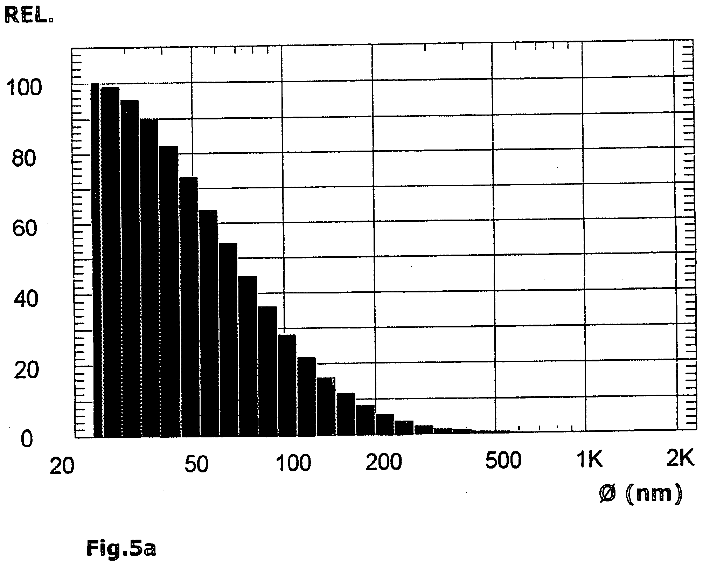

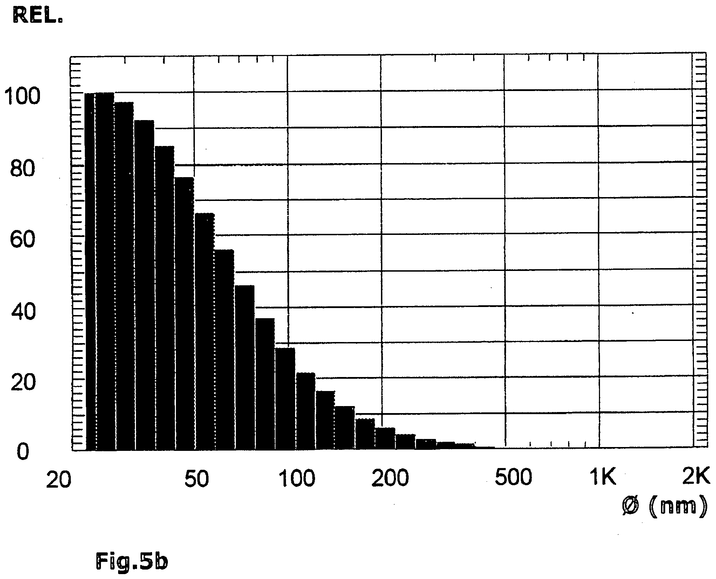

Es ist überraschend, dass mit dem erfindungsgemäßen Verfahren Liposomen mit einer Größenverteilung hergestellt werden können, die der Größenverteilung von durch Hochdruckhomogenisation hergestellten Liposomen entspricht (vgl. Fig. 5), und dass diese Liposomen mit ebenso guter Effizienz wie letztere beladen werden können. Zusätzlich werden weitere Nachteile des Hochdruckhomogenisationsverfahrens überwunden, insbesondere die hohe Belastung der Probe, der niedrige Probendurchsatz, die erforderlichen relativ hohen Probenvolumina, die Notwendigkeit speziellen Know-Hows sowie die Schwierigkeiten bei sterilem Arbeiten und bei Arbeiten mit gefährlichen Substanzen.

Im folgenden werden zunächst einige der verwendeten Begriffe näher definiert: "Lipidbasierte Nanopartikel" im Kontext der vorliegenden Erfindung sind Nanopartikel mit einer Partikelgröße von maximal 1 µm, bestehend mindestens aus einer Lipidkomponente, die optional eine wässerige Komponente und/oder weitere Verbindungen, insbesondere Wirkstoffe, enthalten oder mit diesen beladen werden können. Lipidbasierte Nanopartikel können als Feststoff vorliegen oder in einer wässerigen Komponente dispergiert sein. Es sind bevorzugt Liposomen, SLN oder die Tröpfchen einer Emulsion (SME).

Eine "Lipidkomponente" im Sinne der vorliegenden Erfindung umfasst eine oder mehrere Verbindungen, ausgewählt aus der Gruppe umfassend Amphiphile, Lipide und/oder Detergentien. Bevorzugt umfasst sie mindestens eine Verbindung ausgewählt aus Phospholipiden, Glycolipiden, Cholesterolen, Sphingolipiden, Polyethylenglykol-Lipidderivaten, kationischen Lipiden, Triglyceriden und Wachsen. Wie unterschiedlich die im Verfahren nach (1) eingesetzten Lipide sein können, zeigen Bsp. 3 und 4 für Liposomen, Bsp. 15 für SLN und Bsp. 14 für Emulsionen.

Bei Einsatz einer Mischung aus mehreren Lipiden als Lipidkomponente kann diese als feste Lösung oder als eine Mischung einzelner Lipid-Kristalle eingesetzt werden. Für analytische Zwecke in biologischen, medizinischen und chemischen Untersuchungen können markierte Lipide eingebracht werden. Bevorzugt sind fluoreszenzmarkierte, Spinlabel-markierte oder radioaktiv markierte Lipide.

Die Vielzahl von Kombinationsmöglichkeiten bei der Wahl der Bestandteile der Lipidkomponente für den Aufbau von lipidbasierten Nanopartikeln ermöglicht dem Fachmann das maßgeschneiderte Design dieser Partikel. Beispielhaft seien Immunoliposomen mit spezifischen Antikörpern an ihrer Oberfläche, negativ geladene Liposomen für das RES-targeting, positiv geladene Liposomen für das Targeting des aktivierten Endothels sowie neutrale Liposomen zur passiven Anreicherung in Tumorgewebe durch den EPR-Effekt genannt.

Die "wässerige Komponente" ist im Kontext der Erfindung Wasser oder eine wässerige alkoholische und/oder pufferhaltige Lösung. Zusätzlich können in ihr Salze sowie andere niedermolekulare wasserlösliche Substanzen gelöst sein. Sie wird im folgenden auch als "wässerige Phase" oder "wässeriges Medium" bezeichnet.

Ein "Lösungsmittel" (synonym auch als "Lösemittel" bezeichnet) ist eine Flüssigkeit, die Gase, andere Flüssigkeiten oder Feststoffe lösen kann, ohne dass es dabei zu chemischen Reaktionen zwischen gelöstem Stoff und lösender Flüssigkeit kommt.

"Physiologisch wirksame Verbindungen" (im folgenden auch als "Wirkstoffe" bezeichnet) im Sinne der vorliegenden Erfindung sind Verbindungen, die in Lebewesen, insbesondere in Pflanze, Mensch oder Tier, eine physiologische Reaktion hervorrufen können. Sie umfassen insbesondere in Kosmetik, Pflanzenschutz und Therapie eingesetzte Wirkstoffe.

Ein "Liposomen-Gel" (Liposomengel) im Sinne der vorliegenden Erfindung ist eine liposomale Formulierung, die viskos, d. h. nicht mehr frei fließend ist aufgrund eines hohen Lipidkomponenten-Gehalts und damit einer hohen Dichte an Liposomen (Vesikeln). Ein Liposomen-Gel enthält bevorzugt über 20 % Lipidkomponente, besonders bevorzugt membranbildende Amphiphile, ganz besonders bevorzugt über 30 % membranbildende Amphiphile. Ein Liposomen-Gel ist von Liposomen-enthaltenden Gelen abzugrenzen, bei denen die Gel-Eigenschaften (Viskosität, Verhindern des freien Fließens) nicht durch eine hohe Liposomendichte, sondern durch eine gel-bildende Komponente wie z. B. Polyacrylamid hervorgerufen wird.

Ein "Vesikuläres Phospholipid Gel" (VPG) ist ein Liposomen-Gel, bei dem die zur Vesikelbildung eingesetzten Amphiphile zu einem gewissen Anteil, der bevorzugt wenigstens 30% ausmacht, aus Phospholipiden bestehen. Vesikuläre Phospholipidgele (VPG) werden in der Literatur häufig auch als Liposomen-Gele bezeichnet, da fast alle bekannten Liposomen-Gele VPG sind.

"Passive loading" (EP 1087752) bezeichnet einen Vorgang, bei dem eine vorgefertigte liposomale Formulierung, die bevorzugt ein Liposomen-Gel und ganz besonders bevorzugt ein VPG ist, mit einem Wirkstoff, der über Liposomenmenbranen diffundieren kann, vermischt wird. Dabei wird der Wirkstoff initial zwischen die Liposomen eingelagert. Durch anschließende Inkubation, die bei Raumtemperatur, bevorzugt aber bei leicht erhöhter Temperatur, ganz besonders bevorzugt bei 40-80°C stattfindet, diffundiert der Wirkstoff in die Liposomen, bis sich zwischen den Anteilen des Wirkstoffs innerhalb und ausserhalb der Liposomen ein Gleichgewicht eingestellt hat. Die vorgefertigte liposomale Formulierung kann z. B. durch Hochdruckhomogenisation oder durch das hier vorgestellte Verfahren mit Hilfe einer DAZ hergestellt werden. Wichtige Wirkstoffe, bei denen das passive Loading verwendet werden kann, sind z. B. Gemcitabine, Vincristin, Vindesin und in der Krebstherapie eingesetzte Platinverbindungen.

"Solid lipid nanoparticles" (SLN), auch als "feste Lipidnanopartikel" bezeichnet, sind Nanopartikel im Größenbereich von 10 bis 1000 nm (wenn ein Nanopartikel definitionsgemäß keine Größe von 1000 nm haben darf, so bedeutet "bis 1000 nm" in diesem Kontext "bis zu 1000 nm", also kleiner als 1000 nm), bevorzugt von 20 bis 400 nm, bei denen das Matrixmaterial im Gegensatz zu Emulsionen aus festen Lipiden (vollständig lipophile Lipide) besteht. Als feste Lipide werden überwiegend physiologisch verträgliche Lipide eingesetzt wie entsprechende Triglyceride oder auch Wachse. Einsetzbar sind aber auch nicht physiologische Lipide. Wirkstoffe, die in SLN eingeschlossen sind, sind je nach ihrer Lipophilie entweder in der Lipidmatrix gelöst (feste Lösung), oder in der Matrix dispergiert. In der Regel sind SLN in wässerigen Dispersionen durch Tenside stabilisiert, allerdings ist auch eine tensidfreie Herstellung möglich. SLN können sowohl als wässerige Dispersion als auch als Trockenprodukt formuliert werden (Lyophilisation oder Sprühtrocknung).

Ein "Lipoplex" ist ein Lipid-Nukleinsäure-Komplex, insbesondere ein Lipid-DNA- oder Lipid-RNA-Komplex, und besteht mindestens aus einer Lipidkomponente (bevorzugt ein Amphiphil), wobei der hydrophile Teil der Lipidkomponente unter den Bedingungen der Lipoplexbildung eine positive Ladung trägt, und einer Nukleinsäure-Komponente, die unter den Bedingungen der Lipoplexbildung eine negative Ladung trägt. Je nach Zusammensetzung kann die mittlere Ladung eines Lipoplexes positiv, negativ oder auch neutral sein. Bevorzugt ist eine positive Ladung. Lipoplexe werden vor allem zum Nukleinsäure-Transfer in Zellen eingesetzt. In diesem Fall ist eine positive Ladung des Lipoplexes hilfreich, damit der Lipoplex an die üblicherweise negativ geladene Zellwand binden und von dort aufgenommen werden kann. Sonderformen von Lipoplexen sind z. B. AVE's (Artificial virus-like envelopes), bei denen die Nukleinsäure zunächst kondensiert (z. B. mit einem positiv geladenen Polymer) und dann in Liposomen eingeschlossen wird.

"Mittlere Größe" bezeichnet im Kontext der vorliegenden Erfindung den Median der Vesikelgröße, also denjenigen Vesikeldurchmesser, bei dem 50% der Vesikel kleiner und 50 % der Vesikel größer als der angegebene Wert sind. In der Regel ist dies gleichbedeutend mit dem Maximum einer Gauß-Größenverteilung.

Die "Einschlusseffizienz" ist im Kontext der vorliegenden Erfindung das Verhältnis (der prozentuale Anteil) von derjenigen Menge der gemessenen Substanz (z. B. eines Wirkstoffs), die in den lipidbasierten Nanopartikeln eingeschlossen, an die Nanopartikel assoziiert oder in die Nanopartikel inkorporiert wurde, zur für die Formulierung eingesetzten Gesamtmenge der betreffenden Substanz.

Eine "duale asymmetrische Zentrifuge" (DAZ) ist eine Zentrifuge, bei der zusätzlich zu den Vorgängen einer klassischen Zentrifugation das Gefäß mit dem zu zentrifugierenden Material bevorzugt entgegen der Drehbewegung der Zentrifuge gedreht wird, z. B. mit einem Viertel bis Drittel der Drehzahl der Zentrifuge. Dies führt zu einer stetigen Vermischung des in die DAZ eingebrachten Materials. Die hohe Zentripetalkraft erlaubt es auch, viskose Massen zu mischen (EP 1293245). Bei diesem Verfahren entsteht insbesondere in viskosen Massen eine starke innere Reibung. Die DAZ wird üblicherweise zur Vermischung von Pasten mit Pasten, Pasten mit Pulvern, Pulvern mit Pulvern etc. verwendet. Typische Einsatzgebiete sind die Anmischung von Dichtstoffen und Beschichtungsmitteln (wie z. B. Silikonen, Polyurethanen und Acrylaten), Lacken, Druckfarben und Pigmenten sowie das Vermischen von Ein-, Zwei- oder Multikomponenten-Produkten in flüssiger oder pastöser Form.

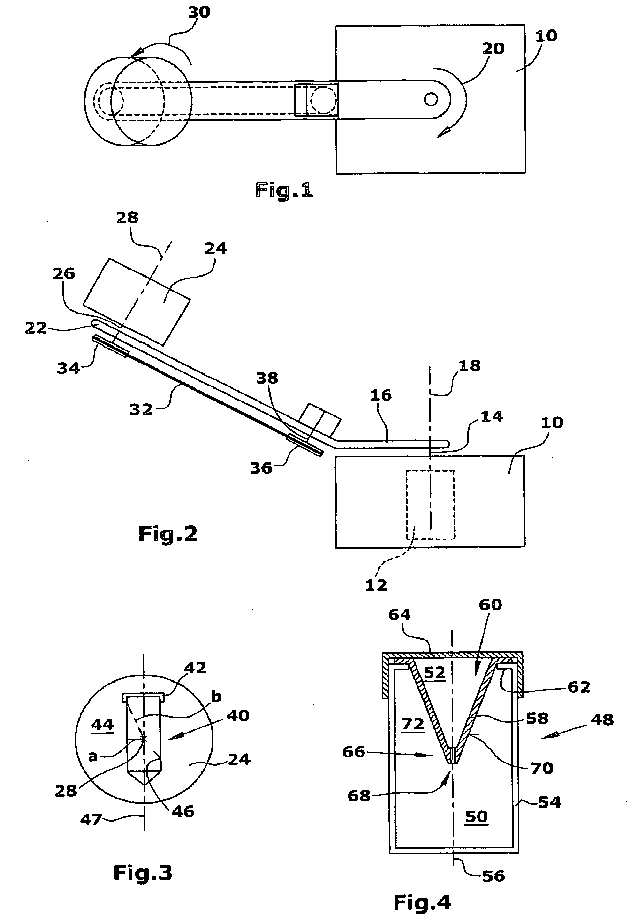

Die im Verfahren nach Ausführungsform (1) bis (7) und der erfindungsgemäßen Verwendung (12) verwendete duale asymmetrische Zentrifuge wird im folgenden anhand der Fig. 1 und Fig. 2 näher beschrieben. Sie weist ein Gehäuse bzw. einen Basiskörper 10 auf. In dem Gehäuse 10 ist eine erste Antriebseinrichtung 12 in Form eines Elektromotors vorgesehen. Durch die erste Antriebseinrichtung 12 wird eine Antriebswelle 14 angetrieben. Mit der Antriebswelle 14 ist ein Ausleger 16 verbunden, der somit mit Hilfe der ersten Antriebseinrichtung um eine erste Drehachse 18 gedreht werden kann. Mit Hilfe der ersten Antriebseinrichtung 12 erfolgt somit ein Drehen des vorzugsweise abgewinkelt ausgebildeten Auslegers 16, beispielsweise in Richtung eines Pfeils 20.

An einem von der Drehachse 18 beabstandeten Ende 22 des Auslegers 16 ist ein Behälter 24 vorgesehen. Der Behälter 24 ist über eine Welle 26 mit dem Ausleger 16 drehbar verbunden, so dass der Behälter 24 um eine zweite Drehachse 28, beispielsweise in Richtung eines Pfeils 30 drehbar ist. Zum Antrieb des Behälters 24 ist eine zweite Antriebseinrichtung 30 vorgesehen, die beispielsweise auf dem Ausleger 16 befestigt ist. Um die Einwirkung von Zentrifugalkräften auf die zweite Antriebseinrichtung 30 zu verringern, ist die zweite Antriebseinrichtung möglichst nahe an der ersten Drehachse 18 angeordnet. Der Antrieb erfolgt sodann über einen Keilriemen 32 sowie zwei entsprechende Keilriemenräder 34, 36, die mit der Welle 26 bzw. mit einer Motorwelle 38 verbunden sind.

Durch das gleichzeitige Drehen des Behälters 24 um die beiden Drehachsen 18 und 28 erfolgt ein Durchmischen von in dem Behälter 24 vorgesehenen Substanzen. Hierbei kann eine hohe Homogenität der Durchmischung erzielt werden. Vorzugsweise sind die beiden Drehachsen 18, 28 nicht nur in einem Abstand zueinander angeordnet, sondern weisen einen Winkel zueinander auf. Dieser kann durch Vorsehen eines abgewinkelten Auslegers 16 hervorgerufen werden. Ebenso ist es möglich, die Drehachse 28 in einem Winkel zum Ausleger anzuordnen. Der Winkel zwischen den beiden Drehachsen 18, 28 liegt vorzugsweise im Bereich von 0 bis 70°, besonders bevorzugt von 30 bis 70 °.

Das "Rückdrehverhältnis" bezeichnet das Verhältnis der vollen Umdrehungen um die beiden Drehachsen 18, 28 einer DAZ zueinander. Es ist die Zahl der Umdrehungen, die der Hauptrotor 16 um die Drehachse 18 vollführen muß, damit sich der Probenbehälter 24 einmal um die Drehachse 28 gedreht hat. Das Rückdrehverhältnis wird entsprechend als XX:1 angegeben und ist positiv, wenn sich der Probenbehälter 24 entgegengesetzt zum Hauptdreharm 16 dreht.

Die Drehzahl des Auslegers 16 beträgt vorzugsweise wenigstens 200 Umdrehungen pro Minute (rpm) und maximal 4000 rpm. Sie liegt bevorzugt im Bereich von 2000-3540 rpm. Die Drehzahl des Behälters 24 um die Drehachse 28 ist vorzugsweise so eingestellt, dass sich ein Rückdrehverhältnis zwischen 1:6 und 6:1 ergibt. Sie beträgt vorzugsweise wenigstens ein Fünftel, besonders bevorzugt wenigstens ein Drittel der Drehzahl des Auslegers 16, was einem Rückdrehverhältnis von 5:1 bzw. 3:1 entspricht. Ferner sind die Drehrichtungen 20, 30 vorzugsweise einander entgegengesetzt.

Es können auch in einem geeigneten Halter Gefäße bzw. Mischbehälter in den Behälter 24 eingesetzt werden, insbesondere Injektionsfläschchen, Glasgefäße oder Kunststoffgefäße wie z. B. Eppendorf-Gefäße. Der Behälter 24 fungiert dann als Aufnahmebehälter. Das Verfahren nach Ausführungsform (1) erlaubt den Einsatz der unterschiedlichsten Gefäße. Bevorzugt sind Gefäße aus Glas oder Kunststoff, deren Durchmesser 5-75 mm, besonders bevorzugt 9-75 mm beträgt. Neben handelsüblichen Injektionsfläschchen sind speziell Eppendorfgefäße mit einem Durchmesser von 9 bis 10 mm und Glasgefäße mit einem Durchmesser von 9 bis 40 mm bevorzugt. Weitere geeignete Spezialgefäße zur Verwendung in Ausführungsform (1) sind die speziellen Mischbehälter der Ausführungsform (10) und (11), wie sie in Fig. 3 und 4 beispielhaft beschrieben sind. Auch die Verwendung von Tuben und Spritzen als Mischbehälter ist möglich.

Um das Mischen weiter zu verbessern, können innerhalb des Behälters 24 oder innerhalb der darin angeordneten Gefäße Mischhilfen (synonyme Begriffe: Dispergierhilfe, Homogenisierhilfe) vorgesehen sein. Hierbei kann es sich um eine definierte Rauheit der Innenseite des Behälters 24/des Gefäßes handeln. Ebenso können stegförmige Mischhilfen innerhalb des Behälters 24/des Gefäßes angeordnet sein. Bevorzugt sind Gefäße, deren Innenfläche aufgerauht, genoppt oder geriffelt ist. Mischhilfe können des weiteren Glasperlen in verschiedenen Mengen und Größen sein, Flügel an der inneren Gefäßwand oder ein entsprechend geformter zentraler und/oder peripherer Gefäßeinsatz. Besonders bevorzugt als Mischhilfe sind Perlen aus Materialien wie Edelstahl, Achat, Zirkonoxid, Wolframcarbid, Teflon, Teflon mit Stahlkern, Polyamid, Sinterkorund, Siliziumnitrid und Glas. Ganz besonders bevorzugt sind Glasperlen und Stahlperlen, vor allem Glasperlen. Bevorzugter Durchmesser dieser Perlen ist ein Durchmesser von mindestens 0,4 mm, besonders bevorzugt von 0,5 bis 6 mm.

"Speedmixen" bedeutet im Kontext der vorliegenden Erfindung die Nutzung eines Speedmixers^{®} oder einer anderen DAZ, bevorzugt eines Speedmixers^{®} vom Typ DAC 150 FVZ der Firma Hauschild GmbH und Co KG, welcher ein Rückdrehverhältnis von ca. 2,8:1 oder 4,2:1, besonders bevorzugt 4,2:1 aufweist, oder einer dualen asymmetrischen Zentrifuge, welche ein ähnliches Rückdrehverhältnis aufweist.

im erfindungsgemäßen Verfahren (1) oder (2) und der erfindungsgemäßen Verwendung (12) sind Ansatzgrößen von wenigen Milligramm bis zu 3000 g (3 kg) möglich, bevorzugt sind Ansatzgrößen von 5 mg bis 150 g, ganz besonders bevorzugt von 20 mg bis 40 g.

Das Verfahren nach (1) oder (2) erfolgt bevorzugt mit einer DAZ, die zur Aufnahme von Reaktionsgefäßen der Größe von 0,1 bis 110 ml, bevorzugt von 0,1 bis 25 ml, besonders bevorzugt von 0,5 bis 10 ml, ganz besonders bevorzugt von 0,5 bis 2 ml geeignet ist.

Auf die zu mischenden, in dem Behälter 24 angeordneten Substanzen wird im Verfahren nach (1) oder (2) vorzugsweise eine Zentrifugalkraft von wenigstens 1,2 g, bevorzugt wenigstens 80 g, besonders bevorzugt wenigstens 300 g, ganz besonders bevorzugt von 550 bis 1000 g oder von 620 bis 1500 g ausgeübt. Die maximale Zentrifugalkraft kann jedoch bis zu 3000 g betragen, bevorzugt bis zu 2500 g.

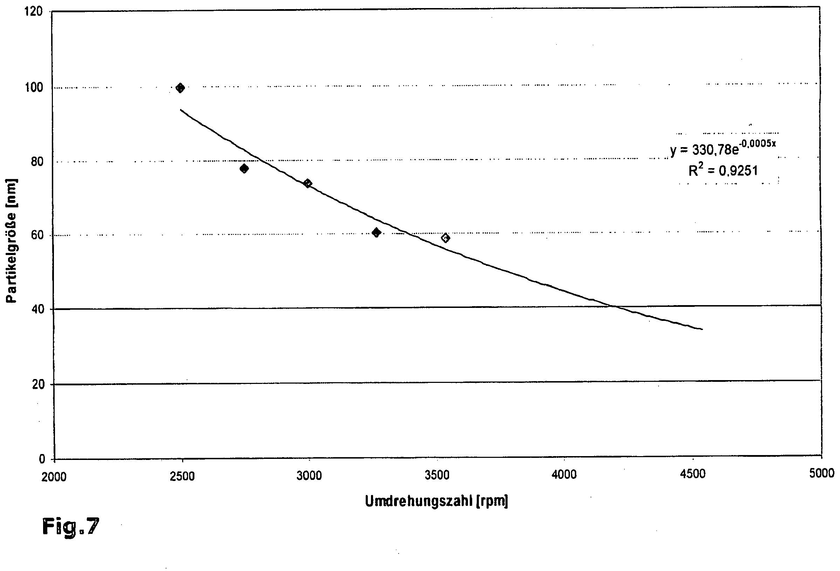

Die Vesikelgröße der Nanopartikel hängt unter anderem von der Geschwindigkeit der DAZ ab. Je höher die Geschwindigkeit (in rpm) bzw. die g-Zahl, desto kleinere Vesikelgrössen sind erreichbar (vgl. Bsp. 19). Relevante Geschwindigkeiten für die Herstellung insbesondere von kleinen Vesikeln mit einem Durchmesser von unter 100 nm liegen im Bereich über 2000 rpm. Bei weiterer Steigerung der Geschwindigkeit sollten Teilchengrößen im Bereich von 30 nm Durchmesser realisierbar sein, voraussichtlich bei einer Geschwindigkeit von 4000 bis 5000 rpm (Fig. 7).

Die Homogenisierung im Verfahren der Ausführungsform (2) erfolgt in einem besonders bevorzugten Aspekt bei 3540 rpm und/oder bei 550 bis 1000 g. Das Rückdrehverhältnis im Verfahren nach (2) ist in einem besonders bevorzugten Aspekt kleiner oder gleich 4,2:1, ganz besonders bevorzugt kleiner oder gleich 2,8:1, die Zentrifugationszeit beträgt von 30 s bis 1 h, bevorzugt von 1 bis 40 min, besonders bevorzugt von 3 bis 30 min, ganz besonders bevorzugt von 3 bis 20 min.

Ein möglicher Aspekt der Ausführungsformen (2) und (12) ist der Einsatz eines Speedmixers^{®} als duale asymmetrische Zentrifuge, besonders bevorzugt eines Speedmixers^{®} vom Typ DAC 150 FVZ der Firma Hauschild GmbH und Co KG, welcher ein Rückdrehverhältnis von ca. 2,8:1 oder 4,2:1, bevorzugt von 4,2:1 aufweist.

Im Verfahren nach (1) oder (2) weist die Lipidkomponente bevorzugt eines oder mehrere Amphiphile, Lipide, Detergentien auf, besonders bevorzugt mindestens ein Lipid ausgewählt aus Phospholipiden, Glycolipiden, Cholesterolen, Sphingolipiden, Polyethylenglykol-Lipidderivaten, kationischen Lipiden, Triglyceriden und Wachsen. Die wässerige Komponente ist bevorzugt Wasser oder eine wässerige alkoholische und/oder pufferhaltige Lösung. Eine oder beide der Komponenten können eine oder mehrere funktionelle Substanz(en) enthalten, die lipophil oder hydrophil sind. Diese Substanzen sind bevorzugt ausgewählt aus der Gruppe der Wirkstoffe und der diagnostisch, biosynthetisch oder für die chemische Synthese relevanten Verbindungen. Als Hilfsstoffe können des Weiteren Detergentien und/oder Emulgatoren zugesetzt werden.

Das Verfahren der Ausführungsform (1) und (2) kann zur Herstellung von lipidbasierten Nanopartikeln verwendet werden, die entweder mindestens einen Wirkstoff und/oder eine Substanz enthalten, welche in der Diagnostik oder chemischen Synthese relevant ist, oder leer sind (also keine solche Substanz enthalten).

Das Verfahren nach Ausführungsform (1) kann wie folgt durchgeführt werden: Die für die jeweilige Formulierung vorgesehenen Lipide (und ggf. Hilfsstoffe) sowie ggf. ein oder mehrere Wirkstoff(e) werden trocken in ein geeignetes Gefäß (Mischbehälter) gegeben, anschließend wird die wässerige Komponente hinzugegeben, die ggf. einen oder mehrere Wirkstoff(e) und weitere Hilfsstoffe enthält, und die Mischung "gespeedmixt". Es kann auch eine als feste Lösung vorliegende entsprechende (Lipid)Mischung, ggf. enthaltend einen oder mehrere Wirkstoff(e), direkt als Lipidkomponente eingesetzt werden. Alternativ kann die Zugabe der wässerigen Komponente auch dosiert während der Homogenisierung in der DAZ erfolgen. Auch die Vorlage der wässerigen Komponente und die Zugabe der Lipidkomponente, ggf. in einem geeigneten Lösemittel, ist möglich.

Wenn Lipidmischungen eingesetzt werden, so können diese als Einzelkomponenten oder als fertige Lipidmischungen ("feste Lösungen", also Mischkristalle aus den einzelnen Lipiden) für das Verfahren nach (1) eingesetzt werden (Bsp. 3). In beiden Fällen werden lipidbasierte Nanopartikel erhalten.

Durch die hohe innere Reibung beim Speedmix-Prozess sowie die stark erhöhten Kontakt-Ereignisse zwischen den entstehenden Partikeln und den einzuschließenden Substanzen entstehen gemäß Verfahren (1) in kurzer Zeit wirkstoffbeladene Nanopartikel. Wird die Menge der zugegebenen wässerigen Komponente niedrig gehalten (was zu relativ hohen Lipid-Konzentrationen führt), dann entsteht eine viskose Lipidpaste aus Nanopartikeln, wie sie z. B. aus der WO 96/005808 für Liposomen-Gele (VPG) bekannt ist. Dieses Vorgehen hat in bestimmten Fällen den Vorteil, dass - bedingt durch die hohe Lipidkonzentration und damit eine hohe innere Reibung - besonders effizient kleine Partikel gebildet werden und im Fall von Liposomen eine besonders hohe Einschlusseffizienz für wasserlösliche Substanzen erzielt werden kann (hoher Anteil eingeschlossener wässeriger Phase). Optional kann nach Ende der Vesikelbildung nicht eingeschlossener Wirkstoff abgetrennt werden. Wird nach der Vesikelbildung weiteres wässeriges Medium zugegeben und (kurz) gespeedmixt, dann wird die entstandene hochkonzentrierte Dispersion redispergiert (verdünnt), so dass im Fall von Liposomen eine normale liposomale Dispersion entsteht, die aber immer noch eine hohe Einschlusseffizienz aufweist. Die vorliegende Erfindung umfasst somit auch die Verdünnung einer bereits vorgefertigten Dispersion lipidbasierter Nanopartikel, bevorzugt einer Liposomendispersion, besonders bevorzugt eines VPG, durch Einarbeiten einer (zusätzlichen) wässerigen Komponente zwischen die Nanopartikel mit Hilfe einer DAZ (Redispersion). Die lipidbasierten Nanopartikel können dabei bereits einen oder mehrere Wirkstoff(e) enthalten. Auch die Redispersion von Cremes und Dispersionen mit festen Partikeln, insbesondere SiO₂-Partikeln, ist möglich (Bsp. 9). Hierbei wird das wässerige Medium entweder in einem oder mehreren Schritten direkt zur vorhandenen Dispersion lipidbasierter Nanopartikel gegeben oder mit Hilfe eines Mischbehälters der Ausführungsform (11) während des Speedmixens appliziert. Besonders vorteilhaft ist dieses erfindungsgemäße Verfahren einsetzbar zur Redispersion von Gemcitabine-enthaltenden VPG (hergestellt durch Hockdruckhomogenisation und anschließendes passive loading), um eine injizierbare liposomale Dispersion zu erhalten (Bsp. 9A).

Das erfindungsgemäße Verfahren erlaubt einfaches steriles Arbeiten: lediglich das Abfüllen der Mischungsbestandteile in sterile Gefäße muss unter sterilen Bedingungen erfolgen. Nach Verschließen der Gefäße kann das Speedmixen in unsterilen Räumen stattfinden. Des Weiteren ist auch der Einsatz gefährlicher Substanzen, insbesondere von Zytostatika, radioaktiven Verbindungen (speziell radioaktiven Lipiden und Wirkstoffen) zur Herstellung der Nanopartikel vergleichsweise gefahrlos möglich, da die Stoffe sich während des Vermischungsvorgangs in verschlossenen Gefäßen befinden. Die Kontaminationsgefahr für Mensch und Maschine ist also bei Einsatz des Verfahrens sehr gering.

Mit dem erfindungsgemäßen Verfahren (1) und (2) können hohe Einschlusseffizienzen erreicht werden. Das erfindungsgemäße Verfahren erlaubt darüberhinaus eine schonende Behandlung der Probe und kann die Entstehung von unerwünschten Bestandteilen deutlich reduzieren. So zeigt ein Vergleich der Entstehung von Lyso-PC bei der Herstellung von Gemcitabin-enthaltenden Liposomen mit der DAZ-Technik mit derjenigen bei Herstellung vergleichbarer Liposomen durch passives Loading von mit Hilfe der Hochdruckhomogenisation hergestellten Liposomen eine signifikant geringere Menge von Lyso-PC bei Verwendung der DAZ-Technik (Bsp. 5D).

Das erfindungsgemäße Verfahren nach (1) und (2) ist hinsichtlich der Verwendung einer Mischhilfe, der Zentrifugiergeschwindigkeit, des Durchmessers des verwendeten Gefäßes, der Zentrifugierzeit und der Temperatur variierbar und eignet sich somit zur gezielten Herstellung von lipidbasierten Nanopartikeln in verschiedenen Partikelgrößen und Mengen (vgl. Bsp. 1 und 2). Bevorzugt haben die Nanopartikel eine Partikelgröße von 5 bis 1000 nm, besonders bevorzugt von 15 bis 200 nm, ganz besonders bevorzugt von 20 bis 150 nm. Wenn ein Nanopartikel definitionsgemäß keine Größe von 1000 nm haben darf, so bedeutet "bis 1000 nm" in diesem Kontext "bis zu 1000 nm", also kleiner als 1000 nm. Durch Variation der variablen Parameter können je nach Wunsch aus ein und derselben Ausgangslipidmischung ganz gezielt unterschiedliche liposomale Formulierungen hergestellt werden. Zudem ist es möglich, auch in Gefäßen mit geringem Durchmesser zu speedmixen, so dass auch Laborstandard-Gefäße wie Eppendorfgefäße^{®} etc. für ein Screening oder für Aufgaben im Bereich der Molekularbiologie eingesetzt werden können. Insbesondere bei einer Erhöhung der DAZ-Zentrifugiergeschwindigkeit und/oder Verkleinerung des Rückdrehverhältnisses ist bei kleinen Gefäßdurchmessern eine wesentliche Reduktion der Vesikelgrössen und eine Verringerung der notwendigen Zentrifugierzeit zu erwarten.

Mit Hilfe der erfindungsgemäßen Verfahren lassen sich Liposomen-Gele und (nach Redispergierung) liposomale Dispersionen herstellen, deren Eigenschaften den Eigenschaften vergleichbar zusammengesetzter Liposomen-Gele und Dispersionen aus Hochdruckhomogenisation ähneln (Bsp. 1, Fig. 5). Aufgrund der geringen Anzahl von Partikeln > 1 µm sind nach dem erfindungsgemäßen Verfahren hergestellte Produkte für die parenterale Applikation geeignet (siehe Vergleich Nutriflex^{®} in Bsp. 1).

Zur Herstellung von Liposomen der Ausführungsform (3) werden als Lipidkomponente membranbildende Lipide (auch Amphiphile genannt) eingesetzt. Ihre Herkunft (synthetisch oder aus natürlichen Quellen isoliert) ist für ihren erfindungsgemäßen Einsatz irrelevant. Besonders bevorzugt sind Phospholipide, Cholesterol und kationische Lipide, vor allem Phosphatidylcholin, Phosphatidylglycerin, Phosphatidylserin, Phosphatidylinositol, Phosphatidylethanolamin sowie Sphingomyelin. Ganz besonders bevorzugt ist Phosphatidylcholin (vgl. Bsp. 1 bis 9, 11, 13, 16). Die Fettsäurekohlenwasserstoffketten der Phospholipide können gesättigt oder ungesättigt und gleich oder unterschiedlich lang sein, bevorzugt sind Kettenlängen von C12 bis C20, besonders bevorzugt Kettenlängen von C14 bis C18. Die Auswahl von Phospholipiden mit bestimmten Fettsäurekompositionen ist z. B. für den Aufbau thermosensitiver Liposomen relevant. Liposomen können auch sog. Lyso-Phospholipide in ihren Membranen enthalten, die sich von Phospholipiden durch das Fehlen einer Fettsäure in der sn-2-Position unterscheiden. Weitere Phospholipide, aus denen bevorzugt Liposomen aufgebaut werden, sind Ceramide, Kardiolipin, Tetraetherlipide und Etherphospholipide. Bei letzteren sind eine oder zwei der Esterbindungen der Phosphophlipide durch Etherbindungen ersetzt. Für den Aufbau pH-sensitiver Liposomen hat sich die Kombination von DOPE und CHEMS bewährt. Liposomen, die besonders rasch von sPLA2 abgebaut werden können, bestehen z. B. aus Phosphatidylcholin, einer Stealth-Komponente (zum Schutz vor RES-uptake) und einem negativ geladenen Phospholipid.

Eingesetzt werden können auch handelsübliche Gemische von Phospholipiden oder Fraktionen solcher Gemische. Ein Beispiel ist das sog. Lecithin, das mindestens 20 % Phosphatidylcholin enthalten muß.

Eine besonders wichtige Lipidkomponente zum Aufbau von Liposomen ist Cholesterol.

Als Lipidkomponente einsetzbar sind auch die sog. Stealth-Komponenten, mit denen Liposomen vor der Aufnahme durch Zellen des RES geschützt werden. Dies können z. B. Phospholipide sein, die an ihrem polaren Ende Polyethylenglycolketten oder Polyglycerole tragen.

Es können auch funktionalisierte Lipide eingesetzt werden, mit denen z. B. Proteine oder Peptide an Liposomen gebunden werden können (Kopplungslipide). Ein Beispiel sind thiolreaktive Maleimidgruppen, die mit unterschiedlich langen PEG-Spacern an membranbildene Lipidkomponenten angebunden sind. Auch nichtkovalente Kopplungsprinzipien spielen eine Rolle, ein Beispiel wäre Avidin-Gruppen, die an membranbildenen Lipidkomponenten gebunden sind.

Mögliche Lipidkomponenten sind auch membranbildende Lipide, die chemische Strukturen mit biologischer Funktion an ihrem hydrophilen Ende tragen. Beispiele für derartige chemische Strukturen sind bestimmte Peptidsequenzen (z. B. für die Bindung an Zellen) oder Folsäure (für Bindung an den Folsäure-Rezeptor).

Ferner können als Lipidkomponenten auch andere membranbildende Amphiphile eingesetzt werden wie beispielsweise Blockpolymere, Alkylester, -ether, -amide von Zuckern, Diolen und Polyolen, Aminen, Aminosäuren, Peptiden und Zuckern und Cholesterol.

Weiterhin werden für die Herstellung von Liposomen oft kationische Lipide als Lipidkomponente eingesetzt. Diese können sein DOTAP, DOTMA, DC-Chol, DOSPER, DMRIE, DAC-30, DOGS, DORI, SpdC, SAINTS, oder aber auch Strukturen, die in EP 03005513.1 beschrieben sind, sowie die N,N,N-trialkylamino-2,3-propandiole, (WO 03/030945). Wichtige nichtkationische Lipidkomponenten bei der Herstellung kationischer Liposomen sind DOPE und Cholesterol.

Für die Herstellung von Liposomen gemäß Ausführungsform (3) kann die Lipidmischung so gewählt werden, dass die Liposomen pH-sensitiv (z. B. durch DOPE/CHEMS(Cholesterol-hemisuccinat)-Mischungen) oder empfindlich gegen erhöhte Temperaturen (Temperatur-sensitive Liposomen) werden. Die Mischung kann auch so gewählt werden, dass die Liposomen besonders rasch von PLA2 abgebaut werden können (PLA2-sensitive Liposomen).

Weiterhin können Liposomen sterisch stabilisiert werden, z. B. durch Einbringen von PEG-Ketten-enthaltenden Lipiden oder von Lipiden, die Polyglycerole als Kopfgruppe tragen. Dadurch können die Liposomen vor einer zu raschen Aufnahme durch Zellen des RES (retikulo-endotheliales System) geschützt werden.

Es können auch Lipidkomponenten eingebracht werden, die im lipophilen Teil oder im hydrophilen Teil einen Ruoreszenzmarker oder ein Spin-Label tragen, ebenso Lipide mit einer radioaktiven Markierung.

Liposomen der Ausführungsform (3) können neutral, anionisch oder kationisch sein (Tab. 3). Sie können unabhängig von der Ladung mit ein und demselben Wirkstoff beladen werden (Bsp. 5C), was dessen Transport zu unterschiedlichen Zielregionen einer Zelle oder eines Zellverbunds, eines menschlichen oder tierischen Körpers, Organs oder Körpergewebes, einer Pflanze oder eines Pflanzenteils, oder einer Reaktionsmischung ermöglicht. Von besonderem Interesse sind Liposomen mit kationischen Lipiden (z. B. DOTAP), die besonders bevorzugt an aktiviertem Endothel binden (z. B. Tumorendothelien), negativ geladene Liposomen, die an das RES binden, und neutrale Liposomen, die sich über den EPR-Effekt in Tumorgewebe einlagern können oder als systemisches Depot Wirkstoffe vor ihren raschen Abbau im Körper schützen können. Darüberhinaus können die genannten Liposomen als lokales Depot für eine langsame Wirkstoffabgabe in den Körper eingesetzt werden. Weiterhin können Lipide eingesetzt werden, mit deren Hilfe weitere Substanzen an die Liposomen gebunden werden können. Dies sind insbesondere sog. Kopplungslipide, mit deren Hilfe man z. B. Antikörper, Antikörperfragmente und andere Proteine, aber auch Peptide, an die Oberfläche von Liposomen binden kann. Dabei kann die Bindung chemisch erfolgen (z. B. via Maleimidgruppen an den Kopplungslipiden und Thiolgruppen auf dem Antikörper/Protein/Peptid). Die reaktiven Gruppen können auch durch längere Spacer mit den Lipiden verbunden sein (z. B. PEG-Ketten). Darüberhinaus kann die Bindung von Substanzen an die Oberfläche von Liposomen auch durch nicht kovalente Prinzipien erfolgen, wie z. B. durch Avidin-Streptavidin-Kopplung.

Von besonderem Interesse sind auch Lipide, die bereits im polaren Kopf bestimmte Peptide oder bestimmte Moleküle tragen, mit denen Bindung an bestimmte Zellen oder Epitope möglich ist (z. B. Folsäure-tragende Lipide; Bindung an Folsäu rerezeptor).

Für den Einschluss von RNA ist es von Vorteil, RNAse-freie Lipide einzusetzen.

Die Konzentration der Lipidkomponente in den Liposomen gemäß (3) beträgt von 1 bis 600 mM, bevorzugt von 20 bis 600 mM, ganz besonders bevorzugt von 200 bis 600 mM.

Die folgenden Verbindungen können in Liposomen gemäß Ausführungsform (3) eingeschlossen werden (Bsp. 5):
- Proteine (z. B. Hämoglobin, Albumin; Bsp. 5A)
- Vitamine (z. B. Tocopherol) und Antioxidantien
- Enzyme (z. B. Luciferase) und Enzyminhibitoren (z. B. sPLA2-Inhibitor, Bsp. 4C)
- Zytokine
- Nukleinsäuren, vor allem RNA (Bsp. 5C), DNA, Plasmid-DNA, Antisense-DNA und -RNA, siRNA, dsRNA
- Peptide (z. B. PSA-Peptide)
- Viren (z. B. onkolytische Viren)
- Cyclodextrine, auch solche, die Wirkstoffe enthalten
- Zell- oder Gewebelysate und deren Fraktionen
- Tumorlysate und deren Fraktionen
- Magnetische Nanopartikel
- Ionen, ATP, Salze (z. B. Kupfersalze)
- SiO₂-Partikel (Bsp. 5F), z. B. für Cremes
- Radioaktive Substanzen, fluoreszenzgelabelte Substanzen (Bsp. 4B)
- Niedermolekulare Verbindungen (z. B. Trypanblau; Bsp. 5B)
- Wirkstoffe und Diagnostika, u.a. Antiöstrogene, Antibiotika, Analgetika, Antirheumatika, Antiallergika, Antibiotika (u.a. Amphotericin B, Cyclosporin, Buparvaquon und Atovaquon), Antiinfektiva, antiparasitische und antiinflammatorische Wirkstoffe, Chemotherapeutika, Antiepileptika, Antimykotika, Corticoide, Dermatika, Diagnostika, Hämostypika, Antihämorrhagika, Hypnotika, Sedativa, Hormone, Peptide, Hormoninhibitoren, Immuntherapeutika, Zytokine, Lokalanaesthetika, Migränemittel, Narkotika, Ophthalmika, Psychopharmaka, Schilddrüsentherapeutika, Sera, Immunglobuline, Impfstoffe, Metastasehemmer, Proteine, Prostaglandine, Proteinkinase-Inhibitoren und andere Inhibitoren wichtiger zellulärer Signalwege (insbesondere im Tumorgeschehen), Metallocene, Zytostatika, Lipid-Wirkstoff-Konjugate; bevorzugte Wirkstoffe sind Antibiotika (v.a. Anthrazykline), Antiparasitika, Zytostatika, Antiinflammativa, Antiinfektiva und Substanzen, mit denen sich üblicherweise keine lagerfähigen Liposomen herstellen lassen.

Bevorzugt erfolgt der Einschluss in Liposomen durch Zugabe der Verbindungen in wässeriger Lösung/Dispersion zu einer Lipid(mischung) und anschließende Anwendung des Verfahrens (1) oder (2). Die Verbindungen können ungeachtet ihrer physikalischen Eigenschaften rasch und mit hoher Effizienz eingeschlossen werden.

Bevorzugt wird das erfindungsgemäße Verfahren (1)- zum Einschluss von Wirkstoffen in Liposomen angewandt.

Insbesondere geeignet ist das erfindungsgemäße Verfahren (1) und (2) zum Einschluss empfindlicher und/oder kurzlebiger Substanzen in Liposomen, mit denen sich üblicherweise keine lagerfähigen Liposomen herstellen lassen bzw. Substanzen, die sich negativ auf die Stabilität der Lipidkomponente auswirken. Hierzu zählen vor allem :
- hydrolyseempfindliche Verbindungen und Wirkstoffe, bevorzugt Alkylantien incl. Bendamustin (Bsp. 5E), Cyclophosphamid, Mafosfamid und Platinverbindungen wie Cis-Platin oder Oxaliplatin
- kurzlebige Substanzen wie die in der Diagnostik eingesetzten radioaktiv markierten Verbindungen oder Verbindungen, die für die Positronen-Emissions-Tomographie (PET)-Diagnostik eingesetzt werden
- Substanzen, die leicht über (Liposomen)-Membranen diffundieren und daher meist via passive loading in die Liposomen eingeschlossen werden, bei diesem Prozess aber selbst instabil sind oder eine Instabilität der Lipidkomponente verursachen, einschließlich der Zytostatika Gemcitabine (Bsp. 5D), Vincristin, Vindesin, Vinblastin, 1-β-D-Arabinofuranosylcytosin (Ara-C).

Hydrolyseempfindliche Verbindungen kommen bislang oft nur lyophylisiert in den Handel, nicht jedoch als liposomale Formulierung. Ein wesentlicher Aspekt der Erfindung ist die Verwendung hydrolyseempfindlicher Zystostatika, mit denen sonst keine liposomalen Formulierungen hergestellt werden können, einschließlich Alkylantien, Platin-Verbindungen, Bendamustin und Mafosfamid. Die rasche erfindungsgemäße Herstellung der Nanopartikel und schonende Verwendung durch rascher (klinisches) Redispergieren stellt sicher, dass auch solche Wirkstoffe in eine Dispersion eingearbeitet werden können.

Das Verfahren nach (1) oder (2) ist ferner geeignet, Wirkstoffe sehr rasch, effektiv und gleichmäßig in ein vorgefertigtes Liposomen-Gel wie z. B. ein VPG oder SLN einzuarbeiten und kann damit sowohl zur Einarbeitung wasserunlöslicher Wirkstoffe als auch zur Vorbereitung des passiven Loadings verwendet werden (vgl. Bsp. 6, Bsp. 8). Von den wasserunlöslichen Wirkstoffen ist Amphothericin B bevorzugt.

Das Einarbeiten von Wirkstoffen als Voraussetzung für das passive loading wurde bisher durch Schütteln der VPG-Masse mit der Wirkstofflösung oder durch Verrühren mit einem Rührer oder Spatel bewerkstelligt (EP 1087752). Ziel ist beim Einarbeiten eine möglichst gleichmäßige Verteilung des Wirkstoffs zwischen den Vesikeln. Dieser Einarbeitungsschritt ist insofern kritisch, als er unter sterilen Bedingungen erfolgen sollte, da nach dem Einarbeiten des Wirkstoffs in das VPG üblicherweise kein Sterilisationsschritt mehr erfolgt (bzw. bei Gemcitabine als Wirkstoff nicht erfolgen darf, da Gemcitabine unter Autoklavierbedingungen die Phospholipidhydrolyse katalysiert). Auch durch Schütteln lässt sich der Wirkstoff gleichmäßig im VPG verteilen; das hat den Vorteil, das dies im geschlossen Gefäß und damit steril durchgeführt werden kann. Dazu ist eine Schüttelhilfe (z. B. Glasperlen) notwendig, die bereits beim Einfüllen des VPG in das Gefäß zugesetzt wird. Der Schüttelvorgang kann dann auf einem Laborschüttler (z. B. Typ Mikrodismembrator, Braun Melsungen) durchgeführt werden. Er benötigt allerdings lange Zeit und ist aufgrund der Geräuschentwicklung störend für das Labor/Apotheken/Stationspersonal. Der Vorgang sollte überdies überwacht werden, da aufgrund der bewegten Massen Schüttler leicht "wandern" und vom Tisch fallen können.

Demgegenüber hat das erfindungsgemäße passive Loading-Einarbeiten den Vorteil, dass das Einarbeiten vergleichsweise schnell erfolgt (Bsp. 8) und somit sehr schonend und zugleich zeitsparend ist.

Ein Aspekt des Verfahrens gemäß (3) ist folglich die Einarbeitung von Wirkstoffen in vorgefertigte VPG zur Vorbereitung des passiven Loadings. Es eignet sich zur Herstellung von wirkstoffhaltigen VPG für klinische Anwendungen, für präklinische Untersuchungen (z. B. Tierexperimente) und für *in vitro*-Untersuchungen (biologisch oder biophysikalisch; Stabilitätsuntersuchungen). Das Verfahren ist auch für Screenings geeignet, bei denen z. B. eine für eine Anwendung besonders geeignete Wirkstoff/Lipidkomponenten-Kombination oder ein optimales Verhältnis zwischen Lipidkomponente und Wirkstoff erarbeitet werden soll. Des Weiteren ist das Verfahren unter sterilen Bedingungen durchführbar, was vor allem für die Herstellung von wirkstoffhaltigen VPG zur Untersuchung an lebenden Organismen notwendig ist. Insbesondere kann dieses Verfahren zur Einarbeitung von empfindlichen Wirkstoffen in Lipidvesikel verwendet werden, vor allem von Gemcitabine, Vincristin, Vindesin und Platinverbindungen (s. oben). Die Wirkstoffe können dazu fest oder in Lösung eingesetzt werden, wobei der Einsatz als Lösung bevorzugt ist.

Wirkstoffhaltige Liposomen werden oft durch die sog. Injektionsmethode (Injektionstechnik) hergestellt, da dieser Prozess großtechnisch durchführbar ist. Insbesondere ist diese Methode von Vorteil, wenn lipophile Wirkstoffe, die sich üblicherweise in die Membran von Liposomen ein- oder anlagern, in Liposomen eingeschlossen werden sollen. Beim Injektionsverfahren wird die Lösung von Lipidkomponente (und optional auch des Wirkstoffes) in einem organischen Lösungsmittel (vorzugsweise Ethanol oder Diethylether) in eine gut gerührte wässerige Phase "injiziert". Wegen des erforderlichen Rührens wird auch im Labor meist in einem etwas größerem Maßstab, vorzugsweise mit wässerigen Volumina von mindestens 10 ml gearbeitet. Um die Injektionsmethode auch zum Screening z. B. von geeigneten Wirkstoff/Lipidkomponenten-Kombinationen zu verwenden, wäre eine Ausführung im Milliliter-Maßstab und geringer wünschenswert. Dies wird durch die Anwendung des Verfahrens (1) erreicht. Hierzu wird der Mischbehälter der Ausführungsform (11) eingesetzt. In einer der Kammern befindet sich dabei die wässerige Lösung, in einer zweiten Kammer das organische Lösungsmittel mit der Lipidkomponente. Durch die Zentrifugalkräfte in der DAZ wird das Volumen der oberen Kammer während des Speedmixens in die untere Kammer gedrückt. Bei einer Anordnung, die der klassischen Injektionsmethode gleicht und bei der das organische Lösungsmittel in die wässerige Phase injiziert wird, bilden sich so z. B. Liposomen (Bsp. 11C). Interessanterweise kann dieser Prozess auch umgekehrt durchgeführt werden, d. h. die wässerige Phase wird in die organische Phase injiziert. Bei der Zugabe von einer geringen Menge an wässeriger Phase während des Speedmixens mit Hilfe des Mehrkammersystems wird zunächst eine viskose, Formulierung erreicht, bei nochmaliger Zugabe von wässeriger Phase und nochmaligem Speedmixen ergibt sich eine einheitliche liposomale Formulierung (Bsp. 1 A).

Die Injektionsmethode ist jedoch auch ohne Applikator oder ein Mehrkammersystem der Ausführungsform (11) durchführbar. Hierzu wird zur organischen Phase, welche die Lipidkomponente enthält, die wässerige Phase in kleinen Schritten zugefügt und zwischendurch gespeedmixt (Beispiel 11 B). Dabei entsteht zunächst eine viskose, abschließend bei vollständige Zugabe der wässerigen Phase eine einheitliche liposomale Dispersion mit vergleichbaren Vesikelgrößen wie bei Verwendung eines Mischbehälters der Ausführungsform (11) (Bsp. 11A). Die Injektionsmethode ist insbesondere für Screenings geeignet, v.a. zum Einschluss lipophiler Substanzen in Liposomen und in kleinen Ansatzgrößen und/oder automatisiert.

Das Verfahren gemäß Ausführungsform (1), die Mischvorrichtung gemäß (10) und der Mischbehälter gemäß (11) kann auch genutzt werden, um bereits hergestellte lipidbasierte Nanopartikel oder partikelhaltige Mischungen zu redispergieren und/oder zu verdünnen. Dies gilt auch für viskose Mischungen und Mischungen, die Feststoffpartikel enthalten (Bsp. 9). Die Zugabe des Verdünnungsmediums kann dabei wahlweise direkt oder mit Hilfe eine Applikators erfolgen. Bevorzugt wird hierfür ein Mischbehälter wie in Ausführungsform (11) oder ein vergleichbar aufgebautes Mischgefäß als Applikator eingesetzt. Dieser Aspekt der Erfindung ist insbesondere von Bedeutung für die Vorbereitung von wirkstoffhaltigen VPG (z. B. Gemcitabine- oder Vincristine-enthaltenden VPG) für die Applikation (z. B. am Patienten). Hierzu wird i.d.R. eine injizierbare Formulierung benötigt, wozu die VPG-Formulierung redispergiert/verdünnt werden muß. Dies gelingt einfach durch das hier beschriebene Verfahren und hat hinsichtlich der Durchführungsgeschwindigkeit, der Sicherheit des Verfahrens und der Einfachheit deutliche Vorteile gegenüber dem bisher eingesetzten Schüttelverfahren.

Bedeutung hat die erfindungsgemäße Redispersion insbesondere auch für die Anwendung auf SiO₂-Dispersionen. Siliziumdioxid-Dispersionen werden traditionell eingesetzt zur Vorbeugung von brüchigen Fingernägeln und Haaren, zur Kräftigung des Bindegewebes und in verschiedenen anderen Anwendungen. Eine typische Formulierung ist hier z. B. einer Dispersion von 2 bis 3 g Kieselsäureanhydrid/100 ml Wasser. Die in solchen Formulierungen vorhandenen Siliziumdioxid-Kristalle neigen zur Aggregation, was u.a. dadurch gezeigt werden kann, das eine Größenbestimmung einer in Wasser aufgeschlämmten Siliziumdioxid-Dispersion mit Hilfe der Photonenkorrelationsspektroskopie (PCS) aufgrund der vorhandenen großen Teilchen nicht möglich ist. Um die offensichtlich entstandenen nicht kovalenten Aggregate von Siliziumdioxid-Partikeln zu trennen, kann das Verfahren nach (1) verwendet werden (vgl. Bsp. 9E).

Überraschenderweise ist das Verfahren nach (1) sogar geeignet, die Hochdruckhomogenisation zu verbessern. Um VPG und andere Dispersionen durch Hochdruckhomogenisation herzustellen, werden die Lipide/Lipidmischungen oder andere Substanzen üblicherweise zuvor mit der notwendigen Menge wässerigem Medium rehydratisiert/verrührt. Bei diesem einfachen Vermengen von Substanz/Lipid und wässerigem Medium entsteht keine homogene Dispersion. Aus diesem Grunde verlaufen die ersten Zyklen bei der Hochdruckhomogenisation oft ungleichmäßig. Erst nach etwa drei Zyklen erscheint die Mischung homogen. Eine deutliche Erleichterung und auch Verbesserung des Hochdruckhomogenisationsverfahrens kann erreicht werden, wenn die Substanz bzw. die Mischung aus Lipid und wässerigem Medium vor der Hochdruckhomogenisation in einer DAZ behandelt wird und dadurch bereits bei Beginn der Hochdruckhomogeniation homogen ist. Dadurch kann dieser Prozess beschleunigt werden, es können zumindest die ersten Zyklen eingespart werden. Dies wurde am Beispiel der Herstellung eines VPG gezeigt (Bsp. 13).

Für die Ausführungsformen (1), (2), (3), (7), (8) und (15) der Erfindung sind als lipidbasierte Nanopartikel VPG besonders bevorzugt.

Die durch das Verfahren gemäß Ausführungsform (4) herstellbaren SLN bestehen aus mindestens einem festen Lipid, bevorzugt einem Triglycerid oder Wachs, und in einem bevorzugten Aspekt zusätzlich aus mindestens einem Emulgator/-Stabilisator oder Tensid. Zur Herstellung gemäß (4) werden prinzipiell das feste Lipid und optional ein geeigneter Emulgator/Stabilisator in einer relativ geringen Menge der wässerigen Komponente auf eine geeignete Temperatur erwärmt. Dies ist diejenige Temperatur, bei der das feste Lipid in flüssiger Form vorliegt und der Emulgator/Stabilisator seine Emulgationseigenschaften entfalten kann. Vorzugsweise liegt diese Temperatur über der Raumtemperatur. Die entstandene - vorzugsweise viskose - Mischung wird gespeedmixt. Nachdem sich die gewünschte SLN-Dispersion gebildet hat, wird durch Zugabe weiterer wässeriger Komponente und kurzem Speedmixen die SLN-Dispersion auf eine gewünschte Konzentration eingestellt und bei niedrigen Temperaturen belassen, bis die SLN ausgehärtet sind (Bsp. 15). Für die Herstellung von SLN werden bei RT feste Lipide verwendet und der Anteil der Lipidkomponente ist in der Regel hoch. Das bedingt die Durchführung der SLN-Herstellung bei Temperaturen, die bevorzugt mindestens der Schmelztemperatur der Lipidkomponente entsprechen und daher in der Regel deutlich über Raumtemperatur (RT) liegen.

### Beispiele für bei RT feste Lipide, die für SLN-Herstellung geeignet sind:

Karnaubawachs, Hydroxyoctacosanylhydroxysterat, chinesisches Wachs, Cetylpalmitat, Bienenwachs und ähnliche Wachse. Weitere Beispiele für feste Lipide sind C20-40 Di- und Triglyceride mit gesättigten und ungesättigten Fettsäuren, C20-40 Fettalkohole, C20-40 Fettamine und ihre Verbindungen sowie Sterole.

### Beispiele für Deteraentien/Tenside/Emulgatoren/Stabilisatoren in SLN:

- Lecithine, Polyethoxysorbitanester (Tween^{®}-Typen), wie beispielsweise Laurat (Tween^{®} 20/21), Palmitate (Tween^{®} 40), Stearate (Tween^{®} 60/61), Tristearate (Tween^{®} 65). Oleate (Tween^{®} 80/81) oder Trioleate (Tween^{®} 85), Natriumglykocholat und Natriumlaurylsulfat (SDS) sowie die Sorbitanfettsäureester (Span-Typen) und TritonX^{®} 100.
- Sterisch stabilisierende Substanzen wie Poloxamere und Poloxamine, ethoxylierte Sorbitanfettsäureester, besonders Polysorbate (z. B. Polysorbat 80 bzw. Tween^{®} 80), ethoxylierte Mono- und Diglyceride, ethoxylierte Lipide, ethoxylierte Fettalkohole oder Fettsäuren, Ether und Ester von Zuckern oder von Zuckeralkoholen mit Fettsäuren oder Fettalkoholen (z. B. Saccharose(di)stearat, -laurat, -octanoat, -palmitat, -myristat)
- Alkohole und Alkoholderivate mit lipophilen Regionen wie z. B. Tyloxapol
- Geladene ionische Stabilisatoren wie Diacetylphosphate, Phosphatidylglycerin, Lecithine unterschiedlicher Herkunft (z. B. Ei- oder Sojalecithin), chemisch modifizierte Lecithine (z. B. hydrierte Lecithine), synthetisch hergestellte Lecithine, genauso Phospholipide und Sphingomyeline, Mischung von Lecithinen mit Phospholipiden, Sterolen (z. B. Cholesterol und Cholesterolderivate, Stigmasterin) und ebenfalls gesättigte und ungesättigte Fettsäuren, Natriumcholat, Natriumglykocholat, Natriumtaurocholat, Natriumdesoxycholat oder ihrer Mischungen, zwitterionische Tenside wie z. B. CHAPSO oder CHAPS sowie kationische Tenside wie Benzyldimethylhexadecylammoniumchlorid oder Cetylpyridiniumchlorid.

Besonders bevorzugt sind als Bestandteil der Lipidkomponente Emulgatoren, welche gleichzeitig auch Wirkstoffe sind, insbesondere das Lungensurfactant Tyloxapol.

Wirkstoffe, die für das erfindungsgemäße Einarbeiten in SLN und/oder zur erfindungsgemäßen Herstellung Wirkstoff-enthaltender SLN geeignet sind, sind vorzugsweise in Wasser unlösliche Wirkstoffe und Wirkstoffe, die in der festen Lipidmatrix dispergiert oder gelöst werden können (slow release Formulierung). Von den in Wasser unlöslichen Wirkstoffen besonders bevorzugt sind Taxane, Amphotericin B, Campthotecin und Dekapeptide wie z. B. Cetrorelix^{®}.

Das Verfahren zur Herstellung von Emulsionen (SME) gemäß Ausführungsform (5) kann prinzipiell gemäß den beiden folgenden Vorgehensweisen erfolgen (Bsp. 14): Bei einer Vorgehensweise werden alle notwendigen Komponenten, i.d.R. eine Lipidkomponente (z. B. ein Triglycerid-Öl), ein Emulgator und eine wässerige Komponente, in ein geeignetes Gefäß gegeben und die Mischung gespeedmixt. Vorzugsweise wird eine Dispergierhilfe verwendet, insbesondere Glasperlen. Es bildet sich nach wenigen Minuten die gewünschte Emulsion (Bsp. 14A). Bei der zweiten Vorgehensweise werden Lipidkomponente und Emulgator(en) sowie eine geringe Menge der wässerigen Komponente zusammengegeben und - vorzugsweise mit einer Dispergierhilfe - gespeedmixt. Dabei bilden sich Emulsionen mit kleinen Tröpfchendurchmessern, auch bei Verwendung einer geringen Emulgatormenge. Durch Zugabe von zusätzlicher wässeriger Komponente und nochmaliges Speedmixen wird die Emulsion auf die gewünschte Konzentration eingestellt (Bsp. 14B). Beide Verfahren können bei höheren Temperaturen als Raumtemperatur durchgeführt werden.

Emulsionen, die nach dem Verfahren gemäß Ausführungsform (5) hergestellt werden, enthalten vorzugsweise 10-20 % Öl und sind mit 0,5 - 2,5% Lecithin, vorzugsweise Ei- oder Sojalecithin, stabilisiert. Diese SME können statt mit Lecithin auch mit Polymeremulgatoren stabilisiert werden. Solche Emulgatoren sind oft vorteilhaft hinsichtlich ihrer höheren chemischen Stabilität und hinsichtlich ihrer besseren Toleranz gegenüber Elektrolyten.

Beispiele für Polymeremulgatoren sind: Proteine wie z. B. Albumin, Casein oder Gelatine, Proteinderivate wie Kollagenhydrolysat-Tenside, Celluloseether wie Methylcellulose oder Methylhydroxyproplylcellulose (MHPC), Polysiloxan-polyalkylpolyether-Copolymere, natürliche Polysaccharide wie z. B. arabisches Gummi, Polyoxyethylen-polyoxypropylen-Blockcopolymere wie die Poloxamere oder die Polyacrylat-polyalkylacrylat-Crosspolymere wie die Pemulene.

Bevorzugte Öle als Lipidkomponente für erfindungsgemäße Emulsionen sind ausgewählt aus der Gruppe bestehend aus Sojaöl, Olivenöl, Safloröl (Distelöl) und anderen Pflanzenölen, langkettigen Triglyceriden (LCT), mittelkettigen Triglyceriden (MCT), wie z. B. Miglyole, Fischölen und Ölen mit einem erhöhten Anteil an ungesättigten Fettsäuren, acetylierten Partialglyceriden, wie in Stesolid^{®}. Diese Öle können einzeln oder als Mischungen eingesetzt werden.

Wirkstoffe, die in gemäß (5) hergestellten Emulsionen enthalten sind, sind vorzugsweise in Wasser unlösliche Wirkstoffe. Besonders bevorzugt sind Taxane, Amphotericin B, Campthotecin und Dekapeptide wie z. B. Cetrorelix^{®}.

Das Verfahren nach (1) eignet sich auch zu Herstellung von Lipoplexen. Lipoplexe werden heute oft für den nicht-viralen Transfer von genetischem Material (z. B. Nukleinsäuren, DNA, cDNA, RNA, antisense RNA, antisense DNA, dsRNA, siRNA) in Zellen eingesetzt. Hierzu müssen die Lipoplexe aus Lipidkomponenten, die ausschließlich oder nur zum Teil aus kationischen Lipiden bestehen, durch Inkubation mit dem genetischen Material hergestellt werden. Ein Problem der Lipoplexbildung ist ihre uneinheitliche Bildung, wodurch der Prozess des Nukleinsäuretransfers in die Zellen und auch ihr *in vivo*-Verhalten beeinflußt werden kann. Dies begründet sich z.T. durch die initial stark unterschiedlichen Konzentrationen von kationischer und anionischer Komponente beim Zusammengeben von Nukleinsäuren und Lipidkomponente und durch den sehr schnellen Vorgang der elektrostatischen Assoziation. Dadurch entstehen initial Lipoplexe, die statistisch zu viel Nukleinsäurekomponente und solche, die statistisch zuviel Lipidkomponente enthalten. Da nach einer ersten Bindung diese Komponenten offensichtlich nicht mehr zu trennen sind, entwickeln sich aus diesen Primärkomplexen die endgültigen Lipoplexe mit dann höchst unterschiedlichen Eigenschaften. Dies kann auch an den unterschiedlichen Größen der Lipoplexe nach ihrer Herstellung abgelesen werden.

Es fehlte also bislang an einem Verfahren, bei dem rasch und insbesondere im kleinen Maßstab eine homogene Vermischung von Lipid und Nukleinsäure-komponente realisiert werden kann mit dem Ergebnis gleichmäßiger Lipoplexe. Dies gelingt durch den Einsatz des Verfahrens nach (1). Dabei kann auf mehrere erfindungsgemäße Arten vorgegangen werden:

Bei Einsatz des Zweikammersystems (Fig. 4) mit einem Loch 68 zwischen den Aufnahmeräumen 50 und 52, das nach Start des Mischvorgangs entweder ein rasches oder ein langsames und gleichmäßiges Durchtreten des Flüssigkeitsvolumens der oberen in die untere Kammer erlaubt, wird in die untere Kammer eine Nukleinsäure-Lösung gegeben, in die obere Kammer eine Dispersion kationischer Liposomen. Nach Start des Mischvorgang treten die kationischen Liposomen zur Nukleinsäure-Lösung über, wodurch einheitliche Lipoplexe entstehen. Dieses Verfahren kann auch bei umgekehrter Vorlage (Nukleinsäure in Aufnahmeraum 52, Liposomen in Aufnahmeraum 50) durchgeführt werden.

Bei Einsatz eines Dreikammersystems, in dem der zweite Aufnahmeraum 52 in Abb. 4 doppelt ausgeführt ist oder durch eine Trennwand in zwei Kammern unterteilt ist und jede der Kammern/Aufnahmeräume 52 eine Austrittsöffnung aufweist, werden die Dispersion mit den kationischen Lipiden sowie die Nukleinsäurelösung in je eine der Kammer/Aufnahmeräume 52 eingefüllt und das Mehrkammersystem gespeedmixt. Durch dieses Verfahren werden die beiden Komponenten in der unteren Kammer unter gleichmäßigem Mischen zusammengeführt, wodurch einheitliche Lipoplexe entstehen.

Als Nukleinsäure werden zur erfindungsgemäßen Herstellung von Lipoplexen bevorzugt cDNA, siRNA oder dsRNA verwendet.

Durch die reproduzierbare und gleichmäßige Herstellung von Lipoplexen und die Möglichkeit des sterilen Arbeitens ist das hier beschriebene System insbesondere auch für klinische Anwendungen im Bereich der Gentherapie geeignet.

Das erfindungsgemäße Verfahren kann genutzt werden zur Herstellung von lipidbasierten Nanopartikeln, insbesondere von Liposomen, aber auch von Emulsionen und SLN, ohne dass vertieftes Fachwissen zur Technologie nötig wäre. Dies gilt speziell bei Verwendung des Kits gemäß Ausführungsform (9). Dies ist von Vorteil in medizinischen, pharmazeutischen und molekularbiologischen Labors, bei denen zwar oft der Bedarf nach Einschluss bestimmter Substanzen (z. B. Proteine, Antikörper, Peptide, DNA, siRNA, Inhibitoren) in lipidbasierte Nanopartikel - z. B. für die Überwindung der Zellmembran - besteht, aber weder die erforderlichen Kenntnisse über Herstellung und/oder Beladung lipidbasierter Nanopartikel noch das Equipment vorhanden ist. Dieses Problem kann gelöst werden durch Bereitstellung von vorgefertigten Kits gemäß (9), bei denen bevorzugt in kleinen Gefäßen (z. B. Eppis) bereits vorgefertigte lipidbasierte Nanopartikel oder geeignete Lipide zur erfindungsgemäßen Herstellung solcher Nanopartikel enthalten sind. Der Anwender muß nur noch eine Testsubstanz (als Lösung oder Festsubstanz) in das Gefäß geben, den Speedmix-Prozess starten und anschließend inkubieren und/oder durch Zugabe von wässerigem Medium die lipidbasierten Nanopartikel verdünnen. Vergleichbares gilt für die Herstellung von lipidbasierten Nanopartikeln, wie sie z. B. in technologischen Labors erforderlich sind.

Das Verfahren nach (1) eröffnet einen neuen Anwendungsbereich für lipidbasierte Nanopartikel, nämlich die Einarbeitung instabiler, insbesondere hydrolysegefährdeter Wirkstoffe und anderer kurzlebiger Substanzen, insbesondere aus der Gruppe der Diagnostika, unmittelbar vor ihrer Applikation wie z. B. die Herstellung einer Injektion direkt am Krankenbett. Klinische Anwendungen von Nanopartikeln, die instabile Substanzen enthalten, sind wegen ihrer bekannten Lagerinstabilität bislang nicht bekannt. Dies gilt insbesondere für liposomale Formulierungen.

Trotz limitierter Haltbarkeit können lipidbasierte Nanopartikel bei Anwendung des erfindungsgemäßen Verfahrens mit Wirkstoff beladen und für die Behandlung von Patienten benutzt werden. Dazu wird eine frisch hergestellte Wirkstoff-Lösung (aus lyophilisierter Substanz) zu einer geeigneten, sterilen Lipidkomponente gegeben und anschließend in einer DAZ das lipidbasierte Nanopartikel hergestellt. Das entstandene, wirkstoffhaltige Nanopartikel kann dann direkt durch Zugabe eines physiologisch verträglichen Mediums (z. B. 0,9 %ige Kochsalzlösung) verdünnt und dann verabreicht werden, z. B. durch überführung in einen Injektionsbeutel. Der Zeitraum der Herstellung der wirkstoffhaltigen Liposomen ist dabei so kurz, dass kaum Hydrolyse des Wirkstoffs stattfinden kann. Besonders geeignet ist dieses Verfahren für hydrolyse-instabile Zytostatika, bevorzugt Alkylantien (Bsp. 5E). Dieses Verfahren kann jedoch auch mit weiteren empfindlichen Substanzen eingesetzt werden, z. B. mit oxidationsempfindlichen Substanzen oder kurzlebigen Diagnostika.

Das erfindungsgemäße Verfahren kann zum Screening (6) nach geeigneten Nanopartikeln für den Einschluß von Wirkstoffen (z. B. für Wirksamkeitsuntersuchungen an Tieren oder *in vitro*) oder für den Bereich der Formulierungsentwicklung, insbesondere für den Bereich der Präformulierung, eingesetzt werden. Dabei werden in Testreihen systematisch verschiedene Lipidkomponenten und/oder deren Mischungen eingesetzt, um eine optimale Formulierung für einen bestimmten Wirkstoff zu identifizieren. Auch ein optimales Wirkstoff/Lipidkomponenten-Verhältnis kann durch ein derartiges Screening ermittelt werden (Bsp. 6). Des weiteren ist das Screening nach Hilfsstoffen und nach der optimalen Gestaltung des Verfahrens (1) möglich. Für das Screening bevorzugt ist der Einsatz erfindungsgemäßer Kits der Ausführungsform (9).

Das Screening gemäß Ausführungsform (6) umfasst auch den Einsatz des erfindungsgemäßen Verfahrens (1) in einem Robotersystem (z. B. Tecan^{®}-Pipettierroboter Genesis 150). Es enthält einen oder mehrere Schritt(e) zur automatisierten Herstellung der lipidbasierten Nanopartikel mit Hilfe einer DAZ. Besonders geeignet ist hierzu die Injektionsmethode. Eine bevorzugte Anwendung des erfindungsgemäßen Screenings ist das Screening von Lipoplexen auf deren Transfektionseigenschaften, wie es in Regelin, A.E. et al., J. Biomol. Screening 6(4):245-254 (2001) beschrieben ist.

Der Kit gemäß Ausführungsform (9) umfasst Komponenten zur Durchführung der erfindungsgemäßen Verfahren.

In einem Aspekt sind dies vorgefertigte lipidbasierte Nanopartikel, insbesondere Liposomen-Gele, hochkonzentrierte Emulsionen oder hochkonzentrierte SLN. Ihre Zusammensetzung richtet sich nach dem Anwendungsbereich des Kits. So enthält ein Kit zur Durchführung des passive loading VPG, während zum Screening der Substanzaufnahme bei lipophilen Substanzen stattdessen oder zusätzlich auch SLN oder Emulsionen enthalten sein können. Vorgefertigte Nanopartikel in diesem Sinne sind auch NanoSolve, Ultraspheres®, Ceraspheres® (Lipod GmbH, Ludwigshafen, Deutschland) und Supravail™ (Phares Drug Delivery AG, Schweiz).

In einem weiteren Aspekt enthält der Kit (9) mindestens eine zur Durchführung des erfindungsgemäßen Verfahrens geeignete Lipidkomponente zum direkten Einschluss von Substanzen mittels des Verfahrens (1) sowie optional mindestens eine wässerige Komponente oder die nichtwässerigen Bestandteile der wässerigen Komponente. Die Komponenten können auch als Prämixe enthalten sein. Solche Kits sind nutzbar zum Screening insbesondere im Bereich der Präformulierung, in der biomedizinischen Forschung und zur Herstellung von Nanopartikeln für die Wirkstoffapplikation im Rahmen der Zubereitung eines Medikamentes. Insbesondere in der biomedizinischen Forschung kann der Kit zusätzlich fluoreszierende oder radioaktive Verbindungen und Lipidkomponenten enthalten.

In wiederum einem weiteren Aspekt enthält der Kit (9) einen Mischbehälter der Ausführungsform (11) und/oder ist zur Durchführung der Injektionsmethode geeignet. In derartigen Kits kann die Lipidkomponente nicht nur als Reinsubstanz, sondern auch in einem geeigneten Lösungsmittel enthalten sein. Ein solcher Kit ist bevorzugt nutzbar zum Einschluss lipophiler Substanzen insbesondere in Liposomen, des weiteren zum Screening (6) im Bereich der Präformulierung und bei der Suche nach optimalen Kombinationen und/oder Konzentrationsverhältnissen von Wirkstoff und Lipidkomponente.

Der Kit enthaltend einen Mischbehälter der Ausführungsform (11) kann auch für die Lipoplex-Herstellung verwendet werden. Bevorzugt enthält er einen Zwei- oder Dreikammer-Mischbehälter gemäß (11), in welchem die kationischen Liposomen bereits enthalten sind. Jedoch können die kationischen Liposomen auch getrennt von dem Mischbehälter gemäß (11) enthalten sein. Des weiteren können die kationischen Liposomen auch *in situ* aus den (dann im Kit enthaltenen) entsprechenden Lipiden hergestellt und in einem nachgeschalteten Schritt die Nukleinsäurelösung zugegeben werden.

Des weiteren ist der Kit enthaltend einen Mischbehälter gemäß (11) auch dann einsetzbar, wenn nicht lipidbasierte Nanopartikel hergestellt oder untersucht werden, sondern der Einschluss von Testsubstanzen in andere Matrices untersucht werden soll. Beispielhaft sei hier der Einschluss von Nukleinsäuren in kationische Polymere genannt.

Optionaler Bestandteil solcher Kits (9) ist eine Substanz, insbesondere ein Adsorbens oder ein Ionentauscher, welche die Abtrennung des ggf. nicht eingeschlossenen Teils der Testsubstanz erlaubt. Weiters kann auch eine oder mehrere Dispergierhilfen enthalten sein.

Lipidbasierte Nanopartikel gemäß Ausführungsform (7), die empfindliche oder kurzlebige Substanzen enthalten, sind bevorzugt Liposomen und SLN, besonders bevorzugt VPG. Die in ihnen enthaltenen empfindlichen oder kurzlebigen Substanzen sind bevorzugt ausgewählt aus hydrolyseempfindlichen Wirkstoffen und kurzlebigen Diagnostika. Insbesondere sind die hydrolyseempfindlichen Wirkstoffe ausgewählt aus Alkylantien einschließlich der N-Lost-Vebindungen Cyclophosphamid, Mafosfamid, Ifosfamid, Trofosfamid, Chlorambucil, Melphalan, Bendamustin, Thiotepa, Busulfan und Treosulfan; aus Nitrosoharnstoffverbindungen einschließlich Carmustin, Lomustin, Nimustin, Dacarbazin, Temozoliomid und Procarbazin; aus Platinverbindungen einschließlich Cis-Platin, Carboplatin und Oxaliplatin; sowie aus Hydroxyharnstoff. Bevorzugte kurzlebige Diagnostika sind Substanzen enthaltend die radioaktiven Isotope Technetium 99, Iod 131 und 123, Indium 111, Thallium 201 und Gallium 67, markierte Verbindungen für die PET (Positronen-Emissions-Tomographie) und Gadoliniumkomplexe.

Daneben enthalten die lipidbasierten Nanopartikel gemäß Ausführungsform (7) bevorzugt solche Verbindungen, welche selbst hydrolytisch auf Lipide wirken können, insbesondere Gemcitabine oder 1-ß-D-Arabinofuranosylcytosin (Ara-C).

Die lipidbasierten Nanopartikel gemäß Ausführungsform (7) werden bevorzugt durch das Verfahren gemäß Ausführungsform (1) bis (5) hergestellt, und diese bevorzugten Nanopartikel sind auch nur durch dieses Verfahren herstellbar. Grund hierfür sind die Vorteile des Herstellverfahrens unter Verwendung einer DAZ. Denn eine vergleichbar hohe Einschlusseffizienz und vergleichbar kleine Vesikelgrößen wie diejenige bei Anwendung des erfindungsgemäßen Verfahrens werden von allen anderen Verfahren zur Herstellung von lipidbasierten Nanonpartikeln nur noch durch die Hochdruckhomogenisation erreicht. Jedoch kann die Hochdruckhomogenisation im Gegensatz zum Verfahren mit DAZ (vgl. Bsp. 20) nicht zur Herstellung sehr kleiner Mengen verwendet werden. Und auch die Herstellungsgeschwindigkeit der DAZ-erzeugten Nanopartikel wird durch die Hochdruckhomogenisation nur selten erreicht. Des weiteren ist steriles Arbeiten und der gegebenenfalls erforderliche Personenschutz bei Hochdruckhomogenisation sehr aufwändig, während diese beim Verfahren mit der DAZ einfach eingerichtet werden können. Vor allem aber ist das DAZ-Verfahren sehr schonend. Beispielhaft seien hier der Einschluss von Gemcitabine und von Bendamustin genannt: Gemcitabine katalysiert unter Hochdruckbedingungen die Hydrolyse von Phospholipiden, was zu unerwünschten Lysophospholipiden im so erzeugten lipidbasierten Nanopartikel führen würde. Bendamustin wird unter Hochdruckbedingungen selbst hydrolysiert. Die durch das Verfahren (1) bis (5) hergestellten lipidbasierten Nanopartikel (7) enthalten dagegen keine Hydrolyseprodukte, wie sie unter Hochdruck entstehen würden. Sie enthalten vielmehr maximal 10 % herstellungsbedingtes Lysolipid bzw. maximal 10 % herstellungsbedingte Abbauprodukte empfindlicher Verbindungen. Letzteres bedeutet, dass maximal 10 % der ursprünglich im Verfahren eingesetzten empfindlichen oder kurzlebigen Substanz während des Einschlussverfahrens zu einem Abbauprodukt umgewandelt wurden.

Es sei darauf hingewiesen, dass auch das passive loading nicht geeignet wäre, um die lipidbasierten Nanopartikel der Ausführungsform (7) zu erzeugen. Dies deswegen, weil das passive loading bei erhöhter Temperatur erfolgt und somit nicht schonen genug für den Einschluss empfindlicher Verbindungen ist. zudem entstehen beim passive loading auch mehr Lyso-Lipide als beim erfindungsgemäßen Verfahren.

Bevorzugte lipidbasierte Nanopartikel gemäß Ausführungsform (7) sind Liposomen mit hoher Einschlusseffizienz und kleiner Vesikelgröße. Bevorzugte Substanzen in diesen Nanopartikeln sind hydrolysempfindliche und lipidhydrolysierende Wirkstoffe wie oben beschrieben, besonders bevorzugt Bendamustin und Gemcitabine.

Wenn zudem sehr kleine Ansatzgrössen notwendig sind, unter anderem im molekularbiologischen Bereich (Einschluss von DNA, RNA, Proteinen oder Peptiden) oder bei radioaktiven einzuschliessenden Substanzen, so können diese Nanopartikel mit der Methode nach (1) bis (5) hergestellt werden, nicht jedoch durch Hochdruckhomogenisation. Für letztere wären die Ansatzgrößen zu niedrig.

Die Verwendung (8) umfasst die Herstellung von Zubereitungen mit lipidbasierten Nanopartikeln, welche bevorzugt in bestimmte Zellen/Gewebe aufgenommen oder an solche Zellen/Gewebe gebunden werden. Dazu zählt auch die Herstellung von Immunoliposomen, von positiv geladenen Liposomen für das Targeting des aktivierten Endothels, von neutralen Liposomen zur passiven Anreicherung in Tumorgewebe durch den EPR-Effekt oder zur Bereitstellung eines systemischen oder lokalen Depots, von negativ geladenen Liposomen für das Makrophagen- und Leber/Milz-Zell-Targeting, von lipidbasierten Nanopartikeln, deren Lipidkomposition den Lipoproteinen ähnelt und so das Zelltargeting und den gezielten Transport bestimmter Lipide, insbesondere Triglyceride erlaubt, von lipidbasierten Nanopartikeln unter Verwendung spezieller Peptide für lysosomalen Escape, von Nukleinsäure-enthaltenden Nanopartikeln, von Nanopartikeln zur Vakzinierung insbesondere durch Aufnahme in dendritische Zellen, und von Nanopartikeln enthaltend eine markierte Lipidkomponente zur Untersuchung des Traffiking. Die Verwendung umfasst auch die Verwendung als Formulierung insbesondere zum Wirkstoff-Boosting und für die orale Applikation. Letztere wird durch Direktbeladung von Tetraetherliposomen ermöglicht.

Zur Verwendung (8) in der Kosmetik beinhalten die lipidbasierten Nanopartikel bevorzugt SiO₂-Partikel.

Ein besonderer Vorteil der Verwendung (8) liegt in ihrer Bedeutung für die Herstellung von Lungentherapeutika. Bei einer Entzündung der Lunge oder beim Frühgeborenen ergibt sich oft ein Mangel und/oder eine fehlerhafte Zusammensetzung des Lungensurfactants. Dies kann substituiert werden. Auf dem Markt befindliche Produkte sind vollsynthetische und teilsynthetische Präparationen oder Isolate aus z. B. Surfactant aus Kalbslunge oder Schweinelunge. Im wässerigen Milieu formieren sich die Surfactantkompositionen überwiegend zu lamellaren Strukturen (z. B. Bilayer, Liposomen), die sich nach Instillation in die Lunge auf der Lungenoberfläche spreiten und in das Lungensurfactant integriert werden. Es besteht auch die Möglichkeit der Aufnahme der Lipide bzw. Lipidvesikel durch Pneumozyten, die aus dem extern zugesetzten Surfactant teilweise wieder natürliches Surfactant recyclieren.

Damit die Lipide rasch in die Surfactantschicht der Lunge eingebaut werden können, muß das eingebrachte Surfactant möglichst fein dispergiert sein. Dies ist auch eine Voraussetzung für eine möglichst gleichmäßige Verteilung. Die feine Dispersion überlassen die meisten Hersteller nicht dem Zufall und bieten ihre Surfactantpräparate bereits als fertige Dispersionen an, d. h. die Vesikel wurden bereits beim Hersteller generiert. Nachteil dieser Präparationen ist ihre verringerte Haltbarkeit (15-18 Monate), da im wässerigen Milieu Phospholipide leicht hydrolysiert werden (Beispiele: Exosurf (Glaxo Wellcome), Savanta (Abbott), Curosurf (Nycomed)). Im Gegensatz dazu wird Alveofact (BI) als Trokkenampulle angeboten, die Ampulle enthält das lyophilisierte Lipidgemisch. Vor der Anwendung wird dieses Gemisch zunächst rehydratisiert (d. h. Zugabe von wässerigem Lösemittel, schütteln und warten), dann wird die Rohdispersion durch mehrmaliges Aufsaugen in eine Spritze und Zurückdrücken der Dispersion in das Injektionsfläschchen noch weiter (feiner) dispergiert. Der Nachteil dieser aufwändigen Präparation wird durch eine erhöhte Haltbarkeit des Produktes ausgeglichen (hier: 24 Monate). Die Art dieser Dispersionstechnik lässt erwarten, das die Dispersion viele große Partikel enthält und zudem inhomogen ist.

Eine erfindungsgemäße Aufgabe bestand also darin, ein Verfahren bereitzustellen, das einerseits die Bereitstellung von sehr kleinen, homogenen Teilchengrößen in der Lipiddispersion gewährleistet, andererseits aber sicher und rasch durchzuführen ist und zudem eine lange Produktlagerzeit erlaubt. Für diese Aufgabe präsentiert die vorliegende Erfindung mindestens zwei Lösungswege, die sich aus den verwendeten Bestandteilen herleiten lassen:

Bei Nutzung von trockenen Lipidmischungen (wie z. B. Alveofact Trockenampulle) kann diese Mischung vor ihrer Anwendung mit Hilfe der DAZ-Technik rasch in die dafür erforderliche homogene Dispersion kleiner Partikel verarbeitet werden (Bsp. 10).

Bei de *novo*-Herstellung der lipidbasierten Nanopartikel eröffnet sich die Möglichkeit, Lungensurfactant-ähnliche Nanopartikel, optional mit einen Tracer oder Wirkstoff beladen, herzustellen, die aufgrund ihrer Zusammensetzung und Partikelgrösse für den therapeutischen Einsatz in der Lunge geeignet sind (Bsp. 4).

Des weiteren können im erfindungsgemäßen Verfahren Spezialmischungen zur Aufnahme in Lungenendothelien, insbesondere Surfactantmischungen für das Drug-Targeting via Lungenendothel verwendet werden.

Ein bevorzugtes Resultat der Verwendung (8) sind pharmazeutische oder diagnostische Zusammensetzungen, welche die lipidbasierten Nanopartikel gemäß Ausführungsform (7) enthalten. Diese Zusammensetzungen der Ausführungsform (15) sind Zusammensetzungen, in denen die erfindungsgemäßen lipidbasierten Nanopartikel als Formulierung für die in den vorangegangenen Abschnitten beschriebenen pharmazeutisch oder diagnostisch wirksamen Verbindungen fungieren.

Weitere Anwendungsgebiete des Verfahrens nach (1) neben der Herstellung von Nanopartikeln sind die Herstellung von Cremes, Salben, Pasten und anderen halbfesten Formulierungen von Medikamenten und Kosmetika, die Tablettenherstellung, die Herstellung von Nahrungsergänzungsmitteln und die Rekostitution von pulverförmigen Arzneimitteln. So ist hinsichtlich der Herstellung von Cremes etc. das erfindungsgemäße Verfahren mit einer DAZ im Vergleich zum heute häufig eingesetzten Unguator® besser, flexibler (Verwendung von unterschiedlichen Gefäßen bis hin zu "upside-down"-befüllbaren Tuben und Spritzen), erlaubt steriles Arbeiten und verhindert das Eintragen von Luft. Die Tablettenherstellung mit dem DAZ-Verfahren erlaubt Granulierung und ist auch in Forschung und Entwicklung mit wenig Aufwand möglich. Nahrungsergänzungsmittel und Flüssignahrung (Sondenernährung) können z. B. in Klinikapotheken individuell formuliert werden. Bei der Rekonstitution von Arzneimitteln, die vor Applikation gelöst werden müssen, ist das erfindungsgemäße Verfahren von Vorteil, weil es ein blasenfreies, rasches und sicheres Lösen von Pulvern ermöglicht.

Ausführungsform (10) der Erfindung ist eine Mischvorrichtung zum Mischen von chemischen und/oder biologischen Substanzen, die insbesondere zur Durchführung der erfindungsgemäßen Verfahren verwendet werden kann und auch das Redispergieren lipidbasierter Nanopartikel umfasst. Bei der erfindungsgemäßen Mischvorrichtung erfolgt ein Mischen der Substanzen, insbesondere durch eine Bewegung eines Mischbehälters, die einem Schütteln entspricht. Die Mischvorrichtung weist einen Mischbehälter zur Aufnahme der zu mischenden Substanzen auf. Der Mischbehälter ist in einem Abstand zu einer ersten Drehachse vorgesehen und mit einem Ausleger verbunden. Der Ausleger ist mit einer ersten Antriebseinrichtung, wie einem Elektromotor verbunden, so dass der Ausleger um die erste Drehachse gedreht wird. Durch Drehen des Auslegers erfolgt somit ein Bewegen des Mischbehälters auf einer Kreislinie. Ferner ist eine zweite Antriebseinrichtung zum Drehen des Mischbehälters um eine zweite Drehachse vorgesehen. Die zweite Drehachse verläuft in einem Abstand zur ersten Drehachse. Insbesondere verläuft die zweite Drehachse durch den Mischbehälter. Erfindungsgemäße sind Innenwände des Mischbehälters in unterschiedlichen Abständen zu der zweiten Drehachse angeordnet. Dies hat bei einem Drehen des Mischbehälters um die erste Drehachse sowie um die zweite Drehachse zur Folge, dass während des Drehens unterschiedliche sich ändernde Kräfte auf die Substanzen wirken. Diese sind vergleichbar mit einer bei einer Schüttelbewegung auf Substanzen wirkende Kräfte. Hierdurch erfolgt ein besonders gutes homogenes Durchmischen der in dem Mischbehälter vorhandenen Substanzen.

Vorzugsweise handelt es sich bei dem Mischbehälter um einen zylindrischen Behälter. Die Längsachse des Mischbehälters ist hierbei vorzugsweise in einem Winkel zur zweiten Drehachse angeordnet. Der Winkel ist ungleich 0°. Hierdurch kann das Mischverhalten der Substanzen weiter verbessert werden. Vorzugsweise liegt der Winkel in einem Bereich von 70 bis 110°.

Bei einer besonders bevorzugten Ausführungsform ist der Mischbehälter in einem Aufnahmebehälter angeordnet. Hierbei kann es sich bei dem Aufnahmebehälter um den Behälter 24 der in dieser Anmeldung beschriebenen asymmetrischen Zentrifuge handeln. Es ist somit erfindungsgemäß möglich, einen beispielsweise zylindrischen Mischbehälter, beispielsweise liegend oder schräg in einem vorzugsweise ebenfalls zylindrischen Aufnahmebehälter anzuordnen. Hierzu ist vorzugsweise innerhalb des Aufnahmebehälters eine Halteeinrichtung zum lagegenauen Halten des Mischbehälters in dem Aufnahmebehälter vorgesehen.

In einer besonders bevorzugten Ausführungsform handelt es sich bei der erfindungsgemäßen Mischvorrichtung somit um eine Weiterentwicklung einer bekannten dualen asymmetrischen Zentrifuge, wie sie beispielsweise in EP 1 293 245 beschrieben ist.

Bevorzugt ist die Mischvorrichtung der Ausführungsform (10) wie in Fig. 3 aufgebaut: Ein Mischbehälter 40 ist innerhalb des Aufnahmebehälters 24 angeordnet. Bei dem Aufnahmebehälter 24 handelt es sich um den an Hand der Fig. 1 und 2 beschriebenen mit dem Ausleger 16 verbundenen Behälter 24 einer asymmetrischen Zentrifuge.

Der Mischbehälter 40 ist im Wesentlichen zylindrisch ausgebildet und mittig in dem Aufnahmebehälter 24 angeordnet. Hierbei verläuft die zweite Drehachse 28 durch den Mischbehälter 40. Insbesondere verläuft die Drehachse 28 im Wesentlichen durch den Schwerpunkt des Mischbehälters 40 bzw. nahe des Schwerpunktes. Der Mischbehälter 40 ist mit einem Deckel 42 verschlossen. Um die Lage des Mischbehälters 40 in dem Aufnahmebehälter 24 eindeutig zu definieren, ist innerhalb des Aufnahmebehälters 24 eine nicht näher dargestellte Halteeinrichtung 44 vorgesehen. Bei dieser kann es sich beispielsweise um einen zylindrischen Körper aus Schaumstoff oder dgl. handeln, der eine Ausnehmung aufweist, in die der Mischbehälter 40 eingelegt werden kann.

Der Mischbehälter 40 ist im wesentlichen zylindrisch. Bevorzugt ist hierbei, dass der Querschnitt (senkrecht zum Zylindermantel) des Mischbehälters kreisförmig, elliptisch, oval oder dergleichen ist. Wesentlich ist hierbei, dass der Zylindermantel, bevorzugt der gesamte Mischbehälter 40 keine Ecken aufweist, da sich in solchen Ecken ungewollt Probenmaterial ansammeln kann. Vielmehr sind die Innenwände des Mischbehälters erfindungsgemäß bevorzugt rund ausgebildet bzw. mit Rundungen versehen.

Um ein gutes Durchmischen der Substanzen in dem Mischbehälter 40 zu gewährleisten, weist eine Innenwand 46, bei der es sich im dargestellten Ausführungsbeispiel um die Zylinderinnenwand handelt, zu der zweiten Drehachse 28 unterschiedliche Abstände auf. Diese sind als Beispiel durch gestrichelte Linien a und b angedeutet.

Durch Drehen des Aufnahmebehälters 24, wie an Hand der Fig. 1 und 2 beschrieben, wirken sich ändernde Kräfte auf die in dem Mischbehälter 40 angeordneten Substanzen.

Bei einer besonders bevorzugten Ausführungsform (11) eines Mischbehälters, der insbesondere in Verbindung mit der beschriebenen dualen asymmetrischen Zentrifuge verwendet werden kann, ist ein erster und ein zweiter Aufnahmeraum vorgesehen. Die Aufnahmeräume dienen zur Aufnahme einer ersten bzw. einer zweiten Substanz. Erfindungsgemäß sind die beiden Aufnahmeräume durch eine Trennwand voneinander getrennt, wobei in der Trennwand eine Öffnung vorgesehen ist. Bevorzugt ist nur eine Öffnung, nicht jedoch mehrere Öffnungen (wie z.B. in einem Sieb) vorgesehen. Die Öffnung dient dazu, dass beim Auftreten von Zentrifugalkräften eine Substanz durch die Öffnung von einem Aufnahmeraum in den anderen Aufnahmeraum überführt wird. Hierbei ist die Querschnittsfläche der Öffnung vorzugsweise derart gewählt, dass in Abhängigkeit der Viskosität und der auftretenden Zentrifugalkräfte erst bei Zentrifugalkräften von mehr als 1,2 g, vorzugsweise mehr als 1,5 g ein Hindurchtreten der Substanz durch die Öffnung erfolgt. Vorzugsweise ist die Öffnung in einer Längsachse des ersten und/oder des zweiten Aufnahmeraums angeordnet. Hierbei erfolgt sodann vorzugsweise ein Anordnen des erfindungsgemäßen Mischbehälters in einer Mischvorrichtung oder einer dualen asymmetrischen Zentrifuge derart, dass die Zentrifugalkräfte im Wesentlichen in Richtung der Längsachse wirken, d. h. dass sie ein Durchtreten des Substrates durch die Öffnung bewirken.

Vorzugsweise ist der zweite Aufnahmeraum innerhalb des ersten Aufnahmeraums angeordnet und insbesondere kegelförmig ausgebildet. Hierbei ist es besonders bevorzugt, dass die Öffnung an der Kegelspitze angeordnet ist. Bei dem ersten Aufnahmehohlraum kann es sich um einen zylindrischen Körper handeln. Der Mischbehälter ist vorzugsweise zu einer Längsachse rotationssymmetrisch ausgebildet. Die Längsachse verläuft vorzugsweise ein einem Winkel von etwa 90° zur zweiten Drehachse.

Ferner ist es möglich, dass mehrere zweite Aufnahmeräume vorgesehen sind und/oder der zweite Aufnahmeraum durch Trennwände in mehrere einzelne Räume bzw. Kammern unterteilt ist. Hierbei können zwischen den einzelnen Kammern und/oder Aufnahmeräumen und dem ersten Aufnahmeraum jeweils eine oder mehrere Öffnungen vorgesehen sein. Die Größe der Öffnungen ist jeweils vorzugsweise in Abhängigkeit der Viskosität der Flüssigkeit gewählt. Vorzugsweise ist die Ausrichtung der Öffnungen derart gewählt, dass die Zentrifugalkräfte auf die Öffnungen wirken.

Eine bevorzugte Ausführungsform des Mischbehälters gemäß (11) ist in Fig. 4 dargestellt. Der in Fig. 4 dargestellte Mischbehälter 48 kann in dem Aufnahmebehälter 24 angeordnet werden. Das Mischen der Substanzen erfolgt sodann wie vorstehend an Hand der Fg. 1 und 2 beschrieben.

Der Mischbehälter 48 weist einen ersten Aufnahmeraum 50 und einen zweiten Aufnahmeraum 52 für eine erste bzw. eine zweite Substanz auf. Der erste Aufnahmeraum 50 ist durch einen zylindrischen Körper 54 gebildet. Der Körper 54 ist rotationssymmetrisch zu einer Längsachse 56.

Innerhalb des ersten Aufnahmeraums 50 ist zur Ausbildung des zweiten Aufnahmeraums 52 ein kegelförmiger Körper 58 angeordnet. Dieser ist ebenfalls rotationssymmetrisch zu der Längsachse 56 ausgebildet. Der Körper 58 ist in eine Öffnung 60 des zylindrischen Körpers 54 eingesteckt und wird an einem Rand 62 der Öffnung gehalten. Die Öffnung ist mit einem Deckel 64 verschlossen.

An einer Kegelspitze 66 des kegelförmigen Teils 58 ist eine Öffnung 68 vorgesehen. Die Öffnung 68 ist somit in einer Trennwand 70, durch die der erste Aufnahmeraum 50 von dem zweiten Aufnahmeraum 52 getrennt ist, vorgesehen.

Wird der Mischbehälter 48 nunmehr wie vorstehend an Hand der Fig. 1 und 2 beschrieben sowohl um eine erste Drehachse 18 als auch um eine zweite Drehachse 28 gedreht, wirken Zentrifugalkräfte auf die in der zweiten Kammer 52 vorgesehene Substanz. Hierdurch wird die Substanz durch die Öffnung 68 in den ersten Aufnahmeraum gedrückt bzw. übergeführt. Auf Grund der kegelstumpfförmigen Ausgestaltung ist gewährleistet, dass seitlich neben dem zweiten Aufnahmeraum in einem ringförmigen Bereich 72 Substrat aufgenommen werden kann, wenn die Zentrifugalkräfte von der Öffnung 68 in den zweiten Aufnahmeraum 52 hineingerichtet sind. Es ist hierdurch vermieden, dass größere Mengen an Substanzen aus dem ersten Aufnahmeraum 50 in den zweiten Aufnahmeraum 52 gelangen. Die Ausbildung eines insbesondere ringförmigen Raums 72 ist auf Grund der kegelförmigen Ausgestaltung des Teils 58 realisiert. Der zweite Aufnahmeraum 52 könnte jedoch auch eine andere Form aufweisen, wobei es bevorzugt ist, dass der zweite Aufnahmeraum 52 von einem Raum 72 umgeben ist, um ein Zurückdrücken von Substanzen aus dem ersten Aufnahmeraum 50 in den zweiten Aufnahmeraum 52 zu vermeiden.

Der zweite Aufnahmeraum 52 kann in zwei oder mehr Kammern bzw. Räume unterteilt sein. Vorzugsweise ist der zweite Aufnahmeraum 52 in zwei Kammern geteilt. Hierbei ist es bevorzugt, dass jede der Kammern eine Öffnung aufweist, die entsprechend der Öffnung 68 angeordnet ist und den gleichen Querschnitt aufweist. Je nach Anwendungsfall, insbesondere in Abhängigkeit der Viskosität der in den Kammern bzw. Räumen vorgesehenen Flüssigkeit, können jedoch auch andere Querschnitte der Öffnungen gewählt werden.

Der Mischbehälter 48 wird bevorzugt verwendet, wenn partikelhaltige Formulierungen redispergiert werden sollen (vgl. Bsp. 9C). In diesem Fall wird die partikelhaltige Formulierung im ersten Aufnahmeraum 50 vorgelegt und das Redispersionsmedium in den zweiten Aufnahmeraum 52 gegeben. Anschließend wird der Mischbehälter 48 wie oben beschrieben zentrifugiert. Er wird des Weiteren bevorzugt verwendet, wenn zwei Phasen miteinander vermischt werden sollen, um Mischungen oder lipidhaltige Nanopartikel zu erhalten (Bsp. 11). Diese Verwendung erlaubt auch den Einsatz eines Lösungsmittels in der lipidischen Phase (Bsp. 11C). Des Weiteren wird der Mischbehälter 48 bevorzugt bei Anwendung der Injektionsmethode und zur Herstellung von Lipoplexen verwendet. Mit anderen Worten: der Mischbehälter gemäß Ausführungsform (11) ist bevorzugt für Mischvorgänge zu verwenden.

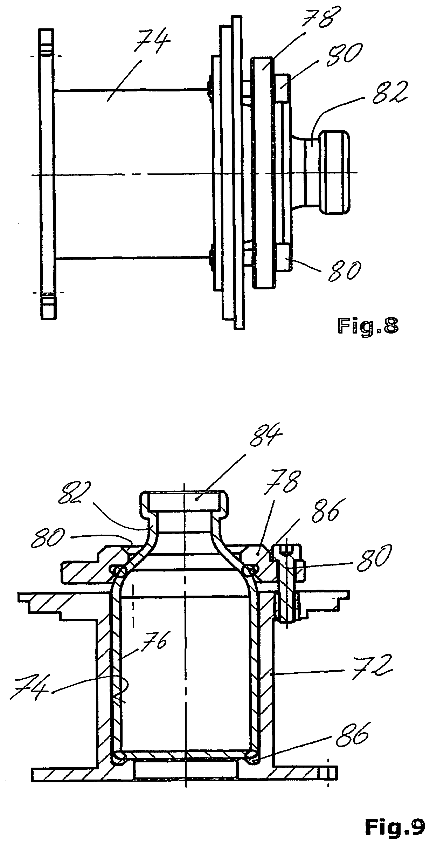

In den Aufnahmebehälter 24 kann in einer weiteren bevorzugten Ausführungsform der Erfindung ein Einsatz eingesetzt werden. Ein erster Einsatz, der insbesondere zur Aufnahme einer Injektionsflasche dient, ist in den Fig. 8 und 9 dargestellt (Ausführungsform (13)). Der Einsatz 72 weist einen Aufnahmeraum 74 auf, der im dargestellten Ausführungsbeispiel zylindrisch ist. Der Einsatz 72 kann in dem Aufnahmebehälter durch Rastelemente oder andere Befestigungsmittel befestigt werden. In den zylindrischen Aufnahmeraum ist ein Mischbehälter 76, bei dem es sich insbesondere um eine Injektionsflasche handeln kann, einsteckbar. Hierbei entsprechen die Innenabmessungen des Aufnahmeraums den Außenabmessungen der Injektionsflasche 76. Vorzugsweise liegt die Außenwand des Mischbehälters 76 vollständig an der Innenwand des Aufnahmeraums 74 an. Der Aufnahmeraum 74 kann jedoch auch mehrere Stege aufweisen, an denen die Außenseite des Mischbehälters 76 anliegt. Insbesondere sind drei Stege vorgesehen, die um 120 Grad zueinander versetzt sind.

Zum Halten des Mischbehälters 76 in dem Aufnahmeraum 74 ist ein Deckel 78 vorgesehen. Der Deckel 78 ist über Schrauben 80 oder andere Befestigungsmittel mit dem Einsatz 72 verbunden. Der Deckel 78 weist eine Öffnung 80 auf, durch die ein Flaschenhals 82 der Injektionsflasche 76 geführt ist. Somit ist ein Verschluss 84 der Injektionsflasche 76 auch dann zugänglich, wenn die Injektionsflasche 76 in dem Einsatz 72 eingesetzt ist.

Um ein Beschädigen einer insbesondere aus Glas hergestellten Injektionsflasche zu vermeiden, sind im dargestellten Ausführungsbeispiel ringförmige Dämpfungselemente 86 vorgesehen. Bei den Dämpfungselementen handelt es sich insbesondere um elastomere Ringe. Hierbei ist ein Dämpfungselement im Bodenbereich des Aufnahmeraums 74 und ein Dämpfungselement 86 im Deckel 78 angeordnet. Wie insbesondere aus Fig. 9 ersichtlich, ist durch die Dämpfungselemente beim Verschließen des Deckels 78 über die Schrauben 80 ein Beschädigen der Injektionsflasche 76 vermieden. Die Dämpfungselemente 86 sind hierzu an den entsprechend kritischen Stellen, insbesondere den Krafteinleitungsstellen am Deckel angeordnet. Die Dämpfungselemente dienen insbesondere zur Fixierung der Flasche im Einsatz. So wird eine Rückdrehung der Flasche infolge der wirkenden Zentrifugalkräfte unterbunden.

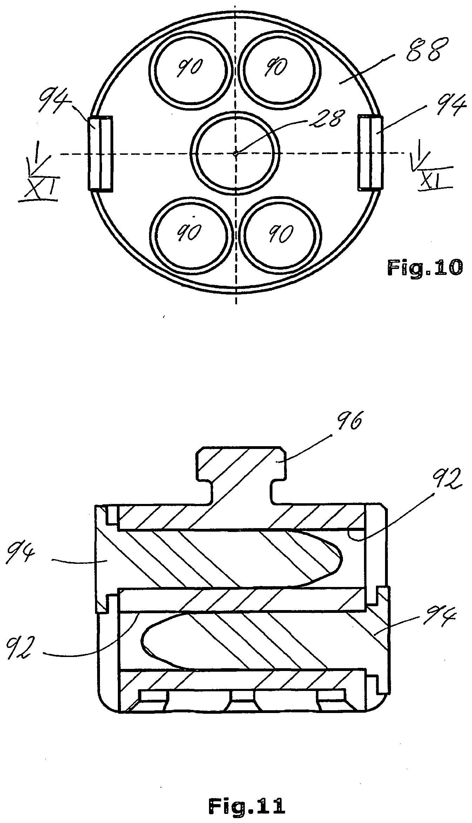

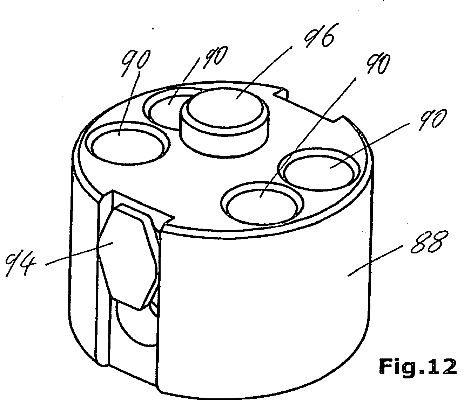

Zur Aufnahme von Mischbehältern 40 (Fig. 3), Mischbehältern 48 (Fig. 4), von Kryo-Vials oder Eppendorfgefäßen und dergleichen, kann ein Aufnahmebehälter-einsatz 88 (Fig. 10 bis 12) vorgesehen sein. Der Aufnahmebehälter 88 weist im dargestellten Ausführungsbeispiel sechs Aufnahmeräume 90, 92 auf. Im dargestellten Ausführungsbeispiel sind die Aufnahmeräume 90, 92 kreiszylindrisch ausgebildet. Die Aufnahmeräume können jedoch auch beispielsweise durch das Vorsehen von Stegen gebildet sein, die zur Lagefixierung der Mischbehälter dienen. Hierbei können sämtliche oder zumindest einzelne Stege elastisch ausgebildet sein.

Die Aufnahmeräume 90 sind im dargestellten Ausführungsbeispiel parallel zur zweiten Drehachse 28 angeordnet. Die Längsachse der Aufnahmeräume 90 verläuft somit parallel zur Drehachse 28 bzw. senkrecht zur Zeichenebene der Fig. 10. Die Längsachsen der Aufnahmeräume 90 sind in einem Abstand zur zweiten Drehachse 28 angeordnet. Die zweiten Aufnahmeräume 92 (Fg. 11) sind senkrecht zur zweiten Drehachse 28 ausgerichtet.

In dem dargestellten Ausführungsbeispiel sind in die Aufnahmeräume 92 Eppendorfgefäße 94 eingesteckt. Hierbei sind die Aufnahmeräume 92 vorzugsweise als Durchgangsöffnungen in dem Einsatz 88 ausgebildet, so dass die Eppendorfgefäße 94 von unterschiedlichen Seiten in die Aufnahmeräume 92 eingesteckt werden können.

Zum erleichterten Transport weist der Einsatz 88 ein Griffelement 96 auf. Das Griffelement 96 ist insbesondere auch zum Einführen und Herausnehmen des Einsatzes in den Aufnahmebehälter 24 geeignet.

Ausführungsform (12) mit der Mischvorrichtung gemäß Ausführungsform (10) ist geeignet, um mit Hilfe einer DAZ auch Schüttelaufgaben zu lösen. Für die Mischvorrichtung gemäß (10) wurde ein DAZ-Einsatz entwickelt, bei dem die Vials horizontal in den Probenhalter eingebracht werden (siehe Fig. 3 und 10 bis 12). Mit einem solchen Einsatz ist praktisch jede Laborschüttelaufgabe sehr effizient, leise und rasch zu leisten.

Das hier entwickelte Verfahren ist erheblich besser als gängige Verfahren (wie z.B. FastPrep^{®} von Qbiogene), weil z. B. zwischen den einzelnen Schüttelvorgängen keine 5-Minuten-Pause eingelegt werden muss und das hierfür benötigte Gerät (DAZ) auch für andere Aufgaben eingesetzt werden kann. Darüber hinaus ist das Schütteln in verschiedenen Gefäßen durch einfachen Austausch des Mischbehälters in der Mischvorrichtung gemäß (10) möglich. Bei Ausführung des DAZ-Einsatzes aus isolierendem Material wie Styropor bleiben tiefgekühlte Proben länger kalt, was insbesondere bei Gewebeaufschluss von Vorteil ist, da so eine schonende Behandlung des Materials trotz des of erforderlichen längeren Schütteins der Probe gewährleistet ist. Insbesondere können mit dieser Technik auch sehr kleine Probenmengen zertrümmert werden. Hierzu wurden tieftemperatur-geeignete Aufnahmebehälter für Probenvials hergestellt (Fg. 10-12), in die verschiedene Typen von sog. Kryo-Vials und Eppendorf^{®}-Gefäßen fest eingespannt werden können. "Tieftemperatur-geeingnet" bedeutet, dass diese Behälter aus einem Material sind, das für die Verwendung bei Temperaturen bis minimal -196°C bzw. in flüssigem Stickstoff geeignet ist. Das Material, aus welchem solche Aufnahmebehälter hergestellt werden, ist vorzugsweise Teflon, PE UHMW oder Aluminium. Für nicht-tieftemperaturgeeignete Aufnahmebehälter können auch andere Materialien verwendet werden, bevorzugt leichte Materialien wie Styropor und Kunststoff.

Eine DAZ, insbesondere eine DAZ mit den Vorrichtungen für das Schüttelverfahren kann also auch zur Zerkleinerung von Feststoffen, insbesondere für die Gewebeaufarbeitung und den Aufschluss von Zellen eingesetzt werden (Bsp. 17). Zerkleinerung bedeutet bevorzugt, dass die Feststoffe nach Durchführung des Verfahrens als Pulver vorliegen. Das entsprechenden Verfahren gemäß Ausführungsform (14) wird unter Zusatz von Zertrümmerungshilfen durchgeführt. Geeignete Zetrümmerungshilfen sind die oben beschriebenen Dispergierhilfen, also Perlen aus Materialien wie Stahl, Achat, Korund und Glas. Ganz besonders bevorzugt sind Stahl- oder Glasperlen. Ihr Durchmesser beträgt bevorzugt mindestens 3 mm, ganz besonders bevorzugt mindestens 5 mm. Der bevorzugte maximale Durchmesser ist 10 mm. Auch Mischungen aus verschiedenen Zertrümmerungshilfen (verschiedene Größe und/oder verschiedene Materialien) können verwendet werden. Wenn Gewebe mit diesem Verfahren zertrümmert werden, so sind sie tierischen (auch menschlichen) oder pflanzlichen Ursprungs. Aufzuschließende Zellen können entweder eukaryotisch oder prokaryotisch sein. Der Aufschluss der Zellen bzw. Gewebe gemäß Ausführungsform (14) kann nicht nur bei Raumtemperatur, sondern auch bei tiefen Temperaturen durchgeführt werden. Hierzu wird die Probe im Probenbehälter gekühlt oder tiefgefroren, z.B. durch Zugabe von flüssigem Stickstoff, und der Aufschluss in der DAZ erfolgt in einem vorgekühlten tieftemperatur-geeigneten Aufnahmebehälter, wie er oben beschrieben wird (Bsp. 17, Fg. 10-12)

Das Verfahren nach Ausführungsform (14) kann auch verwendet werden, um andere Proben als Gewebe oder Zellen zu zerkleinern. Solche anderen Proben sind all jene Materialien, die durch Mörsern zerkleinert werden können. Bevorzugt sind dies Nahrungsmittel, feste Arzneiformen einschließlich Tabletten und Granulaten, und kristalline organische oder anorganische Materialien (vgl. Bsp. 17). Diese Zerkleinerung dient bevorzugt der Probenvorbereitung für die Analytik, wie z.B. der Vorbereitung von KBr-Presslingen für die Infrarotspektrometrie oder der Bestimmung des Wirkstoffgehalts eines Arzneimittels.

Das Verfahren nach Ausführungsform (14) ist gewissermaßen ein Ersatz für den oft mühsam zu handhabenden und für kleine Probenmengen eher ungeeigneten klassischen Mörser. Es ist bevorzugt für die Zertrümmerung kleiner Probenmengen (weniger als 1 g, insbesondere weniger als 300 mg) und für die Handhabung gefährlicher, giftiger oder radioaktiver Substanzen geeignet.

Ein weiterer Vorteil des Verfahrens (14) ist, dass es in Gefäßen mit geringen Volumina durchgeführt werden kann. Bevorzugt sind dies Volumina bis maximal 10 ml, besonders bevorzugt bis maximal 2 ml. Auch die Arbeit in noch kleineren Volumina ist möglich. Dies erlaubt die Arbeit mit kleinen biologischen Proben und das zeitsparende parallele Zerkleinern einer Vielzahl von Proben. Bevorzugte Gefäße sind Eppendorf®-Gefäße und Kryo-Vials.

Die Erfindung wird anhand der nachfolgenden Beispiele näher erläutert, die jedoch das erfindungsgemäße Verfahren nicht einschränken.

### Beispiele

In den folgenden Beispielen bedeutet "Speedmixen" die Nutzung eines Speedmixers^{®} (asymmetrische duale Zentrifuge) vom Typ DAC 150 FVZ der Firma Hauschild GmbH und Co KG, welcher ein Rückdrehverhältnis von ca. 4,2: 1 aufweist. "Eppi" bedeutet ein 2 ml-Eppendorf^{®}-Gefäß. Soweit nicht anders angegeben, beziehen sich %-Angaben auf % (w/v). Soweit nicht anders angegeben, bedeutet "Größe" der lipidbasierten Nanopartikel im folgenden stets "mittlere Größe".

### Beispiel 1: Herstellung von vesikulären Phospholipid Gelen (VPG) und liposomalen Dispersionen mit Hilfe der DAZ-Technik; Vergleich der entstandenen Liposomen mit Liposomen, die mit Hilfe der Hochdruckhomoaenisation hergestellte wurden

1,48 g einer als feste Lösung vorliegenden Mischung aus EPC3 (hydriertes Eilecithin, Fa. Lipoid) und Cholesterol (55 mol%:45 mol%) wurde in eine 25 ml Arzneimittelflasche (Durchmesser außen: 36 mm) eingewogen und zugebördelt (Vial 1). Eine zweite Arzneimittelflasche (Vial 2) enthielt die gleiche Menge Lipid, allerdings wurden hier zusätzlich 3,0 g Glasperlen (Durchmesser ca. 1 mm) zugegeben. In beide Vials wurden 2,22 ml Mannitollösung (5%) injiziert und die Vials jeweils 4* 5min bei RT (Raumtemperatur) mit 3540 rpm gespeedmixt. Durch Zugabe von 6,4 ml Mannitollösung (5%) in 2 Schritten (Schritt 1: 100µl) wurde das entstandene vesikuläre Phospholipidgel (VPG) redispergiert (je 1 min speedmixen) und die mittlere Größe der Liposomen durch Photonenkorrelationsspektroskopie (PCS, Nicomp 370) bestimmt. Die Anzahl der Teilchen > 1 µm wurde mit Hilfe des Abschattungsverfahrens bestimmt (Fa. Nicomp, Accusizer). In die Bestimmung wurde auch VPG einbezogen, welches durch Hochdruckhomogenisation (gleiche Lipidmischung, gleicher Anteil Mannitollösung; 700 bar, 10 Zyklen) hergestellt wurde (Vial 3). Ebenfalls mit einbezogen in die Untersuchung von Teilchen > 1 µm wurde eine kommerzielle Fettemulsion für die parenterale Ernährung (Nutriflex^{®}, Fa. B. Braun).

**Tabelle 1: Vergleich der Liposomengröße und der Zahl der Teilchen, die > 1 µm sind, nach Speedmixen ohne (vial 1) und mit (vial 2) Glasperlen, nach Hochdruckhomogenisation (vial 3) und in einer kommerziellen Fettemulsion (Nutriflex®).**

| Vial | Größe der Liposomen | Anzahl Teilchen > 1 µm pro ml (Verdünnung: 1 :20 Mio.) |
|---|---|---|
| 1 | 171 nm | 173 |
| 2 | 36 nm | 97 |
| 3 | 36 nm | 209 |
| Nutriftex^{®} | 190 nm | 58 |

### Beispiel 2: Herstellung von vesikulären Phospholipid Gelen (VPG) und liposomalen Dispersionen mit Hilfe der DAZ-Technik: Variation der DAZ-Parameter (i) MixGeschwindigkeit, (ii) Gefäßdurchmesser. (iii) Speedmix-Zeit, (iv) Temperatureinfluß und (v) Zusatz einer Mischhilfe

Bei den im folgenden beschriebenen Experimenten wurden Liposomen mit Hilfe der DAZ-Technik analog zu Beispiel 1 hergestellt (gleiche Lipidmischung (als feste Lösung vorliegenden Mischung aus EPC3 (hydriertes Eilecithin, Fa. Lipoid) und Cholesterol (55:45 mol%)), gleiches wässeriges Medium (5%ige Mannitollösung)). Variiert wurde die Geschwindigkeit des Speedmixers®, die Zeit des Speedmixens, der Durchmesser des für das Speedmixen eingesetzten Gefäßes und die Temperatur. Zudem wurde mit und ohne den Zusatz einer Mischhilfe gearbeitet, hierfür wurden bei diesen Beispielen Glasperlen eingesetzt.

Das beim Speedmixen entstandene Liposomen-Gel wurde mit den 3-fachen des primär eingesetzten wässerigen Volumens redispergiert und die jeweils entstandenen Liposomen wurden anschließend mit Hilfe der PCS auf ihre mittlere Größe untersucht.

**Tabelle 2: Effekte der Variation der Speedmix-Parameter**

| Experiment Nr. | MixGesch windigkeit [rpm] | Temperatur | Gefäßtyp/Durchmesser [mm] | Zeit [min] | Glasperlen [mg] | Ansatzgröße [mg Lipid] | Partikelgröße [nm] (Bulkmenge) |
|---|---|---|---|---|---|---|---|
| Einfluss der Zeit | | | | | | | |
| 3a | 3540 | RT | Eppi/10 | 20 | 100 | 50 | 72,7 |
| 3b | 3540 | RT | Eppi/10 | 30 | 100 | 50 | 97,0 |
| 3c | 3540 | RT | Eppi/10 | 50 | 100 | 50 | 69,4 |
| 3d | 3540 | RT | Eppi/10 | 70 | 100 | 50 | 59,4 |
| 3e | 3540 | RT | Eppi/10 | 90 | 100 | 50 | 53,5 |

| Einfluss der Temperatur | | | | | | | |
|---|---|---|---|---|---|---|---|
| 11 | 3540 | 60°C Heizblock Siliconöl | Eppi/10 | 20 | 100 | 50 | 55,3 |
| 17b | 3540 | RT | Glas/10 | 5 | 100 | 50 | 79,7 |
| 18a | 3540 | 60 Heizblock Siliconöl | Glas/10 | 5 | 100 | 50 | 40,3 |

| Einfluss Gefäß und Mischhilfe (Glasperlen) | | | | | | | |
|---|---|---|---|---|---|---|---|
| X17b | 3540 | RT | Glas/10 | 5 | 100 | 50 | 79,7 |
| 17c | 3540 | RT | Glas/ 11 | 5 | 100 | 50 | 87 |
| 17d | 3540 | RT | Glas/ 15 | 5 | 100 | 50 | 53,6 |
| 17f | 3540 | RT | Glas/10 | 5 | - | 50 | 87,4 |
| 17g | 3540 | RT | Glas/11 | 5 | - | 50 | 271 |
| 17h | 3540 | RT | Glas/ 15 | 5 | - | 50 | 119,5 |

| Einfluss der Mixgeschwindigkeit | | | | | | | |
|---|---|---|---|---|---|---|---|
| X17b | 3540 | RT | Glas/10 | 5 | 100 | 50 | 79,7 |
| 30a | 3000 | RT | Glas/10 | 5 | 100 | 50 | 285,1 |
| 30b | 2000 | RT | Glas/10 | 5 | 100 | 50 | Keine Liposomenformation |
| X: Experiment ist aus Vergleichsgründen nochmals aufgeführt | | | | | | | |

### Beispiel 3: Herstellung von vesikulären Phospholipid Gelen (VPG) und/oder liposomalen Dispersionen aus verschiedenen Lipiden/Lipidmischungen mit Hilfe der DAZ-Technik

Bei den im folgenden beschriebenen Experimenten wurden Liposomen mit Hilfe der DAZ-Technik analog zu Beispiel 1 und 2 hergestellt. Variiert wurden bei diesem Experiment die Lipidmischungen. Tabelle 3 stellt die Experimente dar, bei denen Lipidmischungen eingesetzt wurden, die bereits als feste Lösungen vorlagen bzw. bei denen nur Lipide eines Typs eingesetzt wurden. Hierzu wurden die Lipidmischungen zuvor in CHCl₃/MeOH 2:1 aufgenommen, das Lösungsmittel abrotiert und der entstandene Lipidfilm im Vakuum getrocknet und aus dem Kolben gekratzt bzw. die Lipidmischung wurde schon molekulardispers geliefert.

Bei den Experimenten, die in Tabelle 4 dargestellt sind, wurden die Lipide als Einzelkomponenten eingesetzt. Zum Vergleich wurden Werte aus Tab.3 mit Lipidmischungen, die als feste Lösung verwendet wurden, nochmals aufgeführt (durch "X" bzw. "*" markiert).

Die beim Speedmixen entstandenen Liposomen-Gele wurde mit dem 3fachen des primär eingesetzten wässrigen Volumens redispergiert. Die jeweils entstandenen Liposomen wurden anschließend mit Hilfe der PCS auf ihre mittlere Größe untersucht.

**Tabelle 3: Experimente zur Herstellung von Liposomen aus Lipidmischungen, die als feste Lösungen eingesetzt wurden**

| Experiment Nr. | Lipid-Komposition | Ansatzgröße [mg Lipid] | Gefäßtyp/Durchmesser [mm] | Zeit [min] | Glasperlen [mg] | Partikelgröße [nm] (Bulkmenge) |
|---|---|---|---|---|---|---|
| Hydriertes Ei-Phosphatidylcholin/Cholesterol (55/45 mol/mol): siehe Bsp. 2 | | | | | | |
| Phosphatidylcholin aus Soja (min. 80%, S80 Fa. Lipoid), 5% Mannitollösung | | | | | | |
| 4a | E80 | 50 | Eppi | 20 | 100 | 52,7 |
| 4b | E80 | 50 | Eppi | 30 | 100 | 53,5 |
| 4c | E80 | 50 | Eppi | 40 | 100 | 52,4 |
| 8 | E80 | 50 | Eppi | 20 | 100 | 47,3 |

| Kationische Lipide | | | | | | |
|---|---|---|---|---|---|---|
| 5a | DOTAP | 50 | Eppi | 20 | 100 | 57,7 |
| 31 | KL-1-14/Chol 1:0,55 | 50 | Eppi | 10 | 100 | 112,2 |

| Surfactant-Lipide (Aleofact, BI) | | | | | | |
|---|---|---|---|---|---|---|
| 21 | Surfactant-Lipide | Ca. 50 | Original- | 5 | - | 48,1 |
| | | | Vial, Glas Durchmesser: 14mm | | | |

| Negativ geladene Lipidmischung | | | | | | |
|---|---|---|---|---|---|---|
| 32a | HEPC, Chol, DPPG | 50 | Eppi | 5 | 100 | 238,1 |

| Stealth-Liposomen | | | | | | |
|---|---|---|---|---|---|---|
| 29b | HePC/Chol/ DSPE-PEG-2000 | 104 | Glas 11 mm | 25 | 1200 | 88,4 |

**Tabelle 4: Experimente zur Herstellung von Liposomen aus Lipidmischungen, die als Einzelkomponenten in die Experimente eingesetzt wurden**

| Experiment No | Lipid-Komposition | Ansatzgröße [mg Lipid] | Gefäßtyp/Durchmesser [mm] | Zeit [min] | Glasperlen [mg] | Partikelgröße [nm] (Bulkmenge) | Zusatzbehandlung |
|---|---|---|---|---|---|---|---|
| Hydriertes Ei-Phosphatidylcholin/Cholesterol (55/45 mol%/mol%) | | | | | | | |
| 15a | EPC3/Chol | 800 | PE-Becher/ 14 | 20 | 1600 | 60,4 | |
| 15a | EPC3/ Chol | 800 | PE-Becher/ 14 | 25 | 1600 | 54,8 | |
| 15a | EPC3/Chol | 800 | PE-Becher/ 14 | 30 | 1600 | 41,9 | |
| X2 Vergleich | EPC3/Chol-Fertigmischung von Lipoid | 1480 | Injektionsflasche/30 | 20 | 3000 | 36 | |
| 11 | EPC3/Chol | 50 | Eppi | 20 | 100 | Keine Liposomenform ation | RT |
| 11a | EPC3/Chol | 50 | Eppi | 20 | 100 | 56,1 | Erhitzt auf 60°C |

| E80/Chol-Gemische (im Eppi, im großen PE-Becher mit und ohne Erwärmung) | | | | | | | |
|---|---|---|---|---|---|---|---|
| 9 | E80/Chol | 50 | Eppi | 30 | 100 | 200,9 | |
| 13a | E80/Chol | 1200 | PE-Becher/ 14 | 1,5 | 2400 | 133,7 | |
| 13b | E80/Chol | 1200 | PE-Becher/14 | 10 | 2400 | 66,3 | |
| 13c | E80/Chol | 1200 | PE-Becher/ 14 | 15 | 2400 | 55,3 | |
| 13d | E80/Chol | 1200 | PE-Becher/ 14 | 20 | 2400 | 71,2 | |
| 13e | E80/Chol | 1200 | PE-Becher/14 | 25 | 2400 | 70,4 | |
| 14a | E80/Chol | 1200 | PE-Becher/ 14 | 1 | 2400 | 158,3 | Erhitzt auf 60°C |
| 14b | E80/Chol | 1200 | PE-Becher/14 | 5 | 2400 | 85,7 | Erhitzt auf 60°C |
| 14c | E80/Chol | 1200 | PE-Becher/ 14 | 10 | 2400 | 65,4 | Erhitzt auf 60°C |
| 14d | E80/Chol | 1200 | PE-Becher/14 | 15 | 2400 | 60,2 | Erhitzt auf 60°C |
| 14e | E80/Chol | 1200 | PE- | 20 | 2400 | 54 | Erhitzt |
| | | | Becher/ 14 | | | | auf 60°C |

| Kationisches Lipid KL-1-14/Chol (Variation: Mix-Zeit und Temperatur) | | | | | | | |
|---|---|---|---|---|---|---|---|
| 26a | KL-1-14/Chol | 50 | Eppi | 20 | 100 | 371,2 | |

| Experiment No | Lipid-Komposition | Ansatzgröße [mg Lipid] | Gefäßtyp/Durchmesser [mm] | Zeit [min] | Glasperlen [mg] | Partikelgröße [nm] (Bulkmenge) | Zusatzbehandlung |
|---|---|---|---|---|---|---|---|
| 26b | KL-1-14/Chol | 50 | Eppi | 30 | 100 | 298,9 | |
| 26c | KL-1-14/Chol | 50 | Eppi | 40 | 100 | 299,9 | |
| 26d | KL-1-14/Chol | 50 | Eppi | 50 | 100 | 197,7 | Erhitzt auf 60°C |
| X 31 | KL-1-14/Chol* 1 : 0,55 | 50 | Eppi | 10 | 100 | 112,2 | |

| Stealth-Liposomen | | | | | | | |
|---|---|---|---|---|---|---|---|
| 29a | HePC/Chol/DSPE-PEG-2000 | 114 | Glas/10 | 25 | 1500 | 70,3 | |
| X 29b | HePC/Chol/DSPE-PEG-2000* | 114 | Glas/10 | 25 | 1500 | 88,4 | |
| X: Vergleichsexperimente mit Lipidmischungen, die als feste Lösung eingesetzt wurden; *: feste Lösung von Lipidmischungen | | | | | | | |

### Beispiel 4: Herstellung von Surfactant-ähnlichen Liposomen, die für therapeutische Zwecke in die Lunge eingebracht werden können

A) Surfactant-ähnliche Liposomen ohne spezielle Wirkstoffe. (Komposition nach Wissel et al., Am J Physiol 271: L432-40 (1996); oder Müller, B. et al., Thorax 58:127-134 (2003); oderal-Mehdi, A.B. et al., BBA 1167:56-62 (1993)). Die Liposomen wurden in Analogie zum oben beschriebenen Vorgehen durch Speedmixen hergestellt. Alle Arbeiten wurden in einer Sterilbank durchgeführt. Die Lipidkomposition war DPPC (Sigma P5911)/PG (Sigma-P05141) /EPC (Lipoid) /Cholesterol im Gewichtsverhältnis 5,5/1/2,5/1. Die Lipide wurden eingewogen, in Chloroform/MeOH 2:1 aufgenommen, das Lösemittel abrotiert und der entstandene Lipidfilm getrocknet. 50 mg dieses Lipidgemisches wurde dann in ein autoklaviertes Speedmix-Gefäß (Glasvial, 10 mm) eingewogen, 100 mg autoklavierte Glasperlen zugefügt und ein Volumen von 75 µl sterilfiltriertem PBS (25 mM NaH₂PO₄, 125 mM NaCl, pH 6,9) zugegeben und für 10 Minuten im geschlossen Gefäß gespeedmixt. Anschließend wurde mit 250 µl PBS redispergiert. Es wurden Liposomen der Größe 74,6 nm erhalten.
B) Surfactant-ähnliche Liposomen mit Fluoreszenz- (NBD, Rhodamin) und Radio-Label (³H). Für dieses Experiment wurden folgende Lipide in ein steriles Glasgefäß eingewogen bzw. als Lösung in CHCl₃/MeOH 2:1 zugegeben: DPPC, 81,2 mg (Sigma P5911) / PG, 17,1 mg (Sigma-P05141) / EPC, 42,7 mg (Lipoid) / Cholesterol, 17,1 mg / Rhodamin-PE, 1,7 mg / NBD-PC 7,7 mg / ³H-DPPC (1,46nCi/µg DPPC). Die Mischung wurde im Speedmixgefäß (Glas 15 mm) vollständig mit CHCl₃/MeOH 2:1 gelöst und das Lösungsmittel vorsichtig in einem Stickstoffstrom bis zur Trockene abgedampft. 250 µl steriler PBS-Puffer und 350 mg sterile Glasperlen (Durchmesser 1 mm) wurden zugegeben und die Mischung im geschlossenen Gefäß für 15 Minuten gespeedmixt. Anschließend wurde 17,2 ml sterile PBS-Lösung zugegeben und nochmals für 1 Minute gespeedmixt. Die Vesikel der fertigen liposomalen Dispersion hatten eine mittlere Größe von 83,5 nm.
C) Surfactant-ähnliche Liposomen mit einem lipidischen sPLA2-Inhibitor als antiinflammatorischem Wirkstoff. DPPC, PG, EPC, Cholesterol und der sPLA2-Inhibitor 2-(*R*)-1-O-Phosphocholin-2-N-laurinoyl-octadecan in den Gewichtsverhältnissen 50/10/25/10/5 wurden gemeinsam eingewogen und in CHCl₃/Methanol 2:1 gelöst. Das Lösungsmittel wurde abrotiert, derentstandene Lipidfilm im Vakuum von Lösemittelresten befreit und die Lipidmischung aus dem Kolben gekratzt. Eine Menge von 100 mg Lipidmischung wurde in ein steriles Glasgefäß eingewogen, 200 mg sterile Glasperlen (Durchmesser 1 mm) zugegeben und 150 µl PBS-Puffer (10 mM NaH₂PO₄, 149 mM NaCl, pH 6,9) zugesetzt. Das Gemisch wurde 15 Minuten gespeedmixt, anschließend wurde das entstandene Gel mit 9,8 ml PBS redispergiert. Die Vesikel der fertigen Dispersion hatten eine mittlere Größe von 74,3 nm.

### Beispiel 5: Direkte Herstellung von mit Substanzen beladenen vesikulären Phospholipid Gelen (VPGLund liposomalen Dispersionen mit Hilfe der DAZ-Technik

A) Direkter Einschluss von Albumin in Liposomen. In sechs sterile Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol (55/45 mol%) eingewogen. In drei der Vials wurde je 75 µl einer Albuminlösung in den Konzentrationen 1, 2 und 3 mg/ml zupipettiert. In In das vierte bis sechste Vial wurden je 75 µl NaCl-Lösung der Konzentration 0,9 % gegeben. Die verschlossenen Vials wurden je 10 Minuten bei 3540 rpm gespeedmixt. Die drei mit Albumin beladenen Liposomen-Gele wurden mit je 250 µl NaCl-Lösung redispergiert. Die nur mit Kochsalzlösung hergestellten Liposomen-Gele (Vial 4-6) wurden hingegen mit 175 µl NaCl-Lösung plus 75 µl Albuminlösung (1, 2, 3 mg/ml) redispergiert. Anschließend wurden die so entstandenen liposomalen Dispersionen in geeignete Zentrifugenröhrchen pipettiert und bei 350.000 g zentrifugiert, wobei die Liposomen sedimentierten. Im Überstand wurde anschließend die Albuminkonzentration mit Hilfe eines BCA-Tests (Interchim/ Uptima, Nr. UP95424) bestimmt und die Einschlusseffizienz berechnet. Ergebnis Einschlusseffizienz für Albumin in Liposomen durch Speedmixen:
   1 mg/ml Albumin: 60,9 %; 2 mg/ml Albumin: 70,9 %; 3 mg/ml Albumin: 71,8 %
B) Direkter Einschluss von Trypan-Blau in Liposomen als Beispiel für niedermolekulare Wirkstoffe. In zwei Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol (55/45 mol%) eingewogen. In ein Vial wurde 75 µl einer Trypan-Blau Lösung (1000 µl handelsübliche Trypan Blau-Lösung (0,4 %) plus 30 ml 25%ige Ammoniaklösung) pipettiert. In das zweite Vial wurde 75 µl 0,9 % NaCl-Lösung gegeben. Die verschlossenen Vials wurden je 10 Minuten bei 3540 rpm gespeedmixt. Das mit Trypan Blau Lösung beladene Liposomen-Gel wurde mit 250 µl NaCl-Lösung redispergiert. Das nur mit Kochsalzlösung hergestellte Liposomen-Gel wurde mit 175 µl NaCl-Lösung plus 75 µl Trypan-Blau-Lösung redispergiert. Anschließend wurden beide liposomale Dispersionen in geeignete Zentrifugenröhrchen pipettert und bei 350.000 g zentrifugiert, wobei die Liposomen sedimentierten. Im Überstand wurde anschließend die Trypan-Blau-Konzentration durch Absorptionsspektroskopie bestimmt und die Einschlusseffizienz berechnet. Ergebnis Einschlusseffizienz für Trypan-Blau in Liposomen durch Speedmixen: 51 %
C) Direkter Einschluss von fluoreszenz-gelabelter RNA in neutrale Liposomen sowie in Liposomen mit negativer Oberflächenladung
   i) Neutrale Liposomen: In zwei Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol (55/45 mol%) eingewogen. In ein Vial wurde 75 µl einer 1 µM RNA-Cy3 Lösung (RNA: 27mer; 5'- GGA GCU CGC U T** C GGC GAG GUC GUG CCA-3'; T**=Thymidine-C6-Amino-NHS-Cy3; Cy3: Ruoreszenzfarbstoff; Qiagen) pipettiert. In das zweite Vial wurde 75 µl NaCl-Lösung gegeben. Die verschlossenen Vials wurden je 10 Minuten bei 3540 rpm gespeedmixt. Das mit RNA-Cy3 Lösung beladene Liposomen-Gel wurde mit 250 µl NaCl-Lösung redispergiert. Das nur mit Kochsalzlösung hergestellte Liposomen-Gel wurde mit 175 µl NaCl-Lösung plus 75 µl RNA-Lösung redispergiert. Anschließend wurden beide liposomale Dispersionen in geeignete Zentrifugenröhrchen pipettert und bei 100.000 rpm zentrifugiert, wobei die Liposomen sedimentierten. Im Überstand wurden anschließend durch Fluoreszenzspektroskopie (em: 565, ex: 515nm) die relativen Konzentrationen an fluoreszenzgelabelter RNA bestimmt und die Einschlusseffizienz für RNA-Cy3 durch Dividieren der Werte berechnet (60,1 %). Die mittlere Größe der redispergierten Vesikel wurde mit Hilfe der PCS zu 127,8 nm bestimmt.
   ii)Negativ geladene Liposomen: In zwei Glasvials (Durchmesser 10 mm) wurden je 100 mg Glasperlen (Durchmesser 1 mm) sowie jeweils 50 mg der als feste Lösung vorliegenden Mischung von hydriertem Ei-Phosphatidylcholin und Cholesterol und Dipalmitoyl-Phosphatidylglycerol (DPPG) (46/33/20 mol%) eingewogen. In ein Vial wurde 75 µl einer 1 µM RNA-Cy3 Lösung (RNA: 27mer; 5'-GGA GCU CGC U T** C GGC GAG GUC GUG CCA-3'; T**=Thymidine-C6-Amino-NHS-Cy3; Cy3: Fluoreszenzfarbstoff; Qiagen) pipettiert. In das zweite Vial wurde 75 µl NaCl-Lösung gegeben. Die verschlossenen Vials wurden je 10 Minuten bei 3540 rpm gespeedmixt. Das mit RNA-Cy3 Lösung beladene Liposomen-Gel wurde mit 250 µl NaCl-Lösung redispergiert. Das nur mit Kochsalzlösung hergestellte Liposomen-Gel wurde mit 175 µl NaCl-Lösung plus 75 µl RNA-Lösung redispergiert. Anschließend wurden beide liposomalen Dispersionen in geeignete Zentrifugenröhrchen pipettert und bei 100.000 rpm zentrifugiert, wobei die Liposomen sedimentierten. Im Überstand wurden anschließend durch Fluoreszenzspektroskopie (em: 565, ex: 515nm) die relativen Konzentrationen an fluoreszenzgelabelter RNA bestimmt und die Einschlusseffizienz für RNA-Cy3 durch Dividieren der Werte berechnet (82,9 %). Die mittlere Größe der redispergierten Vesikel wurde mit Hilfe der PCS zu 238,1 nm bestimmt.
D) Direkter Einschluss von Gemcitabine in vesikuläre Phospholipid-Gele durch Speedmixen. In eine 25 ml Injektionsflasche (Durchmesser aussen: 36 mm) wurden unter sterilen Bedingungen 1,48 g der als feste Lösung vorliegenden Mischung von hydriertem Ei-Phosphatidylcholin und Cholesterol (55/45 mol%) sowie 3 g Glasperlen (Durchmesser 1 mm) eingewogen. Die Rasche wurde zugebördelt. Durch das Septum wurden 500 µl einer Gemzar®-Lösung (enthält 38,02 mg Gemcitabine/ml; Gemzar^{®} ist der Name des Gemcitabine enthaltenden Arzneimittels der Fa. Lilly, 1 Durchstechflasche enthält 200 mg Gemcitabine (lyophilisiert) und wird mit 5 ml 0,9 % NaCl rekonstituiert) sowie 2,22 g 5%ige Mannitollösung zugegeben. Die Mischung wurde 20 Minuten bei 3540 rpm gespeedmixt, wodurch ein Gemcitabine enthaltendes VPG entsteht.
   Die Einschlusseffizienz für Gemcitabine betrugt 80,7 % (Verfahren: Moog et al., Cancer Chem Pharmacol 49:356 (2002)), der Lyso-PC-Gehalt weniger als 0,5 % LysoPC/Gesamtlipid (mit Hilfe der HPTLC), die mittlere Größe der Vesikel war 38,7 nm (PCS).
   Damit konnte gezeigt werden, dass Gemcitabine durch speedmixen in VPG eingeschlossen werden kann und dass das Produkt mit Gemcitabine enthaltendem VPG vergleichbar ist, bei dem das leere VPG durch Hochdruckhomogenisation hergestellt wurde und Gemcitabine durch passives Loading (EP 1087752) nachträglich eingearbeitet wurde (Einschlusseffizienz bei passives Loading: 43-47%). Das hier durchgeführte Verfahren ist offensichtlich aber schonender, was der niedrige Lyso-PC-Gehalt der Formulierung zeigt (Gehalt bei HochdruckHomogenisation und anschließendem passivem Loading: 3-5%).
E) Direkter Einschluss von Bendamustin in vesikuläre Phospholipid-Gele durch Speedmixen. In eine 25 ml Injektionsflasche (Durchmesser aussen: 36 mm) wurden unter sterilen Bedingungen 1,0 g der als feste Lösung vorliegenden Mischung von hydriertem Ei-Phosphatidylcholin und Cholesterol (55/45 mol%) sowie 1,0 g Glasperlen (Durchmesser 1 mm) eingewogen. Die Flasche wurde zugebördelt. Durch das Septum wurde 1,5 ml einer Bendamustin^{®}hydrochloridLösung gegeben (enthielt 9,09 mg Bendamustin/ml; die Lösung wurde durch Rekonstitution von Bendamustin der Fa. Ribosepharm hergestellt. Hierzu wurde 0,9 % NaCl-Lösung in die Arzneiflasche mit dem lyophilisiertem Arzneistoff injiziert). Die Mischung wurde 30 Minuten bei 3540 rpm gespeedmixt, wobei ein Bendamustin-hydrochlorid enthaltendes VPG entstand. Es wurden Proben der Bendamustin enthaltenden VPGs entnommen und die Einschlusseffizienz für Bendamustin mit 40,6 +- 3,08 % (SD) (n: 4) bestimmt (Abtrennung des freien Bendamustins: per Ionentauscher, Moog et al., Cancer Chem Pharmacol 49:356 (2002), Bendamustin-Analytik: Maas, B. et al., Pharmazie 49:775-777 (1994)). Die mittlere Größe der Vesikel wurde mit 62,5 +- 3,5 nm (SD) (n: 8) durch PCS bestimmt.
F) Direkte Herstellen eines vesikulären Phospholipid-Gels unter Verwendung einer Siliziumdioxid-Dispersion

10 % Phospholipide in Siliziumdioxid-Dispersion: 2,25 g eines Kieselsäuregels mit 2,8 g Kieselsäureanhydrid/100 ml Wasser wurde mit 250 mg S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurden mit PCS bestimmt und lag bei 31,5 nm.

15 % Phospholipid in Siliziumdioxid-Dispersion: 2,15 g eines Kieselsäuregels mit 2,8 g Kieselsäureanhydrid/100 ml Wasser wurde mit 0,38 g S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurde mit PCS bestimmt und lag bei 31,3 nm.

30 % Phospholipid in Siliziumdioxid-Dispersion: 1,76 g eines Kieselsäuregels mit 2,8 g Kieselsäureanhydrid/100 ml Wasser wurde mit 710 mg S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurde mit PCS bestimmt und lag bei 64,5 nm.

40 % Phospholipid in Siliziumdioxid-Dispersion: 1,49 g eines Kieselsäuregels mit 2,8 g Kieselsäureanhydrid/100 ml Wasser wurde mit 1004 mg S80 versetzt. Die Mischung wurde 20 Minuten im PE-Becher (Durchmesser ca. 34 mm) bei 3540 rpm gespeedmixt. Es entstand eine cremige Formulierung, die in Wasser leicht redispergiert werden kann. Die Teilchengröße wurde mit PCS bestimmt und lag bei 85,2 nm.

### Beispiel 6: Einarbeiten eines wasserunlöslichen Wirkstoffs in vorgefertigte VPG oder feste Lipid Nanopartikel (SLN), Screening nach einem geeigneten Wirkstoff: Lipid-Verhältnis

A) Einarbeiten in Liposomen. Es sollte untersucht werden, ob und in welchen Anteilen sich derwasserunlösliche Wirkstoff PQ013 (PQ: Prokinase GmbH, Freiburg; MG: 399,4 g/mol) in vorgefertigte Liposomen einarbeiten lässt. Hierzu wurden Liposomen-Gele aus E80 (Phosphatidylcholin-Präparation aus Ei, Fa. Lipoid, Ludwigshafen) durch Speedmixen hergestellt (500 mg E80, PE-Becher mit Durchmesser 34 mm, 750 µl 0,9 % NaCl, 10 min Speedmixen bei 3540 rpm). Steigende Mengen PQ013 wurden in Glasvials (Durchmesser 10 mm) eingewogen, bzw. bei sehr kleinen Mengen in Dioxan gelöst einpipettiert. Im letzteren Fall wurde anschließend das Lösungsmittel im Vakuum entfernt. Jeweils 125 mg des E80 Gels (Enthält 50 mg S80, ca. 68 µmol) wurden in die Vials gegeben und es wurde 30 Minuten bei 3540 rpm gespeedm ixt. Anschließend wurde jeweils 1 ml PBS zugegeben und für 2 Minuten redispergiert. 100 µl der jeweiligen liposomalen Formulierungen wurden in eine 24-well Mikrotiterplatte pipettiert, kurz anzentrifugiert und anschließend mikroskopisch auf das Vorhandensein von PQ013-Kristallen untersucht. Es wurde gefunden, das bei einem molaren Verhältnis von PQ013 zu Lipid kleiner als 1:40 keine Kristalle mehr auftraten, d. h. das PQ013 in einem über 40fachen molaren Überschuss an Phospholipid (E80) vollständig löslich ist.

| Ansatz | 1 | 2 | 3 | 4 | 5 | 6 | 7 | 8 |
|---|---|---|---|---|---|---|---|---|
| PQ013 [µmol] | 68 | 6,8 | 3,4 | 1,7 | 1,13 | 0,68 | 0,14 | 0,068 |
| PQ013 [mg] | 27,2 | 2,72 | 1,36 | 0,68 | 0,45 | 0,27 | 0,06 | 0,03 |
| Verhältnis (molar) | 1:1 | 1:10 | 1 :20 | 1 :40 | 1 :60 | 1:100 | 1:500 | 1:1000 |
| Kristalle | J | J | J | N | N | N | N | N |

Ein analoges Experiment wurde mit Liposomen aus reinem hydriertem Phosphatidylcholin aus Ei (EPC, Fa. Lipoid) durchgeführt. Dabei konnte PQ013 ebenfalls ab einem molaren Verhältnis von 30:1 Lipid zu Wirkstoff in den Liposomen gelöst werden.
B) Einarbeiten in SLN. Es sollte untersucht werden, ob und in welchen Anteilen sich der wasserunlösliche Wirkstoff PQ013 (PQ: Prokinase GmbH, Freiburg; MG: 399,4 g/mol) in vorgefertigte SLN einarbeiten lässt. Hierzu wurden SLN gemäß Beispiel 15 durch speedmixen hergestellt, allerdings wurden diese nicht redispergiert/verdünnt sondern im weiteren als viskose Masse eingesetzt. Steigende Mengen PQ013 wurden in Glasvials (Durchmesser 10 mm) eingewogen, bzw. bei sehr kleinen Mengen in Dioxan gelöst einpipettiert. Im letzteren Fall wurde anschließend das Lösungsmittel im Vakuum entfernt. Jeweils 125 mg des SLN-Gels (enthält 33,8% Trimyristin, d. h. 42,3 mg; 58,4 µmol) wurden in die Vials gegeben, die Vials wurden auf 80 °C erwärmt und es wurde 30 Minuten bei 3540 rpm gespeedmixt (6 x 5 Minuten mit zwischenzeitlicher Erwärmung). Anschließend wurde jeweils 1 ml PBS zugegeben und für 2 Minuten redispergiert. Die Proben wurden für 3 Stunden im Kühlschrank bei 4-8 °C belassen, damit die SLN fest wurden. 100 µl der jeweiligen Dispersionen wurden in eine 24-well Mikrotiterplatte pipettiert, kurz anzentrifugiert und anschließend mikroskopisch auf das Vorhandensein von PQ013-Kristallen untersucht. Es wurde gefunden, das bei einem Gewichtsverhältnis von PQ013 zu SLN kleiner als 1:500 keine Kristalle mehr auftraten. PQ013 ist in einem 500fachen molaren Überschuss an Trimyristin (SLN) vollständig löslich.

| Ansatz | 1 | 2 | 3 | 4 | 5 | 6 | 7 | 8 |
|---|---|---|---|---|---|---|---|---|
| PQ013 [µmol] | 58,4 | 5,8 | 2,92 | 1,46 | 0,98 | 0,58 | 0,12 | 0,058 |
| PQ013 [mg] | 23,4 | 2,32 | 1,17 | 0,58 | 0,39 | 0,23 | 0,05 | 0,02 |
| Verhältnis (molar) | 1:1 | 1:10 | 1 :20 | 1:40 | 1:60 | 1:100 | 1:500 | 1:1000 |
| Kristalle | J | J | J | J | J | J | N | N |

### Beispiel 7: Herstellung von Immunoliposomen durch Speedmixen

Durch dieses Beispiel soll die einfache und für das Kopplungslipid schonende Herstellung von Immunoliposomen durch Speedmixen demonstriert werden.
A) Immunoliposomen mit IgG. 37,7 mg hydriertes EPC, 10,8 mg Cholesterol, 6,6 mg DSPE-PEG-2000 und 0,44 mg DSPE-PEG-2000-Maleimid wurden in ein Glasvial (Durchmesser 10 mm) gemeinsam mit 100 mg Glasperlen (Durchmesser 1 mm) eingewogen. Die Lipide wurden mit 150 µl CHCl₃/MeOH 2:1 gelöst, anschließend das Lösemittel durch einen Stickstoffstrom wieder entfernt, so dass ein Lipidfilm zurückblieb. Lösungsmittelreste wurden über Nacht im Vakuum entfernt. 80 µl HEPES-Puffer (20 mM HEPES, 130 mM NaCl, pH: 6,8) wurde zugegeben, das geschlossene Vial bei 37 °C für 5 Minuten inkubiert und anschließend für 10 Minuten bei 3540 rpm gespeedmixt. Unspezifisches IgG wurde gemäß Huwyler, J. et al., Proc. Natl. Acad. Sci. USA 93:14164-14169 (1996) mit 2-Iminothiolan thioliert. Der thiolierte Antikörper wurde anschließend durch Dialyse in Borat-Puffer, pH 8,0, von überschüssigem Iminothiolan befreit und auf ein Volumen von etwa 500 µl konzentriert. Die IgG-Lösung wurde zum Liposomen-Gel gegeben und die Mischung zur Redispergierung 30 Sekunden gespeedmixt und die Mischung zwei Stunden bei RT inkubiert. Anschließend konnten die Liposomen weiter verdünnt und durch Chromatographie an Sephadex® G50 von nicht gebundenem Antikörper getrennt werden.
B) Immunoliposomen mit einem LDL-bindenden Peptid. Es wurde zunächst ein Lipidfilm hergestellt. Hierzu wurden 120 mg EPC3/Cholesterol (55:45 mol:mol), 16,4 mg DSPE-MPEG (2000), 8 mg DSPE-PEG-Maleimid, und 500 µl einer 1 mg/ml-Lösung des fluoreszierenden Farbstoffs Lissamine Rhodamine-PE in Chloroform/MeOH 2:1 (d.h. 0,5 mg Lissamine) in einem 25 ml Kolben gegeben und mit ca. 5 ml CHCl₃/MeOH 2:1 in Lösung gebracht. Das Lösungsmittel wurde mit Hilfe eines Rotationsverdampfers entfernt, so dass ein rötlich-glasiger Lipidfilm zurückblieb. Dieser wurde über Nacht im Vakuum getrocknet, aus dem Kolben gekratzt und unter trockenen Bedingungen aufbewahrt.

20 mg dieser Lipidmischung wurden in ein 10 ml Glasvial zum Zubördeln (Durchmesser aussen 20 mm) gegeben, in dem sich 100 mg Glasperlen (Durchmesser 1 mm) befanden. Durch das Septum wurden 40 µl einer 0,35 M Fosphenytoin-Lösung (in 150 mM Borat-Puffer plus 100 mM EDTA, pH 8) gegeben und das Gemisch wurde zunächst 30 s, nach weiteren 5 min dann für 5 min bei 3540 rpm gespeedmixt. Anschließend wurde 50 µl einer Peptidlösung, enthaltend 30 nmol eines 26 AS-Peptids aus der Bindungsregion des humanen ApoA4 mit einer Mercaptopropionsäure am N-Terminus (zur Bindung an die Maleimidgruppe auf den Liposomen) sowie 100 µl 150 mM Boratpuffer (plus 100 mM EDTA) zu der viskosen Liposomendispersion gegeben und 2 min gespeedmixt. Es wurde über Nacht bei RT inkubiert, die Liposomendispersion anschließend mit 2 ml PBS (1 mM, pH 6 plus 150 mM NaCl) verdünnt und das ungebundene Peptid und das nicht eingeschlossene Fosphenytoin durch Gelfiltration an Sephadex® G-59 Fine abgetrennt.

### Beispiel 8: Gleichmäßiges und rasches Einarbeiten von Wirkstoffen zwischen die Vesikel von vorgefertigten VPG durch Speedmixen

Bei diesen Experimenten sollte gezeigt werden, dass Wirkstoffe/Farbstoffe rasch in bereits vorgefertigte VPG eingearbeitet werden können (VPG-Herstellung z. B. durch Hochdruckhomogenisation). Ziel ist die gleichmäßige Verteilung des Wirk-/Farbstoffs zwischen den Vesikeln, was eine wichtige Voraussetzung für das passive loading darstellt. In den folgenden Beispielen wurde das Einarbeiten von Trypan-Blau als Modellwirkstoff sowie von Gemcitabine als relevanter Wirkstoff zwischen die Vesikel bei vorgefertigten VPGs durch Speedmixen untersucht.
A) Rasches Einarbeiten eines Modellwirkstoffs (Trypan-Blau). In eine 25 ml Injektionsflasche aus Klarglas wurde 1 g VPG (660 mM Lipid, Mischung von hydriertem Ei-Phosphatidylcholin und Cholesterol (55/45 mol%), als feste Lösung vorliegend) eingefüllt, und die Flasche kurz zentrifugiert, so dass sich eine gleichmäßig dicke Schicht des VPG am Gefäßboden ausbildete. Zu dieser Schicht wurden 135 µl einer 0,04%igen Trypan-Blau-Lösung gegeben und das Gefäß für 1 Minute bei 3540 rpm gespeedmixt. Durch genaue Sichtprüfung wurde festgestellt, das der Farbstoff gleichmäßig in das VPG eingearbeitet wurde.
B) Rasches Einarbeiten von Gemcitabine. In eine 25 ml Injektionsflasche wurde 3,7 g VPG (660 mM Lipid, Mischung von hydriertem Ei-Phosphatidylcholin und Cholesterol (55/45 mol%), als feste Lösung vorliegend) eingefüllt, und die Flasche kurz zentrifugiert, so dass sich eine gleichmäßig dicke Schicht des VPG am Gefäßboden ausbildete. Zu dieser Schicht wurden 500 µl µl einer Gemzar^{®}-Lösung gegeben (enthält 38,02 mg Gemcitabine/ml; Gemzar® ist der Name des Gemcitabine enthaltenden Arzneimittels der Fa. Lilly, 1 Durchstechflasche enthält 200 mg Gemcitabine (lyophilisiert) und wird mit 5,0 ml 0,9 % NaCl rekonstituiert). Das Gemisch wurde bei 3540 rpm 0,5 Minuten gespeedmixt.

Anschließend wurde das VPG-Gemcitabine-Gemisch sowie eine Vergleichsprobe, bei der Gemcitabine durch zweimaliges Schütteln über 10 und 5 Minuten und dazwischenliegender 1-stündiger Inkubationpause bei Raumtemperatur in das VPG eingearbeitet wurde, zum passiven Loading 2 Stunden bei 60 °C inkubiert, anschließend das VPG redispergiert und die Einschlusseffizienz für Gemcitabine bestimmt.

Einschlusseffizienz bei Einarbeiten des Gemcitabine durch Speedmixen: 44 %. Einschlusseffizienz bei Einarbeiten des Gemcitabine durch Schütteln: 47 %.

Beispiel 9: Schnelles und sicheres Redispergieren von leeren und wirkstoffhaltigen viskosen, partikelenthaltenden Wirkstoffformulierungen durch Speedmixen/Mischen mittels DAZ

A) Redispergieren eines VPG durch Speedmixen bei mehrstufiger Zugabe des Verdünnungsmediums (Redispergiermediums)
   (i) 3,7 g eines VPG (hergestellt durch Hochdruckhomogenisation (700 bar, 10 Zyklen) einer als feste Lösung vorliegenden Mischung von hydriertem Ei-Phosphatidylcholin und Cholesterol (55/45 mol%)) wurden in eine 25 ml Injektionsflasche eingewogen und kurz zentrifugiert. Anschließend wurde für das Redispergieren zunächst 500 µl NaCl-Lösung zugegeben und für 1 Minute gespeedmixt. Anschließend erfolgte die Zugabe von 6,4 ml NaCl-Lösung und es wurde wieder 1 Minute gespeedmixt. Die entstandene Dispersion war homogen und frei von großen Partikeln, die Vesikelgrößenbestimmung durch PCS ergab 28,1 nm.
   (ii) Zu 4,2 g eines Gemcitabine enthaltenden VPGs (hergestellt durch Hochdruckhomogenisation (700 bar, 10 Zyklen) einer als feste Lösung vorliegenden Mischung von hydriertem Ei-Phosphatidylcholin und Cholesterol (55/45 mol%) und anschließendes passives Loading mit Gemcytabine-Hydrochlorid) wurden in einer 25 ml Injektionsflasche zunächst 500 µl NaCl-Lösung zugegeben und für 1 Minutegespeedmixt. Anschließend erfolgte die Zugabe von 5,9 ml NaCl-Lösung und es wurde wieder 1 Minute gespeedmixt. Die entstandene Dispersion war homogen und frei von großen Partikeln, die Vesikelgrößenbestimmung durch PCS ergab 37,8 nm.
B) Redispergieren eines VPG durch einmalige Zugabe des Verdünnungsmediums.
   1,0 g eines VPG (hergestellt durch Hochdruck-homogenistaion einer als feste Lösung vorliegenden Mischung von hydriertem Ei-Phosphatidylcholin und Cholesterol (55/45 mol%)) wurde in ein 25 ml Injektionsflasche eingewogen, es wurden 2 g Glasperlen (Durchmesser ca. 5 mm) zugegeben und kurz zentrifugiert. Anschließend wurde für das Redispergieren 2,0 ml NaCl-Lösung zugegeben und für 2 Minuten gespeedmixt. Die entstandene Dispersion war homogen und frei von großen Partikeln, die Vesikelgrößenbestimmung durch PCS ergab 35,9 nm.
C) Redispergieren durch Zugabe des Verdünnungsmediums mit Hilfe eines Applikators. Hier wurde das Redispergiermedium durch einen Applikator (Fig. 4) zugegeben: In ein Glasvial (Innendurchmesser ca. 13 mm) wurde ein 0,5 ml Eppendorf-Gefäß gesteckt, das am unteren Ende eine kleine Bohrung aufwies. Die Bohrung war so klein, das keine wässerige Lösung durchtropfen konnte. In das Eppendorf-Gefäß wurde 250 µl der 5%igen Mannitollösung gegeben (Redispergiermedium) und in das Glasvial 125 mg eines VPG (hergestellt durch Hochdruck-homogenistaion einer als feste Lösung vorliegenden Mischung von hydriertem Ei-Phosphatidylcholin und Cholesterol (55/45 mol%)). Das Glasvial wurde verschlossen, wodurch gleichzeitig das Eppendorf-Gefäß fixiert wurde. Das Konstrukt wurde für 30 Sekunden gespeedmixt. Die entstandene Dispersion war homogen und frei von großen Partikeln, die Vesikelgrößenbestimmung durch PCS ergab 36,8 nm.
D) Redispergieren einer Liposomen-enthaltenden Creme. 200 mg "Aloe Vera Liposomen Gel" der Fa. Anton Hübner wurde in einen PE-Becher (Innendurchmesser ca. 14 mm) eingewogen und mit 2 ml Wasser versetzt. Die Mischung wurde für 2 Minuten gespeedmixt, die entstandene Dispersion war homogen und frei von großen Partikeln.
E) Probenvorbereitung bei der Größenbestimmung bei einer Siliziumdioxid-Dispersion Es wurde 1 g einer SiO₂-Dispersion (2,8 g SiO₂/100ml) mit 1 g Glasperlen (Durchmesser 1 mm) sowie 9 ml Wasser versetzt und im PE-Becher (Innendurchmesser ca. 34 mm) für 10 Minuten gespeedmixt. Anschließend konnte eine Größenbestimmung mit PCS durchgeführt werden und ergab eine gut meßbare Partikelgröße von 55 nm.

### Beispiel 10: Reproduzierbares Herstellen einer Surfactantdispersion mit kleinen Vesikeln aus lyophilisierten Surfactant durch Speedmixen

Zwei Fläschchen Alveofact Trockenampulle wurden für das Experiment verwendet (Innendurchmesser ca. 13 mm). Ein Fläschchen wurde gemäß der Herstellervorschrift mit der vorgeschriebenen Menge NaCl-Losung rehydratisiert und extrudiert (siehe Alveofact Trockenampulle Fachinformation). Ein zweites Fläschchen wurde mit der gleichen Menge NaCl-Lösung versetzt, danach aber 5 Minuten im Speedmixer® bei 3540 rpm behandelt. Die mittlere Größen der entstandenen Vesikel wurden durch PCS bestimmt und verglichen. Zudem wurden die Dispersionen 9 Tage bei 4 °C aufbewahrt und dann nochmals vermessen.

### Vial I (Herstellung nach Herstellerangabe) :

- starke Schaumbildung, genaues Abmessen der Suspension nicht möglich.
- Partikel: 67,5 nm (die PCS-Messung ergibt eine sehr breite Verteilung, die bis 5 µm reicht). Vereinzelt noch kleine Flocken zu sehen.
- Nach 9 Tagen bei 4-8°C: Es hat sich ein Niederschlag gebildet. Partikelgrößen wurden mit 132,9 nm bestimmt, was ein deutliches Partikelwachstum bedeutet. Auch die enorme Breite der Verteilung (sehr viele Partikel haben Partikeldurchmesser von über 5 µm) lässt auf das Vorhandensein von sehr großen Partikeln schließen.

### Vial II (Herstellung durch Speedmixen):

- kein Schaum, sauberes Abmessen möglich.
- Partikel: 48,1 nm (deutlich schmalere Partikelverteilung, die im PCS bis 1 µm reicht)
- Dispersion scheint den Kontakt zur Glaswand zu scheuen, Bildung von großen Vakuolen beim Schütteln: Dies bedeutet eine sehr verringerte Oberflächenspannung der Dispersion, entsprechend dem Lungen-Surfactant.
- Nach 9 Tagen bei 4-8°C: Partikelgröße: 48,8 nm. Damit hat sich die primär hergestellte Dispersion nicht geändert. Es wurde im Gegensatz zu Vial I kein Niederschlag beobachtet.

### Beispiel 11: Herstellen von Liposomen aus Lösungen von Lipiden in organischen Lösungsmitteln und einer wässerigen Phase durch Speedmixen

Bei diesem Beispiel wurden Liposomen durch eine Kombination der Injektionsmethode mit der Liposomenherstellungsmethode durch Speedmixen kombiniert. Zu diesem Zweck wurde ein Glasvial (Innendurchmesser ca. 13 mm) mit 100 mg Glasperlen (Durchmesser ca. 1 mm) gefüllt. In den Hals des Vials wurde ein 0,5 ml Eppendorf-Gefäß gesteckt, das am unteren Ende eine kleine Bohrung aufwies. Die Bohrung war so klein, das weder Wasser noch Ethanol durchtropfte.
A) Vorlegen der Lipide in organischen Lösungsmitteln. Zugabe der wässerigen Phase durch Applikator. In das Glasvial wurde eine Lösung von 80 mg S80 in 100 µl Ethanol eingefüllt. Das Eppendorf-Gefäß wurde aufgesteckt und mit 400gl NaCl-Lösung befüllt. Das Konstrukt wurde zugeschraubt, wodurch auch das aufgesteckte Eppendorf-Gefäß stabilisiert wurde. Anschließend wurde bei 3540 rpm für 5 Minuten gespeedmixt, wobei ein leicht trübes, stark viskoses Gel entstand. In das obere Eppendorf-Gefäß wurden weitere 500 µl NaCl-Lösung gegeben und das Konstrukt weitere 5 Minuten gespeedmixt. Es entstand eine liposomale Formulierung, die mittlere Größe der Liposomen wurde mit Hilfe der PCS zu 122,7 nm bestimmt.
B) Vorlegen der Lipide in organische Lösungsmitteln, schrittweise Zugabe der wässerigen Phase. Versuch I wurde in dem Sinne wiederholt, als das zur ethanolischen Lösung von S80 die ersten 400 µl NaCl-Lösung in 100 µl Schritten zugegeben wurden und die Mischung jeweils für 2 Minuten gespeedmixt wurde. Anschließend wurden, wie bei Versuch I, die restlichen 500 µl NaCl-Lösung auf einmal zugegeben und nochmals 5 Minuten gespeedmixt. Die mittlere Größe der entstandenen Liposomen wurde mit 116,1 nm bestimmt.
C) Vorlegen der wässerigen Phase, Zugabe der Lipide in organischen Lösungsmitteln mit Applikator. In das Glasvial wurden 900 µl NaCl-Lösung eingefüllt, in das aufgesteckte Eppendorf-Gefäß eine Lösung aus 80 mg S80 in 100 µl Ethanol. Das Konstrukt wurde für 10 Minuten gespeedmixt. Die enstandenen Liposomen wurden mit Hilfe der PCS mit 83,9 nm bestimmt.

### Beispiel 12: Sicheres und rasches Einarbeiten von Liposomen in Salben, Cremes und Gele und deren Grundlagen

In eine handelsübliche Creme (Tagescreme mit Nachtkerzenöl der Fa. Anton Hübner) wurden Siliziumdioxid-Partikel enthaltende Dispersionen eingearbeitet. Zur Verwendung kamen Dispersionen, die ca. 2 g Siliziumdioxid/100 ml enthielten sowie 10, 15, 30 und 40 % des Phosholipids S 80 (Herstellung siehe Bsp. 5F). Hierzu wurden 1,2 - 2,3 g der Creme in einen PE-Becher (Durchmesser innen ca. 34 mm) eingewogen und mit verschiedenen Anteilen von 10 - 50 Gew% der Siliziumdioxid-Dispersionen versetzt und anschließend 20 Minuten bei 3540 rpm gespeedmixt. In allen Versuchen wurden homogene Cremes erhalten.

| Creme [g] | Siliziumdioxid-S80-Dispersion (Gewicht [g] und Gewichts-%) | S80-Anteil in der Silizumdioxid-S80-Dispersion [%] |
|---|---|---|
| 1,19 | 1,22 (50) | 10 |
| 1,40 | 0,27 (20) | 10 |
| 2,26 | 0,24 (10) | 40 |
| 2,24 | 0,23 (10) | 30 |
| 2,27 | 0,22 (10) | 15 |

### Beispiel 13: Erleichterung der Hochdruckhomogenisation durch DAZ- Vorbehandlung

7,0 g einer als feste Lösung vorliegenden Mischung von hydriertem Ei-Phosphatidylcholin und Cholesterol (55/45 mol%) wurde in eine PE-Becher (Innendurchmesser ca. 51 mm) eingewogen und mit 14 ml 5%iger Mannitollösung versetzt. Die Mischung wurde 20 Minuten bei 3540 rpm gespeedmixt, anschließend wurde die dann homogene Mischung der Hochdruckhomogenisation unterworfen (3 Zyklen). Das resultierende VPG wurde redispergiert und die Vesikelgrößen mit 28,9 nm bestimmt (PCS), was der üblichen Partikelgröße in einem VPG entspricht, das nur durch Hochdruckhomogenisation hergestellt wurde (10 Zyklen).

### Beispiel 14: Herstellung von Emulsionen durch Speedmixen

Es wurden Emulsionen aus Sojaöl (10%) in Wasser mit Phospholipid S75/S80 als Emulgator (2,4% und 1,2%) hergestellt.
A) Emulsion mit 2,4 % Emulgator. 2 ml einer Dispersion von S75 (2,66 g S75/l) werden mit 220 µl Sojaöl in einem PE-Becher (Innendurchmesser ca. 14 mm) zusammengeführt und bei Raumtemperatur 5, 10 und 15 Minuten gespeedmixt (3540 rpm). Die Emulsionen wurden mit der PCS vermessen. Nach einer Mix-Zeit von 10 Minuten wurde auch die Teilchenzahl > 1 µm mit Hilfe des Abschattungsverfahrens bestimmt. Zu Vergleichszwecken wurde auch die Lipiddispersion der Lipidemulsion NuTIlflex^{®} der Fa. B. Braun vermessen.
Bereits nach 5 Minuten hatte sich eine Emulsion gebildet mit einer Teilchengröße um 50 nm, die sich auch durch weiteres Speedmixen nicht änderte. Die Anzahl der Teilchen > 1 µm der so hergestellten Emulsion betrug bei einer Verdünnung von 1:20 Mill. 48 (pro ml). Das NuTRIflex^{®}-Produkt hatte ähnlich viele Teilchen in diesem Größenbereich (58, gleiche Verdünnung).

| Exp. | Mix-Zeit | Teilchengröße | Partikel pro ml (Verdünnung 1:20 Mill.) |
|---|---|---|---|
| 22a | 5 | 53,8 | 53 |
| 22b | 10 | 55,4 | 48 |
| 22c | 15 | 51,8 | 66 |
| NuTRIflex® | - | 197,0 | 58 |

B) Emulsion mit 1,2 % Emulgator. 1 g Sojaöl und 120 mg S80 wurden in einem PE-Becher (Innendurchmesser ca. 14 mm) zusammengegeben und 1,8 g Glasperlen (Durchmesser 1 mm) und 1 ml Wasser zugefügt. Die Mischung wurde auf 60 °C erwärmt und anschließend 5 Minuten gespeedmixt (3540 rpm). Durch Zugabe von 9 ml Wasser wurde die Emulsion auf 10 % eingestellt. Die Partikelgröße wurde mit Hilfe von PCS zu 49,2 nm bestimmt.

### Beispiel 15: Herstellung von festen Lipid Nanopartikeln (SLN) durch Speedmixen

Es wurden SLN aus Trimyristin (10%) in Wasser und Tyloxapol als Emulgator (6 %) hergestellt. 1 g Trimyristin und 560 mg Tyloxapol wurden mit 1.400 µl Wasser in einem PE-Becher (Innendruchmesser ca. 34 mm) zusammengegeben. Es wurden 1,5 g Glasperlen (Durchmesser 1 mm) zugegeben und das Gemisch auf 80°C erwärmt. Anschließend wurde 5 Minuten bei 3540 rpm gespeedmixt. Die Erwärmung des viskosen Gemisches und das Speedmixen wurde noch dreimal wiederholt. Anschließend wurde durch Wasserzugabe die Dispersion auf 10 % Trimyristin eingestellt und für 12 Stunden bei 4-6°C im Kühlschrank belassen. Die Teilchengröße wurde mit Hilfe der PCS mit 69,8 nm bestimmt. Die Anzahl der Teilchen > 1 µm der so hergestellten SLN betrug bei einer Verdünnung von 1:20 Mio lediglich 9 Partikel. Ein für die klinische Routine zugelassene Lipiddispersion (NuTRIflex^{®} von B. Braun, siehe Beispiel 1.12) hatte eine weit höhere Anzahl Teilchen in diesem Größenbereich (58, gleiche Verdünnung).

### Beispiel 16: Redispergieren mittels DAZ-Mischvorrichtung

125 mg Liposomen-Gel, hergestellt durch 20 Minuten Speedmixen einer Mischung von 50 mg hydriertem Ei-Phosphatidylcholin und Cholesterol (55/45 mol%) und 75 µl 5%iger Mannitollösung sowie 100 mg Glasperlen, wurde mit 250 µl Mannitollösung versetzt, in einem Eppi in den Mischbehälter wie in Fig. 3 in den Speedmixer gegeben und für 20 Sekunden gespeedmixt. Die redispergierte Liposomendispersion war homogen und frei von großen Partikeln, die durch PCS bestimmte mittlere Größe der Vesikel betrug 74,2 nm.

### Beispiel 17: Herstellung von Liposomen in Injektionsflaschen

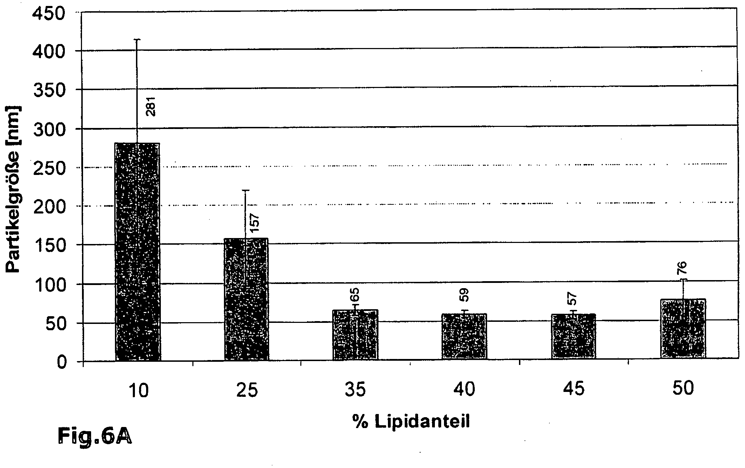

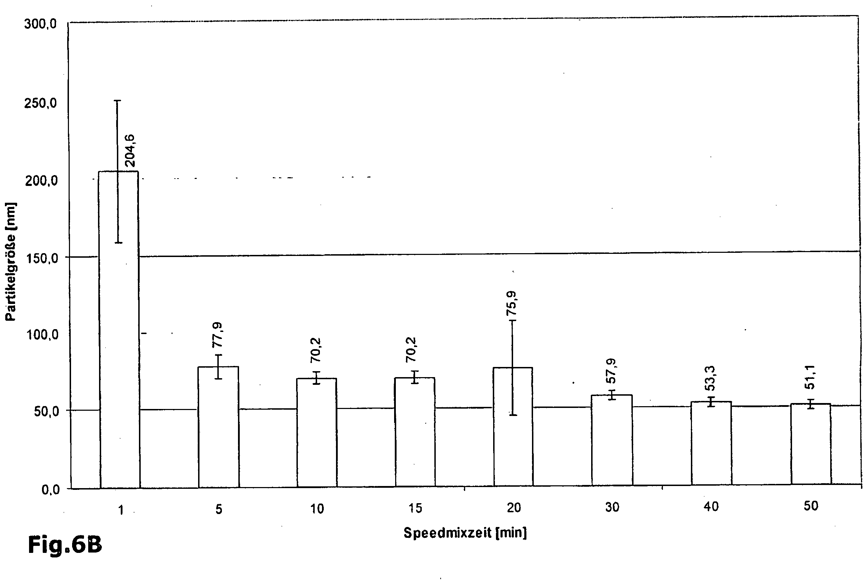

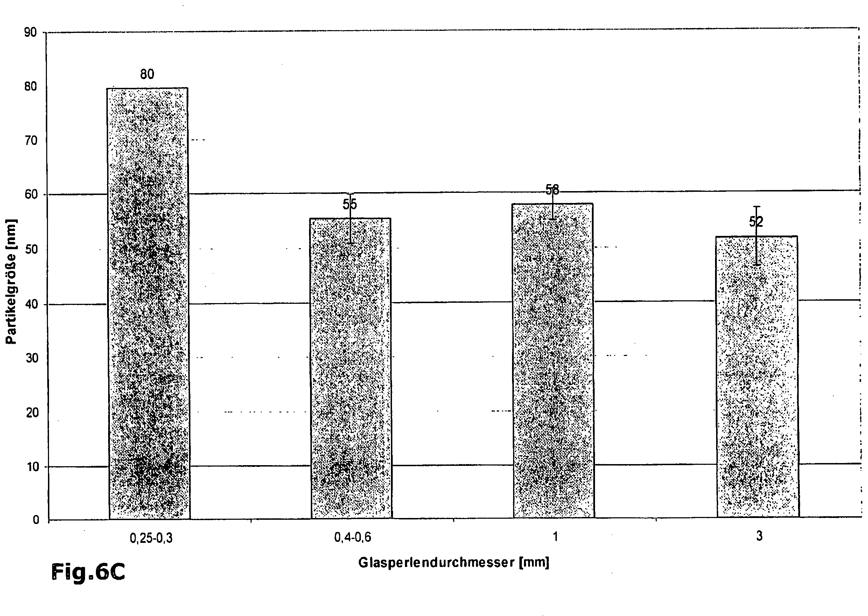

Bei zukünftigen "Bedside-Preparations" von liposomalen Formulierungen werden aus heutiger Sicht Injektionsfläschchen aus Glas zum Einsatz kommen. Aus diesem Grunde wurden auf Basis solcher Gefäße (25 ml Injektionsflasche, Aussendurchmesser 36 mm) Experimente (i) zum Einfluß der Lipidkonzentration während des Speedmixens, (ii) zum Einfluß der Speedmix-Zeit und (iii) zum Einfluß unterschiedlicher Homogenisierungs-Hilfen (Glaskugeln mit unterschiedlichem Durchmesser) auf den Homogenisiererfolg (Vesikelgröße) durchgeführt.
(i) Einfluß der Lipidkonzentration: Jeder Ansatz enthielt 0,5 g einer als feste Lösung vorliegenden Mischung von hydriertem Ei-Phosphatidylcholin und Cholesterol (55/45 mol%) sowie 0,5 g Glasperlen (Durchmesser 1 mm) als Homogenisier-Hilfe. Es wurde soviel 0,9%ige Kochsalzlösung zugegeben, dass Dispersionen mit 10, 25, 35, 40, 45 und 50 Gew.% an Lipid enstanden. Diese Dispersionen wurden jeweils 20 min bei 3540 rpm gespeedmixt, anschließend wurden die Vesikelgrößen mit Hilfe der PCS bestimmt. Jedes Experiment wurde viermal durchgeführt. Die Ergebnisse sind in Fig. 6A dargestellt. Es zeigte sich, dass die kleinsten Vesikel mit Lipidkonzentrationen von 35-45 % herstellbar sind (57-65 nm). In diesem Konzentrationsbereich sind die Ergebnisse auch am reproduzierbarsten, was an den kleinen Standardabweichungen zu erkennen ist.
(ii) Einfluß der Speedmix-Zeit: Es wurde wie unter (i) beschrieben gearbeitet, allerdings wurde in allen Experimenten mit dem gleichen Lipidkonzentration von 40%. Die Speedmix-Zeiten waren 1, 5, 10, 15, 20, 30, 40 und 50 min. Es konnte eine Verkleinerung der Vesikelgrößen mit steigender Speedmixdauer gezeigt werden (Fig. 6B). Nur nach 1 min herrschten sehr große und heterogen größenverteilte Vesikel vor. Bereits nach 5 Minuten waren Vesikel mit einer Größe kleiner als 80 nm nachweisbar. Nach 50 Minuten hatten die Vesikel eine Größe, die knapp über 50 nm lag und sich nicht stark von den Größen bei 30 und 40 Minuten Speedmixdauer unterschieden.
(iii) Einfluß der Dispergierhilfen: Es wurde wie unter (ii) beschrieben gearbeitet, allerdings in allen Experimenten mit der gleichen Lipidkonzentration (40%) und der gleichen Speedmix-Zeit (30 min). Variiert wurde die Art der Glasperlen, die als Aufschüttelhilfe zu den Lipidmischungen gegeben wurden. Es wurde jeweils 0,5 g Glasperlen mit den Durchmessern 0,25-3 mm, 0,4-0,6 mm, 1 mm oder 3 mm zugegeben. Es konnte gezeigt werden, das die Glasperlen mit dem kleinsten Durchmesser zum schlechtesten Homogenisiererfolg führten, die Vesikel hatten eine Größe von etwa 80 nm (Fig. 6C). Glasperlen von 0,4 mm bis 3 mm zeigten keinen signifikant unterschiedlichen Effekt auf das Homogenisierergebnis, die Vesikelgrößen lagen zwischen 52 und 58 mm.

### Beispiel 18: Abhängigkeit der erzielbaren Vesikelgrößen von der DAZ-Geschwindigkeit

Um die Abhängigkeit der Teilchengrößen von der Geschwindigkeit des Speedmixers und damit von der Intensität des Homogenisiervorganges zu zeigen, wurden wie in Beispiel 17 (Liposomen-Herstellung in Injektionsfläschchen) beschrieben Liposomen hergestellt. Dabei wurde immer 20 min gespeedmixt, die Speedmixgeschwindigkeit wurde bei den Experimenten von 2000 rpm bis zu 3450 rpm - der maximalen Geschwindigkeit des zur Verfügung stehenden Speedmixers - Variiert. Das Rückdrehverhältnis lag konstant bei 4,2:1. Die Ergebnisse dieser Experimente sind in Fig. 7 dargestellt.

Die Untersuchung zeigt, dass die erzielbaren Vesikelgrößen von der Speedmixgeschwindigkeit abhängen und dass durch Steigerung der Speedmixgeschwindigkeit kleinere Vesikelgrößen erzielbar sind. Relevante Geschwindigkeiten für die Herstellung insbesondere von kleinen Vesikeln unter 100 nm liegen im Bereich über 2000 rpm. Durch Berechnung einer Ausgleichsfunktion kann zudem gezeigt werden, dass bei weiterer Steigerung der Geschwindigkeit Teilchengrößen im Bereich von 30 nm realisierbar sein sollten (zwischen 4000 und 5000 rpm).

### Beispiel 19: Arbeiten mit geringen Ansatzgrößen

Ein wichtiger Aspekt der Herstellung von lipidbasierten Nanopartikeln wie z.B. Liposomen ist, das man mit der DAZ-Technik unter sterilen Bedingungen auch sehr kleine Mengen, wie sie in Tierversuchen oder im Bereich der Zellkultur benötigt werden, verarbeiten kann. Um dies zu zeigen, wurde ein Experiment durchgeführt, bei dem in einem 10 ml Probenglas mit Rollrand (Typ N20-10 DIN (10 ml), Macherey-Nagel), das unter sterilen Bedingungen gefüllt und zugebördelt werden kann, Liposomen aus einer EPC-3/Cholesterol-Mischung (siehe Beispiel 1) hergestellt wurden. Als Ansatzgrößen wurden 40, 20, 10 und 5 mg Lipid gewählt. In jedes Vial wurde zudem 120 mg Glasperlen (Durchmesser 1 mm) als Homogenisierhilfe gegeben. Es wurde jedem Vial 0,9%ige Kochsalzlösung zugesetzt, das Volumen entsprach dabei 150 % des Gewichtes des eingewogenen Lipids. Die Lipidgewichte und die zugegebene Flüssigkeitsmenge sind in Tab. 6 dargestellt. Die Proben wurden jeweils 30 s gespeedmixt, dann für 25 min bei Raumtemperatur belassen. Anschließend wurden die Proben nochmals 5 min bei 3450 rpm gespeedmixt. Es wurde mit Kochsalzlösung auf einen Lipidgehalt von 10 mM verdünnt (Zugabe von 6,86 ml, 3,43 ml, 1,80 ml bzw. 0,95 ml Kochsalzlösung, anschließend 3 min speedmixen), anschließend wurden die Vesikelgrößen mit Hilfe der PCS bestimmt (Tab. 5). Alle Vesikelgrößen, auch die bei den sehr kleinen Ansätzen, waren untereinander vergleichbar und zudem sehr klein.

**Tabelle 5: Vesikelgrößen bei kleinen Ansatzgrößen**

| Experiment | Lipidmenge | Volumen 0,9%ige Kochsalzlösung | Vesikelgröße |
|---|---|---|---|
| 1 | 39,9 mg | 59,9 µl | 51,2 nm |
| 2 | 10,02 mg | 30,0 µl | 49,8 nm |
| 3 | 10,48 mg | 15,7 µl | 44,2 nm |
| 4 | 5,53 mg | 8,3 µl | 51,5 nm |

## Patentansprüche

1. Verfahren zur Herstellung
von lipidbasierten Nanopartikeln durch Homogenisieren einer Lipidkomponente mit einer wässerigen Komponente in einer dualen asymmetrische Zentrifuge (DAZ); oder
von mit Wirkstoff beladenen lipidbasierten Nanopartikeln durch Homogenisieren einer Lipidkomponente mit einer wässrigen Komponente und dem Wirkstoff in einer DAZ oder durch Einarbeiten des Wirkstoffs in vorgefertigte lipidbasierte Nanopartikel in einer DAZ.

2. Verfahren nach Anspruch 1, wobei
(i) die Homogenisierung bzw. das Einarbeiten bei wenigstens 200 rpm, bevorzugt bei wenigstens 1000 rpm, besonders bevorzugt bei 2000 bis 3540 rpm, am bevorzugtesten bei 2500 bis 3540 rpm erfolgt; und/oder
(ii) die g-Zahl wenigstens 1,2 g, bevorzugt wenigstens 80 g, besonders bevorzugt wenigstens 300 g, ganz besonders bevorzugt von 550 bis 1000 g oder von 620 bis 1500 g beträgt; und/oder
(iii) das Rückdrehverhältnis von 1:6 bis 6:1 beträgt, bevorzugt kleiner als 5:1, besonders bevorzugt kleiner als 3:1 ist; und/oder
(iv) die Zentrifugationszeit von 30 s bis 1 h, bevorzugt von 1 bis 40 min, besonders bevorzugt von 3 bis 20 min beträgt; und/oder
(v) eine Mischhilfe, bevorzugt Glasperlen mit einem Durchmesser von 0,5 bis 6 mm, verwendet wird.

3. Verfahren nach Anspruch 1 oder 2, wobei
(i) die Lipidkomponente eines oder mehrere Amphiphile, Lipide, Detergentien umfasst, bevorzugt mindestens ein Lipid ausgewählt aus Phospholipiden, Glycolipiden, Cholesteroten, Sphingolipiden, Polyethylenglykol-Lipidderivaten, kationischen Lipiden, Triglyceriden und Wachsen, wobei bei Einsatz einer Mischung als Lipidkomponente diese als feste Lösung oder als eine Mischung einzelner Lipid-Kristalle eingesetzt wird; und/oder
(ii) die wässerige Komponente Wasser oder eine wässerige alkoholische und/oder pufferhaltige Lösung ist; und/oder
(iii) die Lipid- und/oder wässerige Komponente eine oder mehrere funktionelle Substanzen enthält, die lipophil oder hydrophil sein können, wobei diese Substanzen bevorzugt ausgewählt sind aus der Gruppe von pharmazeutisch wirksamen, diagnostisch relevanten, kosmetisch wirksamen, biosynthetisch oder für die chemische Synthese relevanten Verbindungen; und/oder
(iv) als Hilfsstoffe Detergentien und/oder Emulgatoren zugesetzt werden.

4. Verfahren nach einem oder mehreren der Ansprüche 1 bis 3, worin die lipidbasierten Nanopartikel hergestellt werden
(i) durch Vorlage einer der beiden Komponenten in einem Gefäß und nachfolgende Zugabe der anderen Komponente während der Homogenisierung in der DAZ; oder
(ii) durch Verwendung eines Gemisches aus Lipidkomponente und wässeriger Komponente und anschließende Homogenisierung in der DAZ; und/oder
(iii) durch Einarbeiten einer wässerigen Komponente in eine vorgefertigte Dispersion lipidbasierter Nanopartikel (Redispersion).

5. Verfahren nach einem oder mehreren der Ansprüche 1 bis 4, wobei
(i) die Nanopartikel eine Partikelgröße von 5 bis 1000 nm haben, bevorzugt von 15 bis 200 nm, besonders bevorzugt von 20 bis 150 nm; und/oder
(ii) Gefäße aus Glas oder Kunststoff eingesetzt werden; und/oder
(iii) Injektionsfläschchen als Gefäße eingesetzt werden; und/oder
(iv) Gefäße mit einem Durchmesser von 5 bis 75 mm, bevorzugt von 9 bis 75 mm eingesetzt werden, besonders bevorzugt Eppendorf®-Gefäße mit einem Durchmesser von 9 bis 10 mm und Glasgefäße mit einem Durchmesser von 9 bis 40 mm; und/oder
(v) das Verfahren mit einer DAZ erfolgt, die zur Aufnahme von Reaktionsgefäßen der Größe von 0,1 bis 110 ml, bevorzugt von 0,1 bis 25 ml, besonders bevorzugt von 0,5 bis 2 ml geeignet ist.

6. Verfahren nach einem oder mehreren der Ansprüche 1 bis 5, worin die lipidbasierten Nanopartikel Liposomen einschließlich vesikulärer Phospholipidgele (VPG) sind, in denen bevorzugt
(i) die Konzentration der Lipidkomponente von 1 bis 600 mM, bevorzugt von 20 bis 600 mM, besonders bevorzugt von 200 bis 600 mM beträgt; und/oder
(ii) die Lipidkomponente ausgewählt ist aus der Gruppe umfassend Phospholipide, Cholesterol und kationische Lipide und besonders bevorzugt ein Phosphatidylcholin ist.

7. Verfahren nach einem oder mehren der Ansprüche 1 bis 4, worin die lipidbasierten Nanopartikel Tröpfchen in Emulsionen, insbesondere in Nanoemulsionen sind, die vorzugsweise
(i) von 10 bis 20% (w/v) Öl und von 0,5 bis 2,5 % (w/v) Lecithin oder Polymeremulgatoren enthalten, und/oder
(ii) einen oder mehrere in Wasser unlösliche Wirkstoffe enthalten, bevorzugt ausgewählt aus der Gruppe von Taxanen, Amphotericin B, Campthotecin und Dekapeptiden.

8. Verfahren nach einem oder mehren der Ansprüche 1 bis 4, wobei die lipidbasierten Nanopartikel feste Lipidnanopartikel (SLN) sind, die vorzugsweise
(i) bei höheren Temperaturen als Raumtemperatur hergestellt werden; und/oder
(ii) Triglyceride und/oder Wachse als Lipidkomponente sowie bevorzugt des weiteren mindestens einen Emulgator oder ein Tensid enthalten, wobei der Emulgator besonders bevorzugt Tyloxapol ist; und/oder
(iii) in Wasser unlösliche Wirkstoffe enthalten, bevorzugt ausgewählt aus der Gruppe von Taxanen, Amphotericin B, Campthotecin und Dekapeptiden.

9. Verfahren nach einem oder mehreren der Ansprüche 1 bis 6, das zur Einarbeitung einer Verbindung in vorgefertigte Liposomen zur Vorbereitung des passiven Loadings oder zur Lipoplexbildung geeignet ist, wobei
(i) der beim passiven Loading verwendete Wirkstoff bevorzugt ausgewählt ist aus Gemcitabine, Vincristin, Vindesin und Platinverbindungen, und
(ii) die bei der Lipoplexbildung verwendete Nukleinsäure bevorzugt cDNA, siRNA oder dsRNA ist.

10. Verfahren nach einem oder mehreren der Ansprüche 1 bis 9, das zum Screening lipidbasierter Nanopartikel im Bereich der Präformulierung geeignet ist.

11. Lipidbasierte mit Wirkstoff beladene Nanopartikel, die Liposomen sind und die gemäß dem Verfahren nach einem oder mehreren der Ansprüche 1 bis 9 herstellbar oder hergestellt sind, und die empfindliche oder kurzlebige Wirkstoffe oder Diagnostika enthalten, weiche ausgewählt sind aus kurzlebig radioaktiv markierten Verbindungen oder Verbindungen für die Positronen-Emissions-Tomographie (PET)-Diagnostik.

12. Lipidbasierte Nanopartikel nach Anspruch 11,
(i) in denen maximal 10 % der ursprünglich im Verfahren eingesetzten empfindlichen oder kurzlebigen Substanz zu einem Abbauprodukt umgewandelt sind; und/oder
(ii) die maximal 10 % herstellungsbedingtes Lysolipid enthalten.

13. Zusammensetzung, insbesondere pharmazeutische oder diagnostische Zusammensetzung, enthaltend die lipidbasierten Nanopartikel nach Anspruch 11 oder 12.

14. Verwendung der lipidbasierten Nanopartikel nach Anspruch 11 oder 12 zur Herstellung von pharmazeutisch oder diagnostisch einsetzbaren Zubereitungen.

15. Mischvorrichtung für lipidbasierte Nanopartikel, insbesondere eine für ein Verfahren gemäß Ansprüchen 1 bis 10 geeignete DAZ, mit
einer ersten Antriebseinrichtung (12) zum Drehen eines Auslegers (16) um eine erste Drehachse (18),
einem in einem Abstand zur ersten Drehachse (18) mit dem Ausleger (16) verbundenen Mischbehälter (40) zur Aufnahme der Substanzen,
einer zweiten Antriebseinrichtung (30) zum Drehen des Mischbehälters (40) um eine zweite, durch den Mischbehälter (40) verlaufende Drehachse (28), wobei die Innenwände (46) des Mischbehälters (40) unterschiedliche Abstände zur zweiten Drehachse (28) aufweisen, der Mischbehälter (40) im Wesentlichen zylindrisch ausgebildet ist, und eine Längsachse (47) des Mischbehälters (40) in einem Winkel zur zweiten Drehachse (28) angeordnet ist.

16. Mischvorrichtung nach Anspruch 15, wobei
(i) der Winkel der Längsachse (47) des Mischbehälters zur zweiten Drehachse (28) im Bereich von 70 bis 110° liegt; und/oder
(ii) die Mischvorrichtung einen Aufnahmebehälter (24) zur Aufnahme des Mischbehälters (40) aufweist.

17. Mischvorrichtung nach Anspruch 16 **gekennzeichnet durch** eine innerhalb des Aufnahmebehälters (24) angeordnete Halteeinrichtung zum lagegenauen Halten des Mischbehälters (40) in dem Aufnahmebehälter (24).

18. Mischvorrichtung nach Anspruch 17, wobei
(i) die Halteeinrichtung (72, 88) einen insbesondere zylindrischen Aufnahmeraum (74, 90, 92) aufweist, in dem der Mischbehälter (40, 48, 76, 94) einsteckbar ist, vorzugsweise die Innenabmessungen des Aufnahmeraums (74, 90, 92) den Außenabmessungen des Mischbehälters (40, 48, 76, 94) entsprechen; und/oder
(ii) der Aufnahmeraum (74) mit einem Deckel (78) verschließbar ist, der eine Öffnung (80) zum Hindurchführen eines Mischbehälter-Halses (82) aufweist; und/oder
(iii) die Mischvorrichtung ein insbesondere ringförmig ausgebildetes Dämpfungselement (86) aufweist, das an dem Mischbehälter (76) anliegt;
(iv) die Halteeinrichtung als Aufnahmebehälter-Einsatz (72, 88) ausgebildet ist; und/oder
(v) die Halteeinrichtung (88) mehrere Aufnahmeräume (90) aufweist, wobei die Längsachse mindestens eines Aufnahmeraums (90) parallel zur zweiten Drehachse (28) und in einem Abstand zu dieser verläuft; und/oder
(vi) die Halteeinrichtung (88) mehrere Aufnahmeräume (92) aufweist, wobei die Längsachse mindestens eines Aufnahmeraums (92) senkrecht zur zweiten Drehachse (28) verläuft.

19. Mischbehälter für eine DAZ, insbesondere zur Durchführung des Verfahrens nach einem oder mehreren der Ansprüche 1 bis 10 und/oder für eine Mischvorrichtung nach einem oder mehreren der Ansprüche 15 bis 18, mit einem ersten Aufnahmeraum (50) zur Aufnahme einer ersten Substanz, mindestens einem zweiten Aufnahmeraum (52) zur Aufnahme einer zweiten Substanz und
einer Trennwand (70) zwischen den Aufnahmeräumen (50, 52) mit einer Öffnung (68), um beim Auftreten von Zentrifugalkräften ein Überführen einer der Substanzen in den anderen Aufnahmeraum (50, 52) zu ermöglichen,
**dadurch gekennzeichnet, dass**
der zweite Aufnahmeraum (52) kegelförmig ausgebildet ist, wobei vorzugsweise die Öffnung (68) an der Kegelspitze (66) angeordnet ist.

20. Mischbehälter nach Anspruch 19, wobei
(i) die Öffnung (68) auf einer Längsachse (56) des ersten und/oder zweiten Aufnahmeraums (50, 52) angeordnet ist; und/oder
(ii) der zweite Aufnahmeraum (52) innerhalb des ersten Aufnahmeraums (50) angeordnet ist; und/oder
(iii) der erste Aufnahmeraum (50) zylindrisch ist.

21. Kit zur Durchführung des Verfahrens nach einem oder mehreren der Ansprüche 1 bis 10; umfassend
(i) einen Mischbehälter nach einem oder mehreren der Ansprüche 19 bis 20; und
(ii) eine oder mehrere Lipidkomponente(n), sowie optional eine wässerige Komponente oder die nichtwässerigen Bestandteile der wässerigen Komponente und/oder Prämixe dieser Komponenten; und/oder
(iii) vorgefertigte lipidbasierte Nanopartikel.

22. Kit nach Anspruch 21, weiterhin umfassend eine Mischhilfe, bevorzugt Glasperlen mit einem Durchmesser von 0,5 bis 6 mm, verwendet wird.

## Claims

1. A process for manufacturing
lipid-based nanoparticles by homogenizing a lipid component with an aqueous component in a dual asymmetric centrifuge (DAC); or
drug-charged lipid-based nanoparticles by homogenizing a lipid component with an aqueous component and the drug in a DAC or by incorporating the drug in preformed lipid-based nanoparticles in a DAC.

2. The process according to claim 1, wherein
(i) homogenizing and/or incorporating is performed with at least 200 rpm, preferably at least 1000 rpm, especially preferred at from 2000 to 3540 rpm, most preferred at from 2500 to 3540 rpm; and/or
(ii) the g-number is at least 1.2 g, preferably at least 80 g, especially preferred at least 300 g, particularly preferred from 550 to 1000 g or from 620 to 1500 g; and/or
(iii) the counter-rotation ratio is from 1:6 to 6:1, preferably below 5:1, especially preferred below 3:1; and/or
(iv) the centrifugation time is from 30 s to 1 h, preferably from 1 to 40 min, especially preferred from 3 to 20 min; and/or
(v) a mixing aid, preferably glass beads having a diameter of from 0.5 to 6 mm is used.

3. The process according to claim (1) or (2) wherein
(i) the lipid component comprises one or several amphiphiles, lipids, detergents, especially preferred at least one lipid selected from phospholipids, glycolipids, cholesterols, sphingolipids, polyethylene glycol lipid derivatives, cationic lipids, triglycerides and waxes wherein when using a mixture as lipid component said mixture is employed as a solid solution or as a mixture of single lipid crystals; and/or
(ii) the aqueous component is water or an aqueous alcoholic and/or buffer containing solution; and/or
(iii) the lipid and/or the aqueous component contains one or several functional substances which may be lipophilic or hydrophilic, wherein said substances are preferably selected from the group of pharmaceutically active, diagnostically relevant, cosmetically active, biosynthetic compounds or compounds relevant for chemical synthesis; and/or
(iv) detergents and/or emulsifiers are added as adjuvants.

4. The process according to one or more of claims 1 to 3 wherein the lipid-based nanoparticles are manufactured
(i) by charging a vessel with one of both components and subsequently adding the other component during the homogenization in the DAC or
(ii) by using a mixture of the lipid component and the aqueous component and subsequently homogenizing in the DAC and/or
(iii) by introducing an aqueous component into a preformed dispersion of lipid-based nanoparticles (redispersion).

5. The process according to one or more of claims 1 to 4 wherein
(i) the nanoparticles have a particle size of from 5 to 1000 nm, preferably of from 15 to 200 nm, especially preferred of from 20 to 150 nm; and/or
(ii) vessels made from glass or plastics are used; and/or
(iii) injection bottles are used as vessels; and/or
(iv) vessels having a diameter of from 5 to 75 mm, preferably of from 9 to 75 mm, especially preferred Eppendorf® vessels having a size of from 9 to 10 mm and glass vessels having a diameter of from 9 to 40 mm are used; and/or
(v) the process is performed using a DAC suited to take up reaction vessels having sizes of from 0.1 to 110 ml, preferably of from 0.1 to 25 ml, especially preferred of from 0.5 to 2 ml.

6. The process according to one or more of claims 1 to 5 wherein the lipid-based nanoparticles are liposomes including vesicular phospholipid gels (VPG), wherein preferably
(i) the concentration of the lipid component is from 1 to 600 mM, preferably from 20 to 600 mM, especially preferred from 200 to 600 mM; and/or
(ii) the lipid component is selected from the group comprising phospholipids, cholesterol and cationic lipids and especially preferred is a phosphatidylcholine.

7. The process according to one or more of claims 1 to 4, wherein the lipid-based nanoparticles are droplets in emulsions, in particular in nanoemulsions preferably containing
(i) from 10 to 20% (weight/vol) of oil and from 0.5 to 2.5% (weight/vol) of lecithin or polymer emulsifiers; and/or
(ii) one or more water-insoluble active substances preferably selected from the group of taxanes, amphotericine B, campthotecine and dekapeptides.

8. The process according to one or more of claims 1 to 4, wherein the lipid-based nanoparticles are solid lipid nanoparticles (SLN) that preferably
(i) are manufactured at temperatures exceeding room temperature and/or
(ii) contain triglycerides and/or waxes as lipid component and preferably moreover contain at least one emulsifier or a surfactant, wherein the emulsifier is more preferably tyloxapol; and/or
(iii) contain water-insoluble active substances, said substances preferably being selected from the group of taxanes, amphotericine B, campthotecine and dekapeptides.

9. The process according to one or more of claims 1 to 6 suited for incorporating a compound into preformed liposomes for preparing passive loading or for the formation of lipoplexes, wherein
(i) the active substance used for passive loading is preferably selected from gemcitabine, vincristine, vindesin and platinum compounds and
(ii) the nucleic acid used in the formation of lipoplexes is preferably cDNA, siRNA or dsRNA.

10. The process according to one or more of claims 1 to 9 suited for the screening of lipid-based nanoparticles in the field of preformulation.

11. Lipid-based nanoparticles loaded with an active substance, which are liposomes obtainable or obtained by the process according to one or more of claims 1 to 9 and which contain sensitive or short-lived substances or diagnostics selected from short-lived radioactively labeled compounds or compounds for positron-emission tomography (PET) diagnostics.

12. The lipid-based nanoparticles according to claim 11,
(i) wherein maximally 10% of the sensitive or short-lived substance originally employed in the process is converted to a degradation product and/or
(ii) which contain maximally 10% lysolipid that is due to the manufacturing method employed.

13. A composition, in particular a pharmaceutical or diagnostic composition, containing the lipid-based nanoparticles according to claim 11 or 12.

14. Use of the lipid-based nanoparticles according to claim 11 or 12 for manufacturing pharmaceutically or diagnostically employable formulations.

15. A mixing device for lipid-based nanoparticles, in particular a DAC suited for a process according to claims 1 to 10 having
a first driving device (12) for rotating a cantilever (16) around a first rotation axis (18),
a mixing vessel (40) for taking up the substances connected with the cantilever (16) at a distance from the first rotation axis (18),
a second driving device (30) for rotating the mixing vessel (40) around a second rotation axis (28) extending through the mixing vessel (40), the interior walls (46) of the mixing device (40) having different distances to the second rotation axis (28), said mixing vessel (40) having a substantially cylindrical design, and a longitudinal axis (47) of the mixing vessel (40) is arranged at an angle towards the second rotational axis (28).

16. The mixing device according to claim 15, wherein
(i) the angle between the longitudinal axis (47) of the mixing vessel and the second rotation axis (28) is in the range of from 70 to 110°; and/or
(ii) said mixing device has an uptake vessel (24) for taking up the mixing vessel (40).

17. The mixing device according to claim 16, **characterized by** a holding device arranged within the uptake vessel (24) for holding the mixing vessel (40) in a positionally accurate manner within the uptake vessel (24).

18. The mixing device according to claim 17, wherein
(i) the holding device (72, 88) has an uptake space (74, 90, 92), in particular a cylindrical one, into which the mixing vessel (40, 48, 76, 94) is insertable, preferably the inner dimensions of the uptake space (74, 90, 92) correspond to the outer dimensions of the mixing vessel (40, 48, 76, 94); and/or
(ii) the uptake space (74) is closable with a lid (78) having an opening (80) for passing through a mixing vessel neck (82); and/or
(iii) the mixing device has a dampening element (86), in particular with an annular design, bearing against the mixing vessel (76);
(iv) the holding device is designed as an uptake vessel insert (72, 88); and/or
(v) the holding device (88) has multiple uptake spaces (90) wherein the longitudinal axis of at least one uptake space (90) is arranged in parallel to the second rotation axis (28) and spaced apart therefrom; and/or
(vi) the holding device (88) has multiple uptake spaces (92) wherein the longitudinal axis of at least one uptake space (92) is arranged perpendicular to the second rotation axis (28).

19. A mixing vessel for a DAC, in particular for performing the process according to one or more of claims 1 to 10 and/or for a mixing device according to one or more of claims 15 to 18 having
a first uptake space (50) for taking up a first substance,
at least one second uptake space (52) for taking up a second substance, and
a separating wall (70) between the uptake spaces (50, 52) with an opening (68) to enable the transfer of one of the substances into the other uptake space (50, 52) upon the occurrence of centrifugal forces;
**characterized in that**
the second uptake space (52) is conically designed, preferably wherein the opening (68) is arranged at the cone tip (66).

20. The mixing vessel according to claim 19, wherein
(i) the opening (68) is arranged on a longitudinal axis (56) of the first and/or second uptake space (50, 52); and/or
(ii) the second uptake space (52) is arranged within the first uptake space (50); and/or
(iii) the first uptake space (50) is cylindrical.

21. A kit for performing the process according to one or more of claims 1 to 10, comprising
(i) a mixing vessel according to one or more of claims 19 to 20; and
(ii) one or more lipid components and optionally an aqueous component or the non-aqueous components of the aqueous component and/or premixes of said components and/or
(ii) premanufactured lipid-based nanoparticles.

22. The kit according to claim 21 further comprising a mixing aid, preferably glass beads having a diameter of from 0.5 to 6 mm.

## Revendications

1. Procédé de préparation de nanoparticules lipidiques, par homogénéisation d'un composant lipidique avec un composant aqueux dans une centrifugeuse asymétrique double ;
ou
de nanoparticules lipidiques chargées en principe actif, par homogénéisation d'un composant lipidique avec un composant aqueux et le principe actif, dans une centrifugeuse asymétrique double, ou en incorporant le principe actif à des nanoparticules lipidiques préalablement confectionnées dans une centrifugeuse asymétrique double.

2. Procédé selon la revendication 1, dans lequel
(i) l'homogénéisation ou, selon les cas, l'incorporation est réalisée à au moins 200 tpm, de préférence à au moins 1000 tpm, de façon particulièrement préférée entre 2000 et 3540 tpm, de façon préférée entre toutes entre 2500 et 3540 tpm ; et/ou
(ii) le nombre de g est d'au moins 1,2 g, de préférence d'au moins 80 g, de façon particulièrement préférée d'au moins 300 g, de façon plus particulièrement préférée entre 550 et 1000 g, ou entre 620 et 1500 g ; et/ou
(iii) le rapport de rotation lévogyre est compris entre 1:6 et 6:1, de préférence est inférieur à 5:1, de façon particulièrement préférée est inférieur à 3:1 ; et/ou
(iv) la durée de centrifugation est comprise entre 30 secondes et 1 heure, de préférence entre 1 et 40 minutes, de façon particulièrement préférée entre 3 et 20 minutes ; et/ou
(v) un auxiliaire de mélange est utilisé, de préférence des perles de verre d'un diamètre compris entre 0,5 et 6 mm.

3. Procédé selon la revendication 1 ou 2, dans lequel
(i) le composant lipidique englobe un ou plusieurs amphiphiles, lipides, détergents, de préférence au moins un lipide sélectionné parmi des phospholipides, des glycolipides, des cholestérols, des sphingolipides, des dérivés de lipides conjugués à un polyéthylèneglycol, des lipides cationiques, des triglycérides et des cires, où, lors de l'incorporation d'un mélange en tant que composant lipidique, celui-ci est utilisé sous forme d'une solution solide ou d'un mélange de cristaux lipidiques distincts ; et/ou
(ii) le composant aqueux est l'eau ou une solution alcoolique aqueuse et/ou tamponnée ; et/ou
(iii) le composant lipidique et/ou aqueux englobe une ou plusieurs substances fonctionnelles, qui peuvent être lipophiles ou hydrophiles, ces substances étant de préférence sélectionnées dans le groupe constitué de composés efficaces sur le plan pharmaceutique, pertinents en terme de diagnostic, efficaces sur le plan cosmétique, pertinents en terme de synthèse chimique ou de biosynthèse ; et/ou
(iv) on ajoute des agents détergents et/ou des agents émulsifiants en tant qu'auxiliaires.

4. Procédé selon une ou plusieurs des revendications 1 à 3, dans lequel les nanoparticules lipidiques sont préparées
(i) en plaçant l'un des deux composants dans un réacteur, puis en ajoutant l'autre composant au cours d'un processus d'homogénéisation dans une centrifugeuse asymétrique double ; ou
(ii) en utilisant un mélange de composant lipidique et de composant aqueux, et en l'homogénéisant ensuite dans la centrifugeuse asymétrique double ; et/ou
(iii) en incorporant un composant aqueux à une dispersion de nanoparticules lipidiques confectionnée au préalable (redispersion).

5. Procédé selon une ou plusieurs des revendications 1 à 4, dans lequel
(i) les nanoparticules présentent une taille de particules comprise entre 5 et 1000 nm, de préférence entre 15 et 200 nm, de façon particulièrement préférée entre 20 et 150 nm ; et/ou
(ii) des réacteurs en verre ou en plastique sont utilisés ; et/ou
(iii) des petits flacons pour injection sont utilisés en guise de réacteurs ; et/ou
(iv) des réacteurs présentant un diamètre compris entre 5 et 75 mm, de préférence entre 9 et 75 mm, sont utilisés, de façon particulièrement préférée des réacteurs de type Eppendorf® au diamètre compris entre 9 et 10 mm, et des flacons de verre au diamètre de 9 à 40 mm ; et/ou
(v) le procédé est conduit en centrifugeuse asymétrique double adaptée pour loger des réacteurs d'une contenance de 0,1 à 110 ml, de préférence de 0,1 à 25 ml, de façon particulièrement préférée de 0,5 à 2 ml.

6. Procédé selon une ou plusieurs des revendications 1 à 5, dans lequel les nanoparticules lipidiques sont des liposomes, notamment des gels vésiculaires de phospholipides, dans lesquels, de préférence,
(i) la concentration en composant lipidique est comprise entre 1 et 600 mM, de préférence entre 20 et 600 mM, de façon particulièrement préférée entre 200 et 600 mM ; et/ou
(ii) le composant lipidique est sélectionné dans le groupe constitué de phospholipides, de cholestérol et de lipides cationiques, et de façon particulièrement préférée est une phosphatidylcholine.

7. Procédé selon une ou plusieurs des revendications 1 à 4, dans lequel les nanoparticules lipidiques sont des gouttelettes dans des émulsions, en particulier des nanoémulsions, qui de préférence
(i) contiennent 10 à 20 % (poids/volume) d'huile et 0,5 à 2,5 % (poids/volume) de lécithine ou d'émulsifiants de type polymères, et/ou
(ii) contiennent une ou plusieurs substances actives insolubles dans l'eau, sélectionnées de préférence dans le groupe constitué de taxanes, d'amphotéricine B, de campthotécine et de décapeptides.

8. Procédé selon une ou plusieurs des revendications 1 à 4, dans lequel les nanoparticules lipidiques sont des nanoparticules lipidiques solides, qui de préférence
(i) sont préparées à des températures supérieures à la température ambiante ; et/ou
(ii) contiennent des triglycérides et/ou des cires en tant que composant lipidique, de même qu'elles contiennent de préférence en outre au moins un agent émulsifiant ou un agent tensioactif, l'émulsifiant étant de façon particulièrement préférée le tyloxapol ; et/ou
(iii) contiennent des substances actives insolubles dans l'eau, sélectionnées de préférence dans le groupe constitué des taxanes, de l'amphotéricine B, de la campthotécine et des décapeptides.

9. Procédé selon une ou plusieurs des revendications 1 à 6, adapté à l'incorporation dans des liposomes préalablement confectionnés d'un composé, en vue de la préparation de la charge passive, ou de la formation d'un complexe de liposome dans lequel
(i) la substance active utilisée lors de la charge passive est de préférence sélectionnée parmi des composés à base de gemcitabine, de vincristine, de vindésine et de platine, et
(ii) les acides nucléiques utilisés dans la formation du complexe de liposome sont de préférence de l'ADN complémentaire, de l'ARN interférent ou de l'ARN bicaténaire.

10. Procédé selon une ou plusieurs des revendications 1 à 9, adapté au criblage de nanoparticules lipidiques dans le domaine de la préformulation.

11. Nanoparticules lipidiques chargées en principe actif, qui sont des liposomes et qui peuvent être préparées ou sont préparées selon le procédé selon une ou plusieurs des revendications 1 à 9, et qui renferment des principes actifs sensibles ou à vie courte, ou des agents de diagnostic qui sont sélectionnés parmi des composés à vie courte marqués par une substance radioactive ou des composés pour le diagnostic par TEP (tomographie à émissions de positrons).

12. Nanoparticules lipidiques selon la revendication 11,
(i) dans lesquels au plus 10 % de la substance sensible ou à courte vie utilisée initialement dans le procédé sont transformés en un produit de dégradation ; et/ou
(ii) qui contiennent au plus 10 % de lysolipides pour des raisons propres à la préparation.

13. Composition, en particulier composition de diagnostic ou pharmaceutique, contenant les nanoparticules lipidiques selon la revendication 11 ou 12.

14. Utilisation des nanoparticules lipidiques selon la revendication 11 ou 12 pour confectionner des préparations pouvant être utilisées à des fins pharmaceutiques ou diagnostiques.

15. Dispositif de mélange pour nanoparticules lipidiques, en particulier centrifugeuse asymétrique double appropriée pour un procédé selon les revendications 1 à 10, comportant
un premier mécanisme d'entraînement (12) pour la rotation d'un bras (16) autour d'un premier axe de rotation (18),
un récipient de mélange (40) destiné à recevoir les substances, relié au bras (16) avec une certaine distance au premier axe de rotation (18),
un second mécanisme d'entraînement (30) pour la rotation du récipient de mélange (40) autour d'un second axe de rotation (28) passant par le récipient de mélange (40), les parois intérieures (46) du récipient de mélange (40) présentant différents écartements par rapport au second axe de rotation (28), le récipient de mélange (40) étant de conception essentiellement cylindrique, et un axe longitudinal (47) du récipient de mélange (40) étant disposé en formant un angle avec le second axe de rotation (28).

16. Dispositif de mélange selon la revendication 15, dans lequel
(i) l'angle entre l'axe longitudinal (47) du récipient de mélange (40) et le second axe de rotation (28) est compris dans la plage de 70 à 110 ° ; et/ou
(ii) le dispositif de mélange présente un dispositif receveur (24) destiné à loger le récipient de mélange (40).

17. Dispositif de mélange selon la revendication 16, **caractérisé par** un dispositif de retenue disposé à l'intérieur du dispositif receveur (24) et destiné à garantir le positionnement exact du récipient de mélange (40) dans le dispositif receveur (24).

18. Dispositif de mélange selon la revendication 17, dans lequel
(i) le dispositif de retenue (72, 88) présente un espace de réception, en particulier cylindrique (74, 90, 92), dans lequel le récipient de mélange (40, 48, 76, 94) peut venir s'emboîter, les dimensions internes de l'espace de réception (74, 90, 92) correspondant de préférence aux dimensions externes du récipient de mélange (40, 48, 76, 94) ; et/ou
(ii) l'espace de réception (74) peut être fermé par un couvercle (78), qui présente une ouverture (80) permettant le passage d'un col (82) d'un récipient de mélange ; et/ou
(iii) le dispositif de mélange présente un élément amortisseur (86), en particulier de conception annulaire, qui s'appuie contre le récipient de mélange (76) ;
(iv) le dispositif de retenue est conçu en tant que pièce d'insertion du dispositif receveur (72, 88) ; et/ou
(v) le dispositif de retenue (88) présente plusieurs espaces de réception (90), l'axe longitudinal d'au moins un espace de réception (90) s'étendant parallèlement au second axe de rotation (28) et à une certaine distance de celui-ci ; et/ou
(vi) le dispositif de retenue (88) présente plusieurs espaces de réception (92), l'axe longitudinal d'au moins un espace de réception (92) s'étendant perpendiculairement au second axe de rotation (28).

19. Récipient de mélange pour centrifugeuse asymétrique double, en particulier pour mettre en oeuvre le procédé selon une ou plusieurs des revendications 1 à 10, et/ou pour un dispositif de mélange selon une ou plusieurs des revendications 15 à 18, comprenant
un premier espace de réception (50) destiné à recueillir une première substance, au moins un second espace de réception (52) destiné à recueillir une seconde substance, et
une cloison de séparation (70) entre les espaces de réception (50, 52) avec une ouverture (68), pour permettre, sous l'effet de forces centrifuges, un transfert de l'une des substances dans l'autre espace de réception (50, 52),
**caractérisé en ce que**
le second espace de réception (52) est de conception conique, l'ouverture (68) étant de préférence disposée à la pointe du cône (66).

20. Récipient de mélange selon la revendication 19, dans lequel
(i) l'ouverture (68) est disposée sur un axe longitudinal (56) du premier et/ou du second espace de réception (50, 52) ; et/ou
(ii) le second espace de réception (52) est disposé à l'intérieur du premier espace de réception (50) ; et/ou
(iii) le premier espace de réception (50) est cylindrique.

21. Kit de réalisation du procédé selon une ou plusieurs des revendications 1 à 10, comprenant
(i) un récipient de mélange selon une ou plusieurs des revendications 19 à 20 ; et
(ii) un ou plusieurs composant(s) lipidique(s), ainsi qu'éventuellement un composant aqueux ou les constituants non aqueux du composant aqueux et/ou des prémélanges de ces composants ; et/ou
(iii) des nanoparticules lipidiques confectionnées au préalable.

22. Kit selon la revendication 21, comprenant en outre un auxiliaire de mélange, de préférence des perles de verre d'un diamètre compris entre 0,5 et 6 mm.
